# EUROPEAN PATENT APPLICATION

(11) **EP 4 566 632 A1**
(43) Date of publication of application: **11.06.2025**
(21) Application number: 23849414.0
(22) Date of filing: 01.08.2023
(51) Int. Cl.: A61K 47/68, A61P 35/00, C07K 16/28

(54) **MSLN ANTIBODY-DRUG CONJUGATE**

(30) Priority: 02.08.2022 CN 202210922954; 24.07.2023 CN 202310912034
(71) Applicant: Nona Biosciences (Suzhou) Co., Ltd., Suzhou, Jiangsu 215000 (CN)
(72) Inventor: WANG, Xiaoxiao, Suzhou, Jiangsu 215000 (CN); ZHANG, Meihong, Suzhou, Jiangsu 215000 (CN); XIAO, Liang, Suzhou, Jiangsu 215000 (CN); CAI, Jiaqiang, Suzhou, Jiangsu 215000 (CN); WANG, Di, Suzhou, Jiangsu 215000 (CN); XUE, Tongtong, Suzhou, Jiangsu 215000 (CN); ZHANG, Wei, Suzhou, Jiangsu 215000 (CN); GUAN, Guangkuo, Suzhou, Jiangsu 215000 (CN)
(74) Representative: Lee, Nicholas John
(86) International application number: PCT/CN2023/110552
(87) International publication number: WO 2024/027708

(57) **Abstract**

Provided is an antibody-drug conjugate, which is a compound represented by formula (I) or a tautomer or a pharmaceutically acceptable salt thereof: Ab-[S-X-(Y)n-Z-E-D]q(I), wherein Ab is an antibody that specifically binds to mesothelin (MSLN) or an antigen-binding fragment thereof, and D is a bioactive molecular drug or a derivative thereof. Provided is a pharmaceutical composition, comprising the antibody-drug conjugate represented by formula (I). Provided is use of the antibody-drug conjugate represented by formula (I) and the pharmaceutical composition thereof in the preparation of a drug for treating cancer.

## Description

### Technical Field

The present invention relates to the field of targeted therapy and more specifically to the field of antibody-drug conjugates.

### Background Technology

Mesothelin (MSLN) is a cell surface molecule which is expressed as a 71-kD precursor protein and is further processed to become a 40-kD glycoprotein that is anchored to the cell surface by glycosylphosphatidylinositol (GPI) on the cell surface. The expression of MSLN in normal tissues is highly restricted. It is expressed in mesothelial cells lining the pleura, pericardium, and peritoneum, where it appears to play a role in cell adhesion (Chang et al. (1996), PNAS, 93:136-140). Soluble cleaved MSLN has also been said to play a role in megakaryocyte stimulation, but knocking out same in mice did not show any developmental defects, and as such its biological function remains unclear (Yamaguchi et al. (1994), Journal of Biological Chemistry, 269(2):805-8; Bera et al. (2000), Molecular and Cellular Biology, 20(8):2902-6).

In contrast, MSLN is highly expressed in certain human cancers, including almost all mesotheliomas and pancreatic cancers, as well as approximately 70% of ovarian cancers and 50% of lung adenocarcinomas (Hassan and Ho (2008), European Journal of Cancer, 44:46-53; Miettinen and Sarlomo-Rikala (2003), The American Journal of Surgical Pathology, 27:150-8; Ordonez (2003), The American Journal of Surgical Pathology, 27:1418-1428; Ho et al. (2007), Clinical Cancer Research, 13:1571-5). The high expression levels of MSLN make it an attractive candidate for targeted therapies, as it plays an important role in promoting tumor proliferation and invasion (Servais et al. (2012), Clinical Cancer Research, 18(9):2478-2489).

MSLN interacts with MUC16, mediates cell adhesion, plays a significant role in the peritoneal implantation of ovarian cancer cells, and increases the motility and invasiveness of pancreatic cancer cells (Rump et al. (2004), Journal of Biological Chemistry, 279(10):9190-8; Gubbels et al. (2006), Molecular Cancer, 5(1):50; Coehlo et al., Expert Review of Anticancer Therapy, 18(2):177-186; Chen et al. (2013), Scientific Reports, 3:1870). Particularly, pancreatic tumors often progress too rapidly for patients to notice symptoms of the disease, resulting in a very short average survival time. The prognosis is poor because these tumors typically cannot be (completely) removed by surgical resection and have already metastasized. Chemotherapy also fails to significantly improve the survival time or cure rates of these tumors. Despite multiple attempts made by scientists, antibodies targeting MSLN alone have nonetheless shown defects in terms of efficacy, so they are not excellent targeted therapy means. Currently, the fastest-developing and most well-known anti-mesothelin monoclonal antibody, amatuximab, has still shown disappointing results in clinical trials (Baldo and Cecco (2017), Onco. Targets Ther. 10:5337-5353; Nicolaides et al. (2018), Cancer Biology & Therapy, 19(7):622-630).

To further address this challenge, antibody-drug conjugates (ADCs) have been introduced into MSLN-targeted therapy. Compared to traditional antibodies, MSLN-targeting ADCs offer superior tumor-killing efficacy due to the combination of the targeting ability of antibodies and the potency of small-molecule drugs. Currently, the fastest-progressing ADC in this field is Anetumab ravtansine, developed by Bayer. Its Phase I clinical data demonstrated good safety and efficacy, and it is currently being tested in multiple tumor types with high MSLN expression. Therefore, there is an urgent need in this field for targeted therapies against MSLN. However, in recent years, the development of ADC drugs has accelerated, leading to the continuous emergence of better linker-toxin systems. However, both the traditional non-degradable linker and DM4 toxin used in Anetumab ravtansine pose challenges such as high drug toxicity, a narrow therapeutic window, and a low effective release rate of the drug in targeted organs (WO 2010/124797 A1). Additionally, MSLN itself is cleaved by various proteases at sites near a membrane, producing soluble MSLN (soluble MSLN, sMSLN) (Molecular Cancer Therapeutics; 15(7), July 2016). Such soluble MSLN has a sequence highly similar to that of membrane-anchored MSLN and is therefore recognized by most therapeutic antibodies targeting MSLN, including Anetumab and Amatuximab. Experiments prove that soluble MSLN competitively binds to MSLN antibodies, thereby compromising the efficacy of MSLN-targeted therapies.

Therefore, there is currently an urgent need in this field to develop a superior ADC that targets MSLN, which, on the one hand, demonstrates enhanced anti-tumor efficacy and improved plasma stability, and on the other hand, is less affected by soluble MSLN in terms of its efficacy of inhibiting tumor growth.

### Summary of the Invention

The present invention provides an antibody-drug conjugate targeting MSLN and use thereof for treating cancer.

In a first aspect, the present invention provides an antibody-drug conjugate, which is a compound of formula (I) or a tautomer, stereoisomer, or pharmaceutically acceptable salt thereof:

Ab-[S-X-(Y)n-Z-E-D]_{q} (I),

In the formula:
Ab is an antibody that specifically binds to mesothelin (MSLN) or an antigen-binding fragment thereof;
S is a sulfur bond connecting Ab and X;
X is selected from a 3-10-membered cycloalkyl, a 5-10-membered heterocyclic group, C₆₋₁₀ aryl, or a 5-10-membered heteroaryl group;
X is optionally substituted with 1, 2, 3, 4, or 5 instance(s) of R*;
R* is selected from H, deuterium, halogen, CN, NO₂, OH, C₁-₆ alkyl, C₁-₆ haloalkyl, C₂-₆ alkenyl, or C₂-₆ alkynyl;
Y is -Y₁-Y₂-Y₃-, Y being linked to X via Y₁ and to Z via Y₃
wherein,
Y₁ is selected from -C(O)-, -C(O)O-, -OC(O)-, -C(O)NH-, C₂-₆ alkenyl, C₂-₆ alkynyl, a 3-10-membered cycloalkyl, a 5-10-membered heterocyclic group, C₆₋₁₀ aryl, or a 5-10-membered heteroaryl group;
Y₂ is a bond or C₁-₆ alkylene;
Y₃ is selected from -C(O)-, -OC(O)-, or -NHC(O)-;
Y is optionally substituted with 1, 2, 3, 4, or 5 R# substituents;
R# is selected from H, deuterium, halogen, CN, NO₂, OH, C₁-₆ alkyl, C₁-₆ haloalkyl, C₂-₆ alkenyl, or C₂-₆ alkynyl;
Z is a peptide containing 2 to 8 amino acids, preferably a dipeptide, tripeptide, or tetrapeptide;
E is absent or a spacer unit optionally connecting Z and D;
D is a biologically active molecule drug or a derivative thereof;
n is selected from 0, 1, 2, 3, 4, or 5, preferably 0, 1, 2, or 3;
q is an integer selected from 1 to 10, preferably 8.

In some embodiments, the antibody-drug conjugate is a compound of formula (II) or a tautomer, stereoisomer, or pharmaceutically acceptable salt thereof: 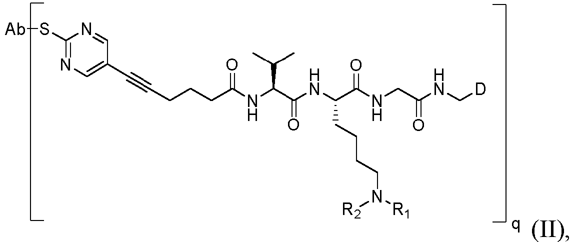 wherein,
Ab is an anti-MSLN antibody;
R₁ and R₂ are independently selected from C₁-₆ alkyl, preferably C₁-₄ alkyl;
D is 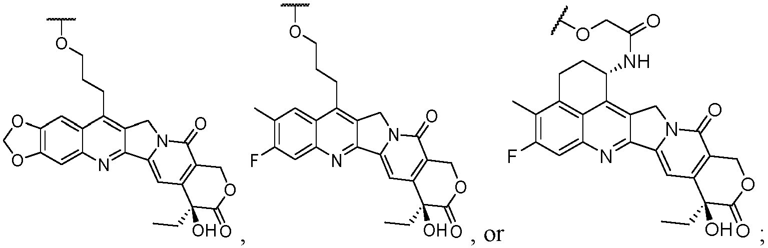
q is an integer selected from 1-10, for example, 4, 5, 6, 7, 8, 9, or 10, preferably 8.

In some embodiments, the antibody-drug conjugate is the following compound or a tautomer, stereoisomer, or pharmaceutically acceptable salt thereof: 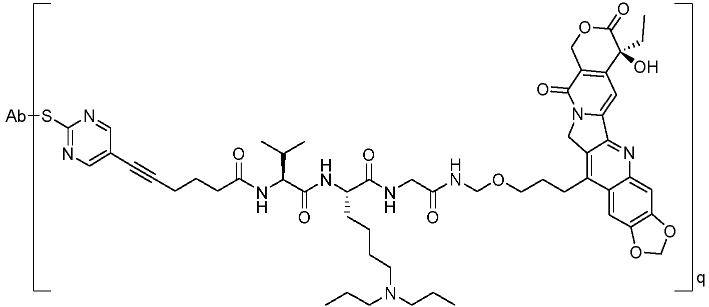
Ab is an anti-MSLN antibody;
q is an integer selected from 1-10, for example, 4, 5, 6, 7, 8, 9, or 10, preferably 8.

In some embodiments, the antibody-drug conjugate is the following compound or a tautomer, stereoisomer, or pharmaceutically acceptable salt thereof: 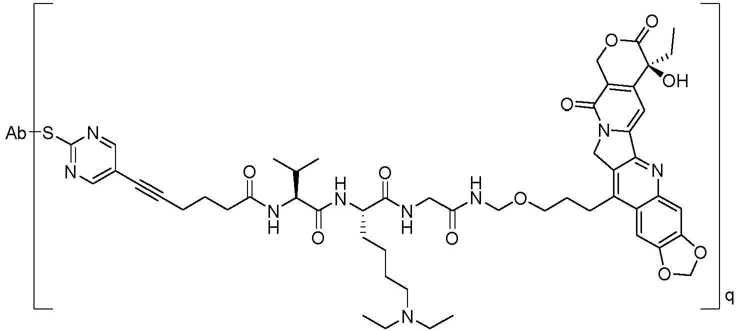
Ab is an anti-MSLN antibody;
q is an integer selected from 1-10, for example, 4, 5, 6, 7, 8, 9, or 10, preferably 8.

In some embodiments, the antibody-drug conjugate is the following compound or a tautomer, stereoisomer, or pharmaceutically acceptable salt thereof: 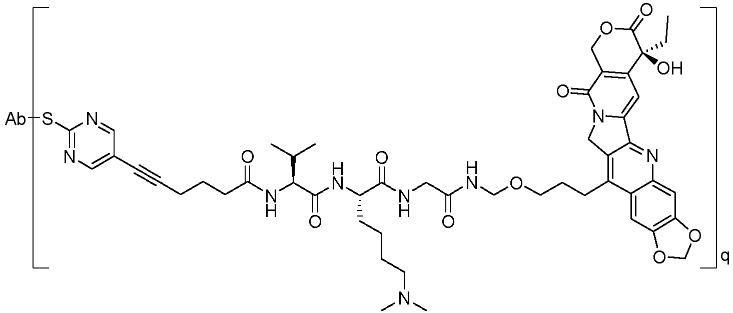
Ab is an anti-MSLN antibody;
q is an integer selected from 1-10, for example, 4, 5, 6, 7, 8, 9, or 10, preferably 8.

In some embodiments, the antibody-drug conjugate is the following compound or a tautomer, stereoisomer, or pharmaceutically acceptable salt thereof: 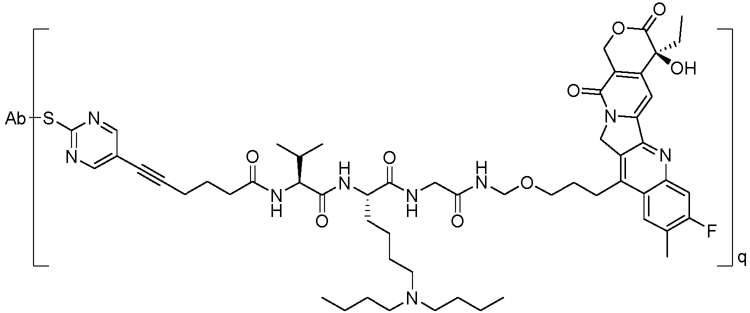
Ab is an anti-MSLN antibody;
q is an integer selected from 1-10, for example, 4, 5, 6, 7, 8, 9, or 10, preferably 8.

In some embodiments, the antibody-drug conjugate is the following compound or a tautomer, stereoisomer, or pharmaceutically acceptable salt thereof: 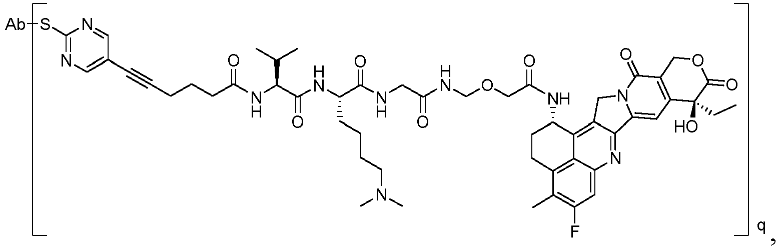
wherein, Ab is an anti-MSLN antibody;
q is an integer selected from 1-10, for example, 4, 5, 6, 7, 8, 9, or 10, preferably 8.

In some embodiments, the antibody Ab in the antibody-drug conjugate of the present invention is an antibody that specifically binds to mesothelin (MSLN) or an antigen-binding fragment thereof, wherein the antibody comprises a heavy chain variable region (VH) and a light chain variable region (VL), wherein,
(1) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 27;
(2) the VH comprises HCDR1, HCDR2, and HCDR3 of the VH with the amino acid sequence shown in SEQ ID NO: 25, and the VL comprises LCDR1, LCDR2, and LCDR3 of the VL with the amino acid sequence shown in SEQ ID NO: 28;
(3) the VH comprises HCDR1, HCDR2, and HCDR3 of the VH with the amino acid sequence shown in SEQ ID NO: 26, and the VL comprises LCDR1, LCDR2, and LCDR3 of the VL with the amino acid sequence shown in SEQ ID NO: 27;
(4) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 29;
(5) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 30;
(6) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 31;
(7) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 32;
(8) the VH comprises HCDR1, HCDR2, and HCDR3 of the VH of amino acid sequence shown in SEQ ID NO: 24, and the VL comprises LCDR1, LCDR2, and LCDR3 of the VL with the amino acid sequence shown in SEQ ID NO: 33;
(9) the VH comprises HCDR1, HCDR2, and HCDR3 of the VH with the amino acid sequence shown in SEQ ID NO: 25, and the VL comprises LCDR1, LCDR2, and LCDR3 of the VL with the amino acid sequence shown in SEQ ID NO: 29;
(10) the VH comprises HCDR1, HCDR2, and HCDR3 of the VH with the amino acid sequence shown in SEQ ID NO: 63, and the VL comprises LCDR1, LCDR2, and LCDR3 of the VL with the amino acid sequence shown in SEQ ID NO: 67;
(11) the VH comprises HCDR1, HCDR2, and HCDR3 of the VH with the amino acid sequence shown in SEQ ID NO: 64, and the VL comprises LCDR1, LCDR2, and LCDR3 of the VL with the amino acid sequence shown in SEQ ID NO: 67;
(12) the VH comprises HCDR1, HCDR2, and HCDR3 of the VH with the amino acid sequence shown in SEQ ID NO: 65, and the VL comprises LCDR1, LCDR2, and LCDR3 of the VL with the amino acid sequence shown in SEQ ID NO: 67;
(13) the VH comprises HCDR1, HCDR2, and HCDR3 of the VH with the amino acid sequence shown in SEQ ID NO: 65, and the VL comprises LCDR1, LCDR2, and LCDR3 of the VL with the amino acid sequence shown in SEQ ID NO: 68;
(14) the VH comprises HCDR1, HCDR2, and HCDR3 of the VH with the amino acid sequence shown in SEQ ID NO: 63, and the VL comprises LCDR1, LCDR2, and LCDR3 of the VL with the amino acid sequence shown in SEQ ID NO: 68;
(15) the VH comprises HCDR1, HCDR2, and HCDR3 of the VH with the amino acid sequence shown in SEQ ID NO: 66, and the VL comprises LCDR1, LCDR2, and LCDR3 of the VL with the amino acid sequence shown in SEQ ID NO: 67;
(16) the VH comprises HCDR1, HCDR2, and HCDR3 of the VH with the amino acid sequence shown in SEQ ID NO: 64, and the VL comprises LCDR1, LCDR2, and LCDR3 of the VL with the amino acid sequence shown in SEQ ID NO: 69;
(17) the VH comprises HCDR1, HCDR2, and HCDR3 of the VH with the amino acid sequence shown in SEQ ID NO: 66, and the VL comprises LCDR1, LCDR2, and LCDR3 of the VL with the amino acid sequence shown in SEQ ID NO: 69;
(18) the VH comprises HCDR1, HCDR2, and HCDR3 of the VH with the amino acid sequence shown in SEQ ID NO: 63, and the VL comprises LCDR1, LCDR2, and LCDR3 of the VL with the amino acid sequence shown in SEQ ID NO: 69; or
(19) the VH comprises HCDR1, HCDR2, and HCDR3 of the VH with the amino acid sequence shown in SEQ ID NO: 65, and the VL comprises LCDR1, LCDR2, and LCDR3 of the VL with the amino acid sequence shown in SEQ ID NO: 69.

In some embodiments, the antibody Ab in the antibody-drug conjugate of the present invention is an antibody that specifically binds to mesothelin (MSLN) or an antigen-binding fragment thereof, wherein the antibody comprises a heavy chain variable region (VH) and a light chain variable region (VL), wherein,
(1) the VH comprises HCDR1, HCDR2, and HCDR3, the HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 13;
(2) the VH comprises HCDR1, HCDR2, and HCDR3, the HCDR1 comprising the amino acid sequence shown in SEQ ID NO: 15, HCDR2 comprising the amino acid sequence shown in SEQ ID NO: 16, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 18;
(3) the VH comprises HCDR1, HCDR2, and HCDR3, the HCDR1 comprising the amino acid sequence shown in SEQ ID NO: 15, HCDR2 comprising the amino acid sequence shown in SEQ ID NO: 17, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 13;
(4) the VH comprises HCDR1, HCDR2, and HCDR3, the HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 19;
(5) the VH comprises HCDR1, HCDR2, and HCDR3, the HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 20;
(6) the VH comprises HCDR1, HCDR2, and HCDR3, the HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 21;
(7) the VH comprises HCDR1, HCDR2, and HCDR3, the HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 22;
(8) the VH comprises HCDR1, HCDR2, and HCDR3, the HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 23;
(9) the VH comprises HCDR1, HCDR2, and HCDR3, the HCDR1 comprising the amino acid sequence shown in SEQ ID NO: 15, HCDR2 comprising the amino acid sequence shown in SEQ ID NO: 16, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 19;
(10) the VH comprises HCDR1, HCDR2, and HCDR3, the HCDR1 comprising the amino acid sequence shown in SEQ ID NO: 45, HCDR2 comprising the amino acid sequence shown in SEQ ID NO: 47, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 52, LCDR2 comprising the amino acid sequence shown in SEQ ID NO: 54, and LCDR3 comprising the amino acid sequence shown in SEQ ID NO: 56;
(11) the VH comprises HCDR1, HCDR2, and HCDR3, the HCDR1 comprising the amino acid sequence shown in SEQ ID NO: 58, HCDR2 comprising the amino acid sequence shown in SEQ ID NO: 47, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 52, LCDR2 comprising the amino acid sequence shown in SEQ ID NO: 54, and LCDR3 comprising the amino acid sequence shown in SEQ ID NO: 56;
(12) the VH comprises HCDR1, HCDR2, and HCDR3, the HCDR1 comprising the amino acid sequence shown in SEQ ID NO: 58, HCDR2 comprising the amino acid sequence shown in SEQ ID NO: 59, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 52, LCDR2 comprising the amino acid sequence shown in SEQ ID NO: 54, and LCDR3 comprising the amino acid sequence shown in SEQ ID NO: 56;
(13) the VH comprises HCDR1, HCDR2, and HCDR3, the HCDR1 comprising the amino acid sequence shown in SEQ ID NO: 58, HCDR2 comprising the amino acid sequence shown in SEQ ID NO: 59, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 61, LCDR2 comprising the amino acid sequence shown in SEQ ID NO: 54, and LCDR3 comprising the amino acid sequence shown in SEQ ID NO: 56;
(14) the VH comprises HCDR1, HCDR2, and HCDR3, the HCDR1 comprising the amino acid sequence shown in SEQ ID NO: 45, HCDR2 comprising the amino acid sequence shown in SEQ ID NO: 47, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 61, LCDR2 comprising the amino acid sequence shown in SEQ ID NO: 54, and LCDR3 comprising the amino acid sequence shown in SEQ ID NO: 56;
(15) the VH comprises HCDR1, HCDR2, and HCDR3, the HCDR1 comprising the amino acid sequence shown in SEQ ID NO: 58, HCDR2 comprising the amino acid sequence shown in SEQ ID NO: 60, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 52, LCDR2 comprising the amino acid sequence shown in SEQ ID NO: 54, and LCDR3 comprising the amino acid sequence shown in SEQ ID NO: 56;
(16) the VH comprises HCDR1, HCDR2, and HCDR3, the HCDR1 comprising the amino acid sequence shown in SEQ ID NO: 58, HCDR2 comprising the amino acid sequence shown in SEQ ID NO: 47, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 62, LCDR2 comprising the amino acid sequence shown in SEQ ID NO: 54, and LCDR3 comprising the amino acid sequence shown in SEQ ID NO: 56;
(17) the VH comprises HCDR1, HCDR2, and HCDR3, the HCDR1 comprising the amino acid sequence shown in SEQ ID NO: 58, HCDR2 comprising the amino acid sequence shown in SEQ ID NO: 60, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 62, LCDR2 comprising the amino acid sequence shown in SEQ ID NO: 54, and LCDR3 comprising the amino acid sequence shown in SEQ ID NO: 56;
(18) the VH comprises HCDR1, HCDR2, and HCDR3, the HCDR1 comprising the amino acid sequence shown in SEQ ID NO: 45, HCDR2 comprising the amino acid sequence shown in SEQ ID NO: 47, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 62, LCDR2 comprising the amino acid sequence shown in SEQ ID NO: 54, and LCDR3 comprising the amino acid sequence shown in SEQ ID NO: 56; or
(19) the VH comprises HCDR1, HCDR2, and HCDR3, the HCDR1 comprising the amino acid sequence shown in SEQ ID NO: 58, HCDR2 comprising the amino acid sequence shown in SEQ ID NO: 59, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 62, LCDR2 comprising the amino acid sequence shown in SEQ ID NO: 54, and LCDR3 comprising the amino acid sequence shown in SEQ ID NO: 56.

In some embodiments, the antibody Ab in the antibody-drug conjugate of the present invention is an antibody that specifically binds to mesothelin (MSLN) or an antigen-binding fragment thereof, wherein the antibody comprises VH and VL, wherein,
(1) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 27;
(2) the VH comprises an amino acid sequence with at least 80%, at least 85%, at least 90%, at least 95%, at least 98%, at least 99%, or 100% sequence identity to SEQ ID NO: 25, and the VL comprises an amino acid sequence with at least 80%, at least 85%, at least 90%, at least 95%, at least 98%, at least 99%, or 100% sequence identity to SEQ ID NO: 28;
(3) the VH comprises an amino acid sequence with at least 80%, at least 85%, at least 90%, at least 95%, at least 98%, at least 99%, or 100% sequence identity to SEQ ID NO: 26, and the VL comprises an amino acid sequence with at least 80%, at least 85%, at least 90%, at least 95%, at least 98%, at least 99%, or 100% sequence identity to SEQ ID NO: 27;
(4) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 29;
(5) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 30;
(6) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 31;
(7) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 32;
(8) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 33;
(9) the VH comprises an amino acid sequence with at least 80%, at least 85%, at least 90%, at least 95%, at least 98%, at least 99%, or 100% sequence identity to SEQ ID NO: 25, and the VL comprises an amino acid sequence with at least 80%, at least 85%, at least 90%, at least 95%, at least 98%, at least 99%, or 100% sequence identity to SEQ ID NO: 29;
(10) the VH comprises an amino acid sequence with at least 80%, at least 85%, at least 90%, at least 95%, at least 98%, at least 99%, or 100% sequence identity to SEQ ID NO: 63, and the VL comprises an amino acid sequence with at least 80%, at least 85%, at least 90%, at least 95%, at least 98%, at least 99%, or 100% sequence identity to SEQ ID NO: 67;
(11) the VH comprises an amino acid sequence with at least 80%, at least 85%, at least 90%, at least 95%, at least 98%, at least 99%, or 100% sequence identity to SEQ ID NO: 64, and the VL comprises an amino acid sequence with at least 80%, at least 85%, at least 90%, at least 95%, at least 98%, at least 99%, or 100% sequence identity to SEQ ID NO: 67;
(12) the VH comprises an amino acid sequence with at least 80%, at least 85%, at least 90%, at least 95%, at least 98%, at least 99%, or 100% sequence identity to SEQ ID NO: 65, and the VL comprises an amino acid sequence with at least 80%, at least 85%, at least 90%, at least 95%, at least 98%, at least 99%, or 100% sequence identity to SEQ ID NO: 67;
(13) the VH comprises an amino acid sequence with at least 80%, at least 85%, at least 90%, at least 95%, at least 98%, at least 99%, or 100% sequence identity to SEQ ID NO: 65, and the VL comprises an amino acid sequence with at least 80%, at least 85%, at least 90%, at least 95%, at least 98%, at least 99%, or 100% sequence identity to SEQ ID NO: 68;
(14) the VH comprises an amino acid sequence with at least 80%, at least 85%, at least 90%, at least 95%, at least 98%, at least 99%, or 100% sequence identity to SEQ ID NO: 63, and the VL comprises an amino acid sequence with at least 80%, at least 85%, at least 90%, at least 95%, at least 98%, at least 99%, or 100% sequence identity to SEQ ID NO: 68;
(15) the VH comprises an amino acid sequence with at least 80%, at least 85%, at least 90%, at least 95%, at least 98%, at least 99%, or 100% sequence identity to SEQ ID NO: 66, and the VL comprises an amino acid sequence with at least 80%, at least 85%, at least 90%, at least 95%, at least 98%, at least 99%, or 100% sequence identity to SEQ ID NO: 67;
(16) the VH comprises an amino acid sequence with at least 80%, at least 85%, at least 90%, at least 95%, at least 98%, at least 99%, or 100% sequence identity to SEQ ID NO: 64, and the VL comprises an amino acid sequence with at least 80%, at least 85%, at least 90%, at least 95%, at least 98%, at least 99%, or 100% sequence identity to SEQ ID NO: 69;
(17) the VH comprises an amino acid sequence with at least 80%, at least 85%, at least 90%, at least 95%, at least 98%, at least 99%, or 100% sequence identity to SEQ ID NO: 66, and the VL comprises an amino acid sequence with at least 80%, at least 85%, at least 90%, at least 95%, at least 98%, at least 99%, or 100% sequence identity to SEQ ID NO: 69;
(18) the VH comprises an amino acid sequence with at least 80%, at least 85%, at least 90%, at least 95%, at least 98%, at least 99%, or 100% sequence identity to SEQ ID NO: 63, and the VL comprises an amino acid sequence with at least 80%, at least 85%, at least 90%, at least 95%, at least 98%, at least 99%, or 100% sequence identity to SEQ ID NO: 69; or
(19) the VH comprises an amino acid sequence with at least 80%, at least 85%, at least 90%, at least 95%, at least 98%, at least 99%, or 100% sequence identity to SEQ ID NO: 65, and the VL comprises an amino acid sequence with at least 80%, at least 85%, at least 90%, at least 95%, at least 98%, at least 99%, or 100% sequence identity to SEQ ID NO: 69.

In some embodiments, the antibody Ab in the antibody-drug conjugate of the present invention is an antibody that specifically binds to mesothelin (MSLN) or an antigen-binding fragment thereof, wherein the antibody comprises a heavy chain (HC) and a light chain (LC), and wherein,
(1) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 37;
(2) the HC comprises an amino acid sequence with at least 80%, at least 85%, at least 90%, at least 95%, at least 98%, at least 99%, or 100% sequence identity to SEQ ID NO: 35, and the LC comprises an amino acid sequence with at least 80%, at least 85%, at least 90%, at least 95%, at least 98%, at least 99%, or 100% sequence identity to SEQ ID NO: 38;
(3) the HC comprises an amino acid sequence with at least 80%, at least 85%, at least 90%, at least 95%, at least 98%, at least 99%, or 100% sequence identity to SEQ ID NO: 36, and the LC comprises an amino acid sequence with at least 80%, at least 85%, at least 90%, at least 95%, at least 98%, at least 99%, or 100% sequence identity to SEQ ID NO: 37;
(4) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 39;
(5) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 40;
(6) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 41;
(7) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 42;
(8) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 43;
(9) the HC comprises an amino acid sequence with at least 80%, at least 85%, at least 90%, at least 95%, at least 98%, at least 99%, or 100% sequence identity to SEQ ID NO: 35, and the LC comprises an amino acid sequence with at least 80%, at least 85%, at least 90%, at least 95%, at least 98%, at least 99%, or 100% sequence identity to SEQ ID NO: 39;
(10) the HC comprises an amino acid sequence with at least 80%, at least 85%, at least 90%, at least 95%, at least 98%, at least 99%, or 100% sequence identity to SEQ ID NO: 70, and the LC comprises an amino acid sequence with at least 80%, at least 85%, at least 90%, at least 95%, at least 98%, at least 99%, or 100% sequence identity to SEQ ID NO: 74;
(11) the HC comprises an amino acid sequence with at least 80%, at least 85%, at least 90%, at least 95%, at least 98%, at least 99%, or 100% sequence identity to SEQ ID NO: 71, and the LC comprises an amino acid sequence with at least 80%, at least 85%, at least 90%, at least 95%, at least 98%, at least 99%, or 100% sequence identity to SEQ ID NO: 74;
(12) the HC comprises an amino acid sequence with at least 80%, at least 85%, at least 90%, at least 95%, at least 98%, at least 99%, or 100% sequence identity to SEQ ID NO: 72, and the LC comprises an amino acid sequence with at least 80%, at least 85%, at least 90%, at least 95%, at least 98%, at least 99%, or 100% sequence identity to SEQ ID NO: 74;
(13) the HC comprises an amino acid sequence with at least 80%, at least 85%, at least 90%, at least 95%, at least 98%, at least 99%, or 100% sequence identity to SEQ ID NO: 72, and the LC comprises an amino acid sequence with at least 80%, at least 85%, at least 90%, at least 95%, at least 98%, at least 99%, or 100% sequence identity to SEQ ID NO: 75;
(14) the HC comprises an amino acid sequence with at least 80%, at least 85%, at least 90%, at least 95%, at least 98%, at least 99%, or 100% sequence identity to SEQ ID NO: 70, and the LC comprises an amino acid sequence with at least 80%, at least 85%, at least 90%, at least 95%, at least 98%, at least 99%, or 100% sequence identity to SEQ ID NO: 75;
(15) the HC comprises an amino acid sequence with at least 80%, at least 85%, at least 90%, at least 95%, at least 98%, at least 99%, or 100% sequence identity to SEQ ID NO: 73, and the LC comprises an amino acid sequence with at least 80%, at least 85%, at least 90%, at least 95%, at least 98%, at least 99%, or 100% sequence identity to SEQ ID NO: 74;
(16) the HC comprises an amino acid sequence with at least 80%, at least 85%, at least 90%, at least 95%, at least 98%, at least 99%, or 100% sequence identity to SEQ ID NO: 71, and the LC comprises an amino acid sequence with at least 80%, at least 85%, at least 90%, at least 95%, at least 98%, at least 99%, or 100% sequence identity to SEQ ID NO: 76;
(17) the HC comprises an amino acid sequence with at least 80%, at least 85%, at least 90%, at least 95%, at least 98%, at least 99%, or 100% sequence identity to SEQ ID NO: 73, and the LC comprises an amino acid sequence with at least 80%, at least 85%, at least 90%, at least 95%, at least 98%, at least 99%, or 100% sequence identity to SEQ ID NO: 76;
(18) the HC comprises an amino acid sequence with at least 80%, at least 85%, at least 90%, at least 95%, at least 98%, at least 99%, or 100% sequence identity to SEQ ID NO: 70, and the LC comprises an amino acid sequence with at least 80%, at least 85%, at least 90%, at least 95%, at least 98%, at least 99%, or 100% sequence identity to SEQ ID NO: 76; or
(19) the HC comprises an amino acid sequence with at least 80%, at least 85%, at least 90%, at least 95%, at least 98%, at least 99%, or 100% sequence identity to SEQ ID NO: 72, and the LC comprises an amino acid sequence with at least 80%, at least 85%, at least 90%, at least 95%, at least 98%, at least 99%, or 100% sequence identity to SEQ ID NO: 76.

In some embodiments, the antibody-drug conjugate of the present invention comprises an antibody that specifically binds to mesothelin (MSLN), wherein the antibody is a chimeric antibody, a humanized antibody or a human antibody.

In some embodiments, the antibody-drug conjugate of the present invention comprises an antigen-binding fragment that specifically binds to mesothelin, wherein the antigen-binding fragment is selected from Fab, Fab', F(ab')₂, Fd, Fd', Fv, scFv, and ds-scFv.

In some embodiments, the antibody-drug conjugate of the present invention comprises an antibody or antigen-binding fragment that specifically binds to mesothelin, wherein the antibody is a monoclonal antibody, a bispecific antibody, or a multispecific antibody.

In a second aspect, the present invention provides a pharmaceutical composition comprising the antibody-drug conjugate of the present invention and optionally a pharmaceutically acceptable carrier or excipient.

In some embodiments, the composition of the present invention further comprises a second therapeutic agent, and the second therapeutic agent is selected from chemotherapeutic agents, monoclonal antibody drugs, bispecific/multispecific antibody drugs, recombinant protein drugs, nucleotide drugs (siRNA, antisense oligonucleotides), small molecule drugs, immunomodulatory drugs, and cell therapy drugs.

In a third aspect, the present invention provides a method for treating cancer in a subject, comprising administering to the subject an effective amount of the antibody-drug conjugate or pharmaceutical composition of the present invention. In some embodiments, the cancer is selected from colon cancer, mesothelioma, ovarian cancer, lung cancer, breast cancer, pancreatic cancer, gastric cancer, prostate cancer, squamous cell carcinoma, and cholangiocarcinoma. In some embodiments, the method of the present invention further comprises administering a second therapeutic agent to the subject. In some embodiments, the second therapeutic agent is selected from chemotherapeutic agents, monoclonal antibody drugs, bispecific/multispecific antibody drugs, recombinant protein drugs, nucleotide drugs (siRNA, antisense oligonucleotides), small molecule drugs, immunomodulatory drugs, and cell therapy drugs.

In a fourth aspect, the present invention provides the antibody-drug conjugate or pharmaceutical composition of the present invention for use in treating cancer. In some embodiments, the cancer is selected from colon cancer, mesothelioma, ovarian cancer, lung cancer, breast cancer, pancreatic cancer, gastric cancer, prostate cancer, squamous cell carcinoma, and cholangiocarcinoma. In some embodiments, the antibody or pharmaceutical composition of the present invention is used in combination with a second therapeutic agent. In some embodiments, the second therapeutic agent is selected from chemotherapeutic agents, monoclonal antibody drugs, bispecific/multispecific antibody drugs, recombinant protein drugs, nucleotide drugs (siRNA, antisense oligonucleotides), small molecule drugs, immunomodulatory drugs, and cell therapy drugs.

In a fifth aspect, the present invention provides the use of the antibody-drug conjugate or pharmaceutical composition of the present invention in the preparation of a drug for treating cancer. In some embodiments, the cancer is selected from colon cancer, mesothelioma, ovarian cancer, lung cancer, breast cancer, pancreatic cancer, gastric cancer, prostate cancer, squamous cell carcinoma, and cholangiocarcinoma. In some embodiments, the drug is for use in combination with a second therapeutic agent. In some embodiments, the second therapeutic agent is selected from chemotherapeutic agents, monoclonal antibody drugs, bispecific/multispecific antibody drugs, recombinant protein drugs, nucleotide drugs (siRNA, antisense oligonucleotides), small molecule drugs, immunomodulatory drugs, and cell therapy drugs.

### Description of the Figures

Figure 1. Binding of antibody PR300186 and its PTM variants PR300186-2, PR300186-3, PR300186-4, PR300186-5, PR300186-6, PR300186-7, PR300186-8, PR300186-9, and PR300186-10 to human MSLN protein.
Figure 2. Binding of antibody PR300186 and its PTM variants PR300186-2, PR300186-3, PR300186-4, PR300186-5, PR300186-6, PR300186-7, PR300186-8, PR300186-9, and PR300186-10 to cynomolgus monkey MSLN protein.
Figure 3. Binding of PR300159 and its PTM variants PR300159-1, PR300159-3, PR300159-5, PR300159-6, PR300159-8, and PR300159-9 to COV644 cells.
Figure 4. Binding of PR300186 and its PTM variants PR300186-2, PR300186-3, PR300186-4, PR300186-5, PR300186-6, PR300186-7, PR300186-8, PR300186-9 (Figure 4A) and PR300186-10 (Figure 4B) to COV644 cells.
Figure 5. Internalization of PR300159 and its PTM variants PR300159-1, PR300159-3, PR300159-4, PR300159-5, PR300159-6, PR300159-7, PR300159-8, and PR300159-9 in COV644 cells.
Figure 6. Internalization of PR300186 and its PTM variants PR300186-3, PR300186-9, and PR300186-10 in COV644 cells.
Figure 7. Internalization of PR300159-8 and PR300186-10 in COV644 cells.
Figure 8. Internalization of PR300159-8 and PR300186-10 in NCI-H226 cells.
Figure 9. Internalization of PR300159-8 and PR300186-10 in HPAC cells.
Figure 10. MSLN shedding in HT29-huMSLN wild-type 2C3 cell line and HT29-huMSLN mutant 1A11 cell line.
Figure 11. Cytotoxicity of the antibody-drug conjugate PR300159-8-B81 against HT29-huMSLN cells expressing huMSLN.
Figure 12. Stability results of PR300159-8-B81 in human, cynomolgus monkey, SD rat, and ICR mouse plasma, and in phosphate-buffered saline (PBS).
Figure 13. Stability results of PR300186-10-B81 in human, cynomolgus monkey, SD rat, and ICR mouse plasma, and in phosphate-buffered saline (PBS).
Figure 14. Tumor volumes in mice treated with varying doses of PR300159-8-B81 and PR300186-10-B81 in the HT29-huMSLN wild-type 2C3 model.
Figure 15. Body weights of mice treated with varying doses of PR300159-8-B81 and PR300186-10-B81 in the HT29-huMSLN wild-type 2C3 model.
Figure 16. Tumor volumes in mice treated with varying doses of PR300159-8-B81 and PR300186-10-B81 in the HT29-huMSLN mutant 1A11 model.
Figure 17. Body weights of mice treated with varying doses of PR300159-8-B81 and PR300186-10-B81 in the HT29-huMSLN mutant 1A11 model.
Figure 18. Tumor volumes in mice treated with varying doses of PR300159-8-B81 and PR300186-10-B81 in the COV644 model.
Figure 19. Body weights of mice treated with varying doses of PR300159-8-B81 and PR300186-10-B81 in the COV644 model.
Figure 20. Tumor volumes in mice treated with varying doses of PR300159-8-DXD, PR300159-8-B81, and PR300186-10-B81 in the OVCAR3 model.
Figure 21. Body weights of mice treated with varying doses of PR300159-8-DXD, PR300159-8-B81, and PR300186-10-B81 in the OVCAR3 model.
Figure 22. Tumor volumes in mice treated with varying doses of PR300159-8-B81 and PR300186-10-B81 in the SKOV3 model.
Figure 23. Body weights of mice treated with varying doses of PR300159-8-B81 and PR300186-10-B81 in the SKOV3 model.
Figure 24. Tumor volumes in mice treated with 1 mg/kg of PR300159-8-DXD, BAY-949343, PR300159-8-B81, and PR300186-10-B81 in the NCI-H226 model.
Figure 25. Tumor volumes in mice treated with 5 mg/kg of PR300159-8-DXD, BAY-949343, PR300159-8-B81, and PR300186-10-B81 in the NCI-H226 model.
Figure 26. Body weight changes in mice treated with 1 mg/kg of PR300159-8-DXD, BAY-949343, PR300159-8-B81, and PR300186-10-B81 in the NCI-H226 model.
Figure 27. Body weight changes in mice treated with 5 mg/kg of PR300159-8-DXD, BAY-949343, PR300159-8-B81, and PR300186-10-B81 in the NCI-H226 model.
Figure 28. Binding of PR300159-B71, PR300159, PR300186-B71, and PR300186 to CHOK-huMSLN cells.
Figure 29. Binding of PR300159-B81, PR300159, PR300186-B81, and PR300186 to COV644 cells.
Figure 30. Internalization of PR300159, PR300159-B71, PR300186, and PR300186-B71 in COV644 cells.
Figure 31. Cytotoxicity of the PR300159 and PR300159-B71 antibody-drug conjugates against CHOK1-huMSLN cells expressing huMSLN (Figure 31A); and cytotoxicity of the PR300186 and PR300186-B71 antibody-drug conjugates against CHOK1-huMSLN cells expressing huMSLN (Figure 31B).
Figure 32. Tumor volumes (Figure 32A) and body weights (Figure 32B) of mice treated with varying doses of PR300159-B71, PR300159-B81, and PR300186-B81 in the COV644 mouse tumor model.

### Detailed Description of the Invention

The following detailed description, in conjunction with the accompanying drawings, of the following embodiments provides a clearer understanding of the above-mentioned features and advantages of the present invention, and other features and advantages thereof.

The embodiments described herein by referring to the accompanying drawings are illustrative and explanatory and are intended to provide a general understanding of the present invention. The embodiments should not be construed as limiting the scope of the present invention.

### Definitions

### Chemical Definitions

The following section provides a more detailed description of specific functional groups and chemical terms.

When numerical ranges are provided, all values and subranges within the stated range are included. For example, "C₁₋₆ alkyl" includes C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

"C₁₋₆ alkyl" refers to a straight or branched saturated hydrocarbon group having 1 to 6 carbon atoms. In some embodiments, C₁₋₄ alkyl and C₁₋₂ alkyl are preferred. Examples of alkyl include: methyl (C₁), ethyl (C₂), n-propyl (C₃), isopropyl (C₃), n-butyl (C₄), tert-butyl (C₄), sec-butyl (C₄), isobutyl (C₄), n-pentyl (C₅), 3-pentyl (C₅), pentyl (C₅), neopentyl (C₅), 3-methyl-2-butyl (Cs), tert-pentyl (Cs), and n-hexyl (C₆). The term "C₁₋₆ alkyl" also includes heteroalkyl groups in which one or more (e.g., 1, 2, 3, or 4) carbon atoms are replaced by heteroatoms (e.g., oxygen, sulfur, nitrogen, boron, silicon, or phosphorus). Alkyl groups may optionally be substituted with one or more substituents, for example, substituted with 1 to 5 substituents, 1 to 3 substituents, or 1 substituent. Common alkyl abbreviations include: Me(-CH₃), Et(-CH2CH₃), iPr(-CH(CH₃)₂), nPr(-CH₂CH₂CH₃), n-Bu(-CH₂CH₂CH₂CH₃), or i-Bu(-CH₂CH(CH₃)₂).

"C₂₋₆ alkenyl" refers to a straight or branched hydrocarbon group having 2 to 6 carbon atoms and at least one carbon-carbon double bond. In some embodiments, C₂₋₄ alkenyl is preferred. Examples of C₂₋₆ alkenyl include: vinyl (C₂), 1-propenyl (C₃), 2-propenyl (C₃), 1-butenyl (C₄), 2-butenyl (C₄), butadienyl (C₄), pentenyl (Cs), pentadienyl (Cs), hexenyl (C₆), and the like. The term "C₂₋₆ alkenyl" also includes heteroalkenyl groups in which one or more (e.g., 1, 2, 3, or 4) carbon atoms are replaced by heteroatoms (e.g., oxygen, sulfur, nitrogen, boron, silicon, or phosphorus). Alkenyl groups may optionally be substituted with one or more substituents, for example, substituted with 1 to 5 substituents, 1 to 3 substituents, or 1 substituent.

"C₂₋₆ alkynyl" refers to a straight or branched hydrocarbon group having 2 to 6 carbon atoms, at least one carbon-carbon triple bond, and optionally one or more carbon-carbon double bonds. In some embodiments, C₂₋₄ alkynyl is preferred. Examples of C₂-₆ alkynyl include but are not limited to: ethynyl (C₂), 1-propynyl (C₃), 2-propynyl (C₃), 1-butynyl (C₄), 2-butynyl (C₄), pentynyl (Cs), hexynyl (C₆), and the like. The term "C₂₋₆ alkynyl" also includes heteroalkynyl groups in which one or more (e.g., 1, 2, 3, or 4) carbon atoms are replaced by heteroatoms (e.g., oxygen, sulfur, nitrogen, boron, silicon, phosphorus). Alkynyl groups may optionally be substituted with one or more substituents, for example, substituted with 1 to 5 substituents, 1 to 3 substituents, or 1 substituent.

"C₀₋₆ alkylene" refers to a chemical bond or "C₁₋₆ alkylene".

"C₁₋₆ alkylene" refers to a divalent group formed by removing another hydrogen from alkyl, and it can be substituted or unsubstituted. In some embodiments, C₁₋₄ alkylene, C₂₋₄ alkylene, and C₁₋₃ alkylene are preferred. Unsubstituted alkylenes include but are not limited to: methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), and the like. Examples of substituted alkylenes, such as those substituted with one or more alkyls (e.g., methyl), include but are not limited to: substituted methylene (-CH(CH₃)-, - C(CH₃)₂-), substituted ethylene (-CH(CH₃)CH₂-, -CH₂CH(CH₃)-, -C(CH₃)₂CH₂-, - CH₂C(CH₃)₂-), and substituted propylene (-CH(CH₃)CH₂CH₂-, -CH₂CH(CH₃)CH₂-, - CH₂CH₂CH(CH₃)-, -C(CH₃)₂CH₂CH₂-, -CH₂C(CH₃)₂CH₂-, -CH₂CH₂C(CH₃)₂-), and the like.

"Halo," or "halogen" refers to fluorine (F), chlorine (Cl), bromine (Br), and iodine (I).

Therefore, "C₁₋₆ haloalkyl" refers to the above-described "C₁₋₆ alkyl," which is substituted with one or more halogen groups. In some embodiments, C₁₋₄ haloalkyl is particularly preferred, with C₁₋₂ haloalkyl being more preferred. Exemplary haloalkyls include, but are not limited to: -CF₃, -CH₂F, -CHF₂, -CHFCH₂F, -CH₂CHF₂, -CF₂CF₃, -CCl₃, -CH₂Cl, -CHCl₂, 2,2,2-trifluoro-1,1-dimethyl-ethyl, and the like. Haloalkyl groups can be substituted at any available point of attachment, for example, with 1 to 5 substituents, 1 to 3 substituents, or 1 substituent.

"C₃₋₁₀ cycloalkyl" refers to non-aromatic cyclic hydrocarbon groups with 3 to 10 ring carbon atoms and no heteroatoms. In some embodiments, "C₃₋₁₀ cycloalkyl" is a saturated ring. In other embodiments, "C₃₋₁₀ cycloalkyl" may be an unsaturated ring, for example, containing 1, 2, 3, or more unsaturated bonds, such as alkenyl or alkynyl bonds. In some embodiments, C₃₋₇ cycloalkyl and C₃₋₆ cycloalkyl are particularly preferred, and C₅₋₆ cycloalkyl is even more preferred. The cycloalkyl also includes ring systems, wherein the cycloalkyl ring is fused with one or more aryl or heteroaryl groups, with the point of attachment being on the cycloalkyl ring, and in such cases, the number of carbons continues to represent the number of carbons in the cycloalkyl systems. Exemplary cycloalkyls include, but are not limited to: cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇), cycloheptadienyl (C₇), cycloheptatrienyl (C₇), and the like. Cycloalkyl groups may optionally be substituted with one or more substituents, for example, substituted with 1 to 5 substituents, 1 to 3 substituents, or 1 substituent.

"3-10-membered heterocyclic group" refers to a 3-10-membered non-aromatic cyclic group having ring carbon atoms and 1 to 5 ring heteroatoms, wherein each heteroatom is independently selected from nitrogen, oxygen, sulfur, boron, phosphorus, and silicon. In the heterocyclic group containing one or more nitrogen atoms, the point of attachment can be either a carbon or nitrogen atom, as long as valence permits. **In** some embodiments, a 5-10-membered heterocyclic group, which is a 3-10-membered non-aromatic cyclic system with ring carbon atoms and 1 to 5 ring heteroatoms, is preferred. **In** some embodiments, a 3-7-membered heterocyclic group, which is a 3-7-membered non-aromatic cyclic system with ring carbon atoms and 1 to 4 ring heteroatoms, is preferred; a 3-6-membered heterocyclic group, which is a 3-6-membered non-aromatic cyclic system with ring carbon atoms and 1 to 3 ring heteroatoms, is preferred; a 4-8-membered heterocyclic group, which is a 4-8-membered non-aromatic cyclic system with ring carbon atoms and 1 to 3 ring heteroatoms, is preferred; a 5-6-membered heterocyclic group, which is a 5-6-membered non-aromatic cyclic system with ring carbon atoms and 1 to 3 ring heteroatoms, is more preferred. The heterocyclic groups also include ring systems wherein the heterocyclic group ring is fused with one or more cycloalkyls, with the point of attachment being on the cycloalkyl ring, or wherein the heterocyclic group ring is fused with one or more aryls or heteroaryl groups, with the point of attachment being on the heterocyclic group ring, and in such cases, the number of ring members continues to represent the number of ring members in the heterocyclic group systems. Examples of 3-membered heterocyclic groups with one heteroatom include, but are not limited to: aziridinyl, oxiranyl, and thiiranyl (thiorenyl). Examples of 4-membered heterocyclic groups with one heteroatom include, but are not limited to: azetidinyl, oxetanyl, and thietanyl. Examples of 5-membered heterocyclic groups with one heteroatom include, but are not limited to: tetrahydrofuranyl, dihydrofuranyl, tetrahydrothiophenyl, dihydrothiophenyl, pyrrolidinyl, dihydropyrrolyl, and pyrrole-2,5-dionyl; examples of 5-membered heterocyclic groups with two heteroatoms include, but are not limited to: dioxolanyl, oxasulfuranyl, disulfanyl, and oxazolidin-2-one; examples of 5-membered heterocyclic groups with three heteroatoms include, but are not limited to: triazolanyl, oxadiazolanyl, and thiadiazolanyl. Examples of 6-membered heterocyclic groups with one heteroatom include, but are not limited to: piperidinyl, tetrahydropyranyl, dihydropyridinyl, and thianyl. Examples of 6-membered heterocyclic groups with two heteroatoms include, but are not limited to: piperazinyl, morpholinyl, dithiocyclohexanyl, and dioxanyl. Examples of 6-membered heterocyclic groups with three heteroatoms include, but are not limited to: triazinanyl. Examples of 7-membered heterocyclic groups with one heteroatom include, but are not limited to: azepanyl, oxepanyl, and thiepanyl. Examples of 5-membered heterocyclic groups fused with a C₆ aryl ring (also referred to herein as 5,6-bicyclic heterocyclic groups) include, but are not limited to: dihydroindolyl, isodihydroindolyl, dihydrobenzofuranyl, dihydrobenzothiophenyl, benzoxazolinonyl, and the like. Examples of 6-membered heterocyclic groups fused with a C₆ aryl ring (also referred to herein as 6,6-bicyclic heterocyclic groups) include, but are not limited to: tetrahydroquinolinyl, tetrahydroisoquinolinyl and the like. The heterocyclic group may optionally be substituted with one or more substituents, for example, substituted with 1 to 5 substituents, 1 to 3 substituents, or 1 substituent.

"C₆₋₁₀ aryl" refers to a group having a monocylic or polycyclic (e.g., bicyclic) 4n+2 aromatic ring system with 6 to 10 ring carbon atoms and zero heteroatoms (e.g., having 6 or 10 π-electrons shared in a cyclic arrangement). In some embodiments, the aryl has six ring carbon atoms ("C₆ aryl"; e.g., phenyl). In some embodiments, the aryl has ten ring carbon atoms ("C₁₀ aryl"; e.g., naphthyl, such as 1-naphthyl and 2-naphthyl). The aryl also includes fused ring systems in which the aryl ring is fused with one or more cycloalkyls or heterocyclic groups, with the point of attachment being on the aryl ring, and in such cases, the number of carbons continues to represent the number of carbons in the aryl ring system. The aryl group may optionally be substituted with one or more substituents, for example, substituted with 1 to 5 substituents, 1 to 3 substituents, or 1 substituent.

"5-10-membered heteroaryl group" refers to a group having a 5-10-membered monocyclic or bicyclic 4n+2 aromatic ring system with ring carbon atoms and 1 to 4 ring heteroatoms (e.g., having 6 or 10 π-electrons shared in a cyclic arrangement), wherein each heteroatom is independently selected from nitrogen, oxygen, and sulfur. In the heteroaryl groups containing one or more nitrogen atoms, the point of attachment can be either a carbon or nitrogen atom, as long as valence permits. The bicyclic heteroaryl group system can include one or more heteroatoms in one or both rings. The heteroaryl group also includes ring systems wherein the heteroaryl group ring is fused with one or more cycloalkyls or heterocyclic groups, with the point of attachment being on the heteroaryl group ring, and in such cases, the number of carbon atoms continues to represents the number of carbon atoms in the heteroaryl group ring system. In some embodiments, the "5-10-membered heteroaryl group" is a saturated ring. In other embodiments, the "5-10-membered heteroaryl group" may be an unsaturated ring, e.g., including 1, 2, 3, or more unsaturated bonds, such as alkenyl or alkynyl bonds. In some embodiments, a 5-6-membered heteroaryl group is particularly preferred, which is a 5-6-membered monocyclic or bicyclic 4n+2 aromatic ring system with ring carbon atoms and 1-4 ring heteroatoms. Examples of 5-membered heteroaryl groups with one heteroatom include, but are not limited to: pyrrolyl, furanyl, and thiophenyl. Examples of 5-membered heteroaryl groups with two heteroatoms include but are not limited to: imidazolyl, pyrazolyl, oxazolyl, isoxazolyl, thiazolyl, and isothiazolyl. Examples of 5-membered heteroaryl groups with three heteroatoms include but are not limted to: triazolyl, oxadiazolyl (e.g., 1,2,4-oxadiazolyl), and thiadiazolyl. Examples of 5-membered heteroaryl groups with four heteroatoms include but are not limited to: tetrazolyl. Examples of 6-membered heteroaryl groups with one heteroatom include but are not limited to: pyridinyl. Examples of 6-membered heteroaryl groups with two heteroatoms include but are not limited to: pyridazinyl, pyrimidinyl, and pyrazinyl. Examples of 6-membered heteroaryl groups with three or four heteroatoms respectively include but are not limited to: triazinyl and tetrazinyl. Examples of 7-membered heteroaryl groups with one heteroatom include but are not limited to: azepinyl, oxepinyl, and thiepinyl. Examples of 5,6-bicyclic heteroaryl groups include but are not limited to: indolyl, isoindolyl, indazolyl, benzotriazolyl, benzothiazolyl, isobenzothizolyl, benzofuranyl, isobenzofuranyl, benzimidazolyl, benzoxazolyl, benzisoxazolyl, benzoxadiazolyl, benzothiazolyl, benzisothiazolyl, benzothiadiazolyl, indazinyl, and purinyl. Examples of 6,6-bicyclic heteroaryl groups include but are not limited to: naphthyridinyl, pteridinyl, quinolinyl, isoquinolinyl, cinnolinyl, quinoxalinyl, phthalazinyl, and quinazolinyl. The heteroaryl group may optionally be substituted with one or more substituents, for example, substituted with 1 to 5 substituents, 1 to 3 substituents, or 1 substituent.

The divalent groups formed by removing the other hydrogen atom from groups such as the above-defined alkyls, alkenyls, alkynyls, cycloalkyls, heterocyclic groups, aryls, and heteroaryl groups are collectively referred to as "alkylenes". Cyclic groups such as cycloalkyls, heterocyclic groups, aryls, and heteroaryl groups are collectively referred to as "cyclic groups".

The alkyls, alkenyls, alkynyls, cycloalkyls, heterocyclic groups, aryls, heteroaryl groups, etc. defined herein are optionally substituted groups.

Examples of substituents on carbon atoms include but are not limited to: halogen, -CN, - NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, - SR^{aa}, -SSR^{cc}, -C(=O)R^{aa}, -CO₂H, -CHO, -C(OR^{cc})₂, -CO₂R^{aa}, -OC(=O)R^{aa}, -OCO₂R^{aa}, - C(=O)N(R^{bb})₂, -OC(=O)N(R^{bb})₂, -NR^{bb}C(=O)R^{aa}, -NR^{bb}CO₂R^{aa}, -NR^{bb}C(=O)N(R^{bb})₂,-C(=NR^{bb})R^{aa}, -C(=NR^{bb})OR^{aa}, -OC(=NR^{bb})R^{aa}, -OC(=NR^{bb})OR^{aa}, -C(=NR^{bb})N(R^{bb})₂,-OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, -SO₂N(R^{bb})₂,-SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, -C(=S)N(R^{bb})₂, - C(=O)SR^{aa}, -C(=S)SR^{aa}, -SC(=S)SR^{aa}, -SC(=O)SR^{aa}, -OC(=O)SR^{aa}, -SC(=O)OR^{aa}, - SC(=O)R^{aa}, -P(=O)₂R^{aa}, -OP(=O)₂R^{aa}, -P(=O)(R^{aa})₂, -OP(=O)(R^{aa})₂, -OP(=O)(OR^{cc})₂, - P(=O)₂N(R^{bb})₂, -OP(=O)₂N(R^{bb})₂, -P(=O)(NR^{bb})₂, -OP(=O)(NR^{bb})₂, -NR^{bb}P(=O)(OR^{cc})₂,-NR^{bb}P(=O)(NR^{bb})₂, -P(R^{cc})₂, -P(R^{cc})₃, -OP(R^{cc})₂, -OP(R^{cc})₃, -B(R^{aa})₂, -B(OR^{cc})₂, -BR^{aa}(OR^{cc}), alkyls, haloalkyls, alkenyls, alkynyls, cycloalkyls, heterocyclic groups, aryls, and heteroaryl groups, wherein each alkyl, alkenyl, alkynyl, cycloalkyl, heterocyclic group, aryl, and heteroaryl group is independently substituted with 0, 1, 2, 3, 4, or 5 R^{dd} groups;
alternatively, the two geminal hydrogens on carbon atoms are replaced by a group, such as =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
R^{aa} is each independently selected from an alkyl, a haloalkyl, an alkenyl, an alkynyl, a cycloalkyl, a heterocyclic group, aryl, and a heteroaryl group, or two R^{aa} groups combine to form a heterocyclic group or heteroaryl group ring, wherein each alkyl, alkenyl, alkynyl, cycloalkyl, heterocyclic group, aryl, and heteroaryl group is independently substituted with 0, 1, 2, 3, 4, or 5 R^{dd} groups.

R^{bb} is each independently selected from hydrogen, -OH, -OR^{aa}, -N(R^{cc})₂, -CN, - C(=O)R^{aa}, -C(=O)N(R^{cc})₂, -CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, an alkyl, a haloalkyl, an alkenyl, an alkynyl, a cycloalkyl, heterocyclic group, an aryl, and a heteroaryl group, or two R^{bb} groups combine to form a heterocyclic group or heteroaryl group ring, wherein each alkyl, alkenyl, alkynyl, cycloalkyl, heterocyclic group, aryl, and heteroaryl group is independently substituted with 0, 1, 2, 3, 4, or 5 R^{dd} groups.

R^{cc} is each independently selected from hydrogen, an alkyl, a haloalkyl, an alkenyl, an alkynyl, a cycloalkyl, a heterocyclic group, an aryl, and a heteroaryl group, or two R^{cc} groups combine to form a heterocyclic group or heteroaryl group ring, wherein each alkyl, alkenyl, alkynyl, cycloalkyl, heterocyclic group, aryl, and heteroaryl group is independently substituted with 0, 1, 2, 3, 4, or 5 R^{dd} groups.

R^{dd} is each independently selected from halogen, -CN, -NO₂, -N₃, -SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, -C(=O)R^{ee}, -CO₂H, - CO₂R^{ee}, -OC(=O)R^{ee}, -OCO₂R^{ee}, -C(=O)N(R^{ff})₂, -OC(=O)N(R^{ff})₂, -NR^{ff}C(=O)R^{ee}, - NR^{ff}CO₂R^{ee}, -NR^{ff}C(=O)N(R^{ff})₂, -C(=NR^{ff})OR^{ee}, -OC(=NR^{ff})R^{ee}, -OC(=NR^{ff})OR^{ee}, - C(=NR^{ff})N(R^{ff})₂, -OC(=NR^{ff})N(R^{ff})₂, -NR^{ff}C(=NR^{ff})N(R^{ff})₂, -NR^{ff}SO₂R^{ee}, -SO₂N(R^{ff})₂, - SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, - C(=S)SR^{ee}, -SC(=S)SR^{ee}, -P(=O)₂R^{ee}, -P(=O)(R^{ee})₂, -OP(=O)(R^{ee})₂, -OP(=O)(OR^{ee})₂, an alkyl, a haloalkyl, an alkenyl, an alkynyl, a cycloalkyl, a heterocyclic group, an aryl, and a heteroaryl group, wherein each alkyl, alkenyl, alkynyl, cycloalkyl, heterocyclic group, aryl, and heteroaryl group is independently substituted with 0, 1, 2, 3, 4, or 5 R^{gg} groups, or two geminal R^{dd} substituents may combine to form =O or =S.

R^{ee} is each independently selected from an alkyl, a haloalkyl, an alkenyl, an alkynyl, a cycloalkyl, an aryl, a heterocyclic group, and a heteroaryl group, wherein each alkyl, alkenyl, alkynyl, cycloalkyl, heterocyclic group, aryl, and heteroaryl group is independently substituted with 0, 1, 2, 3, 4, or 5 R^{gg} groups.

R^{ff} is each independently selected from hydrogen, an alkyl, a haloalkyl, an alkenyl, an alkynyl, a cycloalkyl, a heterocyclic group, an aryl, and a heteroaryl group, or two R^{ff} groups combine to form a heterocyclic group or heteroaryl group ring, wherein each alkyl, alkenyl, alkynyl, cycloalkyl, heterocyclic group, aryl, and heteroaryl group is independently substituted with 0, 1, 2, 3, 4, or 5 R^{gg} groups.

R^{gg} is each independently halogen, -CN, -NO₂, -N₃, -SO₂H, -SO₃H, -OH, -OC₁-₆ alkyl, - ON(C₁-₆ alkyl)₂, -N(C₁-₆ alkyl)₂, -N(C₁-₆ alkyl)₃⁺X⁻, -NH(C₁-₆ alkyl)₂⁺X⁻, -NH₂(C₁-₆ alkyl)⁺X⁻, -NH₃⁺X⁻, -N(OC₁-₆ alkyl)(C₁-₆ alkyl), -N(OH)(C₁-₆ alkyl), -NH(OH), -SH, -SC₁-₆ alkyl, - SS(C₁-₆ alkyl), -C(=O)(C₁-₆ alkyl), -CO₂H, -CO₂(C₁-₆ alkyl), -OC(=O)(C₁-₆ alkyl), -OCO₂(C₁-₆ alkyl), -C(=O)NH₂, -C(=O)N(C₁-₆ alkyl)₂, -OC(=O)NH(C₁-₆ alkyl), -NHC(=O)(C₁-₆ alkyl), - N(C₁-₆ alkyl)C(=O)(C₁-₆ alkyl), -NHCO₂(C₁-₆ alkyl), -NHC(=O)N(C₁-₆ alkyl)₂, - NHC(=O)NH(C₁-₆ alkyl), -NHC(=O)NH₂, -C(=NH)O(C₁-₆ alkyl), -OC(=NH)(C₁-₆ alkyl), - OC(=NH)OC₁-₆ alkyl, -C(=NH)N(C₁-₆ alkyl)₂, -C(=NH)NH(C₁-₆ alkyl), -C(=NH)NH₂, - OC(=NH)N(C₁-₆ alkyl)₂, -OC(NH)NH(C₁-₆ alkyl), -OC(NH)NH₂, -NHC(NH)N(C₁-₆ alkyl)₂, - NHC(=NH)NH₂, -NHSO₂(C₁-₆ alkyl), -SO₂N(C₁-₆ alkyl)₂, -SO₂NH(C₁-₆ alkyl), -SO₂NH₂, - SO₂C₁-₆ alkyl, -SO₂OC₁-₆ alkyl, -OSO₂C₁-₆ alkyl, -SOC₁-₆ alkyl, -Si(C₁-₆ alkyl)₃, -OSi(C₁-₆ alkyl)₃, -C(=S)N(C₁-₆ alkyl)₂, C(=S)NH(C₁-₆ alkyl), C(=S)NH₂, -C(=O)S(C₁-₆ alkyl), - C(=S)SC₁-₆ alkyl, -SC(=S)SC₁-₆ alkyl, -P(=O)₂(C₁-₆ alkyl), -P(=O)(C₁-₆ alkyl)₂, -OP(=O)(C₁-₆ alkyl)₂, -OP(=O)(OC₁-₆ alkyl)₂, C₁-₆ alkyl, C₁-₆ haloalkyl, C₂-C₆ alkenyl, C₂-C₆ alkynyl, C₃-C₇ cycloalkyl, C₆-C₁₀ aryl, a C₃-C₇ heterocyclic group, and a C₅-C₁₀ heteroaryl group, or two geminal R^{gg} substituents may combine to form =O or =S, wherein X⁻ is a counterion.

Exemplary substituents on nitrogen atoms include but are not limited to: hydrogen, - OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{bb})R^{aa}, - C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, - C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyls, haloalkyls, alkenyls, alkynyls, cycloalkyls, heterocyclic groups, aryls, and heteroaryl groups, or the two R^{cc} groups attached to nitrogen atoms combine to form a heterocyclic group or heteroaryl group ring, wherein each alkyl, alkenyl, alkynyl, cycloalkyl, heterocyclic group, aryl, and heteroaryl group is independently substituted with 0, 1, 2, 3, 4, or 5 R^{dd} groups, and wherein R^{aa}, R^{bb}, R^{cc}, and R^{dd} are as defined above.

### Other Definitions

In the description of this specification, references to the terms "an embodiment," "some embodiments," "example," "specific example," or "some examples," etc., mean that the specific features, structures, materials, or characteristics described in connection with that embodiment or example are included in at least one embodiment or example of the present invention. In this specification, the indicative expressions of the above terms do not necessarily refer to the same embodiment or example. Moreover, the specific features, structures, materials, or characteristics described can be appropriately combined in any one or more embodiments or examples. Furthermore, where there is no conflict, those skilled in the art can combine and integrate the different embodiments or examples described in this specification and the features of different embodiments or examples.

Unless otherwise specified or clearly contradicted by the context, the articles "a," "one (type)," and "the," as used herein, are intended to include "at least one" or "one or more." Thus, the articles used herein refer to one or more (i.e., at least one) objects. For example, "a component" refers to one or more components, meaning that more than one component may be considered in the implementation or use of the described embodiments.

Unless otherwise indicated or defined, the term "comprise" and its variants (such as "include" and "have") should be understood as encompassing the specified elements or steps or a group of elements or steps, but not excluding any other elements, steps, or other groups of elements or steps. The term "comprise" includes the meanings of "include" and "composed of." For example, a composition "comprising" X may exclusively consist of X, or may include additional components, for example, the composition may be composed of X+Y.

The term "approximately" concerning a numerical value x is optional and refers, for example, to x + 10% or x ± 5%.

"Steroisomers" refer to compounds with the same chemical structure but different spatial arrangements of atoms or groups. Stereoisomers include enantiomers, diastereomers, conformational isomers (rotamers), geometric isomers (cis/trans isomers), atropisomers, etc.

"Chirality" refers to molecules that cannot be superposed onto their mirror images, whereas "achirality" refers to molecules that can be superposed onto their mirror images.

"Enantiomers" refer to two isomers of a compound that cannot be superposed but mirror each other.

"Diastereomers" refer to stereoisomers that have two or more chiral centers and have molecules that do not mirror each other. Diastereomers exhibit different physical properties, such as melting points, boiling points, spectral properties, and reactivity. Diastereomeric mixtures can be separated using high-resolution analytical techniques such as electrophoresis and chromatography, for example, HPLC.

The stereochemical definitions and rules used in the present invention generally follow those outlined in S.P. Parker, Ed., McGraw-Hill Dictionary of Chemical Terms (1984), McGraw-Hill Book Company, New York, and Eliel, E. and Wilen, S., "Stereochemistry of Organic Compounds," John Wiley & Sons, Inc., New York, 1994.

Many organic compounds exist in optically active forms, meaning they have the ability to rotate the plane of plane-polarized light. When describing optically active compounds, the prefixes D and L or R and S are used to denote the absolute configuration of the molecule around one or more of its chiral centers. The prefixes *d* and *l,* or (+) and (-), are symbols used to indicate the rotation of plane-polarized light induced by the compound, wherein (-) or *l* indicates that the compound is levorotatory. A compound with the prefix (+) or d is dextrorotatory. A specific type of stereoisomer is an enantiomer, and a mixture of enantiomers is referred to as an enantiomeric mixture. A 50:50 mixture of enantiomers is called a racemic mixture or racemate, and this can occur when there is no stereoselectivity or stereospecificity in a chemical reaction or process.

The present invention discloses that any asymmetric atom (e.g., carbon) in the compounds can exist in racemic or enantiomer-enriched forms, such as (*R*)-, (*S*)-, or (*R, S*)-configurations. In certain embodiments, each asymmetric atom exhibits at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess in terms of (*R*)- or (*S*)-configuration.

Depending on the choice of starting materials and methods, the compounds of the present invention may exist as one of the possible isomers or mixtures thereof, such as racemates and diastereomeric mixtures (depending on the number of asymmetric carbon atoms). Optically active (R)- or (S)-isomers can be prepared using chiral auxiliaries or chiral reagents, or separated using conventional techniques. If the compound contains a double bond, the substituents may exhibit an E or Z configuration; if the compound contains disubstituted cycloalkyls, the substituents on the cycloalkyl may have cis or trans configurations.

Any mixture of stereoisomers obtained can be separated into pure or substantially pure geometric isomers, enantiomers, and diastereomers based on differences in the physicochemical properties of their components, for example, through chromatography and/or stepwise crystallization methods.

Racemates mixtures of any obtained final product or intermediate can be split into optical enantiomers using methods known to those skilled in the art, such as by separating the obtained diastereomeric salts thereof. Racemic products can also be separated using chiral chromatography, such as high-performance liquid chromatography (HPLC) that uses a chiral adsorbent. Specifically, enantiomers can be prepared through asymmetric synthesis by referring, for example, to Jacques, et al., Enantiomers, Racemates, and Resolutions (Wiley Interscience, New York, 1981); Principles of Asymmetric Synthesis (2nd Ed., Robert E. Gawley, Jeffrey Aubé, Elsevier, Oxford, UK, 2012); Eliel, E.L. Stereochemistry of Carbon Compounds (McGraw-Hill, NY, 1962); Wilen, S.H., Tables of Resolving Agents and Optical Resolutions, p. 268 (E.L. Eliel, Ed., University of Notre Dame Press, Notre Dame, IN 1972); and Chiral Separation Techniques: A Practical Approach (Subramanian, G. Ed., Wiley-VCH Verlag GmbH & Co. KGaA, Weinheim, Germany, 2007).

The term "tautomer" or "tautomeric form" refers to structural isomers with different energies that can interconvert via a low energy barrier. If tautomerism is possible (e.g., in a solution), a chemical equilibrium between tautomers can be established. For example, protontautomers (also referred to as prototropic tautomers) involve interconversion through proton transfer, such as keto-enol tautomerism and imine-enamine tautomerism. Valence tautomers involve interconversion through the rearrangement of some bonding electrons. A specific example of keto-enol tautomerism is the interconversion between pentane-2,4-dione and 4-hydroxy-pent-3-en-2-one tautomers. Another example of tautomerism is phenol-ketone tautomerism. A specific example of phenol-ketone tautomerism is the interconversion between pyridin-4-ol and pyridin-4(1H)-one tautomers. Unless otherwise specified, all tautomeric forms of the compounds of the present invention are within the scope of the present invention.

The term "pharmaceutically acceptable salts" as used in the present invention refers to both organic and inorganic salts of the compounds described herein. Pharmaceutically acceptable salts are well known in the art, as detailed in S. M. Berge et al., Journal of Pharmaceutical Sciences, 1977, 66: 1-19. Salts formed by non-toxic pharmaceutically acceptable acids include, but are not limited to, inorganic acid salts formed by reaction with an amino group, such as hydrochlorides, hydrobromides, phosphates, sulfates, and perchlorates, and organic acid salts formed in this way, such as acetates, oxalates, maleates, tartrates, citrates, succinates, and malonates, or salts obtained by other methods described in books and literature, such as ion exchange methods. Other pharmaceutically acceptable salts include adipates, alginates, ascorbates, aspartates, benzenesulfonates, benzoates, bisulfates, borates, butyrates, camphorates, camphorsulfonates, cyclopentylpropionates, digluconates, dodecylsulfates, ethanesulfonates, formates, fumarates, gluceptates, glycerophosphates, gluconates, hemisulfates, heptanoates, hexanoates, hydroiodides, 2-hydroxyethanesulfonates, lactobionates, lactates, laurates, laurylsulfates, malates, malonates, methanesulfonates, 2-naphthalenesulfonates, nicotinates, nitrates, oleates, palmitates, pamoates, pectates, persulfates, 3-phenylpropionates, picrates, pivelates, propionates, stearates, thiocyanates, p-toluenesulfonates, undecanoates, valerates, and others. Salts obtained by means of appropriate bases include alkali metals, alkaline earth metals, ammonium, and N⁺(C₁-C₄ alkyl)₄ salts. The present invention also puts forward quaternary ammonium salts formed by compounds of any group containing N. Water-soluble or oil-soluble or dispersible products can be obtained through quaternization. Alkali or alkaline earth metal salts include sodium, lithium, potassium, calcium, magnesium, etc. Pharmaceutically acceptable salts further include suitable non-toxic ammonium, quaternary ammonium salts, and ammonium cations formed by counterions, e.g., halides, hydroxides, carboxylates, sulfates, phosphates, nitrates, C₁-₈ sulfonates, and aromatic sulfonates.

Unless otherwise indicated or defined, all terms used herein have their commonly understood meanings in the field, which are clear to those skilled in the art. For example, reference may be made to standard manuals such as Leuenberger, H.G.W, Nagel, B., and K1bl, H. eds., "A Multilingual Glossary of Biotechnological Terms: (IUPAC Recommendations)," Helvetica Chimica Acta (1995), CH-4010 Basel, Switzerland; Sambrook et al., "Molecular Cloning: A Laboratory Manual" (2nd Ed.), Vols. 1-3, Cold Spring Harbor Laboratory Press (1989); F. Ausubel et al., eds., "Current Protocols in Molecular Biology," Green Publishing and Wiley InterScience, New York (1987); Roitt et al., "Immunology" (6th Ed.), Mosby/Elsevier, Edinburgh (2001); and Janeway et al., "Immunobiology" (6th Ed.), Garland Science Publishing/Churchill Livingstone, New York (2005).

The term "subject" as used in the present invention refers to an animal. Typically, the animal is a mammal. For example, the subject may refer to primates (e.g., humans, male or female), oxen, sheep, goats, horses, dogs, cats, rabbits, rats, mice, fish, birds, and the like. In certain embodiments, the subject is a primate. In other embodiments, the subject is a human.

The term "patient" as used in the present invention refers to a human (including adults and children) or other animals. In some embodiments, the term "patient" means a human.

The terms "antibody-drug conjugate," "antibody-drug conjugate," "antibody conjugate," "conjugate," "immunoconjugate," and "ADC" are used interchangeably to refer to an antibody or its antigen-binding fragment covalently linked to a drug. The drug is typically a small molecule drug, toxin, cytotoxic agent, therapeutic agent, and the like.

As used herein, the term "antibody" refers to an immunoglobulin molecule capable of specifically binding to a particular antigen. Antibodies typically include heavy chains and light chains, with the heavy chain comprising a heavy chain variable region and a heavy chain constant region, and the light chain comprising a light chain variable region and a light chain constant region. The variable regions of the heavy and light chains of the antibody contain binding domains that interact with the antigen. The constant region of the antibody can mediate binding of immunoglobulin to host tissues or factors, the host tissues or factors including various cells of the immune system (e.g., effector cells) and components of the complement system (e.g., C1q). Most antibodies have a heavy chain variable region (VH) and a light chain variable region (VL), which together form the part of the antibody that binds to the antigen.

The "light chain variable region" (VL) and "heavy chain variable region" (VH) both comprise four "framework" regions separated by three "complementarity-determining regions" or "CDRs." The framework regions align the CDRs to allow for specific binding of the CDRs to antigen epitopes. The CDRs include the amino acid residues primarily responsible for antigen binding. From the amino terminus to the carboxyl terminus, the VL and VH domains both contain the following framework (FR) regions and CDR regions: FR1, CDR1, FR2, CDR2, FR3, CDR3, and FR4. The CDR1, CDR2, and CDR3 of the VL domain are also referred to herein as LCDR1, LCDR2, and LCDR3, respectively; the CDR1, CDR2, and CDR3 of the VH domain are referred to herein as HCDR1, HCDR2, and HCDR3, respectively.

The assignment of amino acids for each VL and VH domain follows any conventional definition of CDRs. Conventional definitions include the Kabat definition (Kabat, Sequences of Proteins of Immunological Interest (National Institutes of Health, Bethesda, MD, 1987 and 1991)), or the Chothia definition (Chothia & Lesk, Journal of Molecular Biology, 196:901-917, 1987; Chothia et al., Nature, 342:878-883, 1989); composites of Chothia-Kabat CDRs (also referred to as combined Chothia and Kabat CDRs), wherein each CDR is a composite of a Chothia CDR and a Kabat CDR; the AbM definition used by Oxford Molecular antibody modeling software; and the contact definition by Martin et al. (for the aforementioned conventional CDR definition systems, see world wide web bioinfo.org.uk/abs). Kabat provides a widely used numbering system (the Kabat numbering system), wherein the corresponding residues between different heavy chains or between different light chains are assigned the same number.

The present invention involves CDRs defined according to any of these numbering systems, although preferred embodiments involve CDRs defined by the combined Chothia and Kabat definitions.

Table 1. CDR Definition Methods for Antibodies of the Present Invention (see http://bioinf.org.uk/abs/)

| | Kabat | Chothia | Combined |
|---|---|---|---|
| LCDR1 | L24-L34 | L24-L34 | L24-L34 |
| LCDR2 | L50-L56 | L50-L56 | L50-L56 |
| LCDR3 | L89-L97 | L89-L97 | L89-L97 |
| HCDR1 | H31-H35 | H26-H32 | H26-H35 |
| HCDR2 | H50-H65 | H52-H56 | H50-H65 |
| HCDR3 | H95-H102 | H95-H102 | H95-H102 |

In Table 1, Laa-Lbb may refer to the amino acid sequence from position aa (based on the Chothia numbering system) to position bb (based on the Chothia numbering system) starting from the N-terminus of the antibody light chain; Haa-Hbb may refer to the amino acid sequence from position aa (based on the Chothia numbering system) to position bb (based on the Chothia numbering system) starting from the N-terminus of the antibody heavy chain. For example, L24-L34 may refer to the amino acid sequence from position 24 to position 34 starting from the N-terminus of the antibody light chain (based on the Chothia numbering system); H26-H32 may refer to the amino acid sequence from position 26 to position 32 starting from the N-terminus of the antibody heavy chain (based on the Chothia numbering system).

The term "antibody" as used herein should be understood in its broadest sense, encompassing monoclonal antibodies (including full-length monoclonal antibodies), polyclonal antibodies, antibody fragments, and multispecific antibodies containing at least two different antigen-binding regions (e.g., bispecific antibodies). Antibodies may include additional modifications, such as non-natural amino acids, mutations in the Fc region, and mutations at glycosylation sites. Antibodies can also include post-translationally modified antibodies, fusion proteins containing antibody antigenic determinants, and any other immunoglobulin molecules with modifications to the antigen recognition site, as long as the antibodies exhibit the desired biological activity.

As used herein, the term "antigen-binding fragment" of an antibody refers to one or more fragments of an antibody that retain the ability to specifically bind to an antigen (e.g., MSLN). It has been demonstrated that fragments of full-length antibodies can achieve the antigen-binding function of the antibodies.

Examples of antigen-binding fragments encompassed by the term "antigen-binding portion" of an antibody include: (i) Fab fragments, monovalent fragments composed of VL, VH, CL, and CH1 structure domains; (ii) F(ab')₂ fragments, divalent fragments composed of two Fab fragments connected by a disulfide bond in the hinge region; (iii) Fab' fragments, which essentially are Fab with partial hinge regions (see FUNDAMENTAL IMMUNOLOGY (Paul ed., 3rd ed., 1993)); (iv) Fd fragments, composed of VH and CH1 structure domains; (v) Fd' fragments, composed of VH and CH1 structure domains along with one or more cysteine residues at the C-terminus of the CH1 structure domain; (vi) Fv fragments, composed of VL and VH structure domains of a single antibody arm; (vii) dAb fragments (Ward et al., (1989), Nature, 341:544-546), composed of a VH structure domain; (viii) isolated complementarity-determining regions (CDRs); and (ix) nanobodies, which include a single heavy chain region with a variable region and two constant regions. Additionally, although the two structure domains of an Fv fragment, VL and VH, are encoded by separate genes, they can be connected using recombinant methods through a synthetic linker, the synthetic linker allowing them to form a single protein chain in which the VL and VH regions pair to create a monovalent molecule (referred to as a single-chain Fv (scFv)); see, e.g., Bird et al. (1988), Science, 242:423-426; and Huston et al. (1988), Proceedings of the National Academy of Sciences, USA, 85:5879-5883). Such single-chain antibodies are also included in the term "antigen-binding fragment" of an antibody. Furthermore, this term also includes "linear antibodies," which comprise a pair of tandem Fd fragments (VH-CH1-VH-CH1) forming an antigen-binding region together with complementary light-chain polypeptides, and any modified forms of the aforementioned fragments that retain antigen-binding activity.

These antigen-binding fragments can be obtained using conventional techniques known to those skilled in the art and screened for use in the same manner as intact antibodies.

As used herein, the term "binding" or "specific binding" refers to a non-random binding reaction between two molecules, such as the non-random binding reaction between an antibody and its target antigen. The binding specificity of an antibody can be determined based on affinity and/or avidity. Affinity is represented by the equilibrium dissociation constant (K_{D}) of the antigen and antibody and is a measure of the binding strength between the antigenic determinant (epitope) and the antigen-binding site on the antibody: the smaller the K_{D} value, the stronger the binding between the antigenic determinant (epitope) and the antibody. Alternatively, affinity can also be expressed as the affinity constant (KA), which is 1/K_{D}.

Avidity is a measure of the binding strength between an antibody and its related antigen. Avidity is related to the affinity between the antigenic determinant (epitope) and its antigen-binding site on the antibody and to the number of related binding sites present on the antibody. Typically, antibodies bind with a K_{D} in the range of 10⁻⁵ to 10⁻¹² M or lower, preferably 10⁻⁷ to 10⁻¹² M or lower, more preferably 10⁻⁸ to 10⁻¹² M, and/or with a binding affinity of at least 10⁷ M⁻¹, preferably at least 10⁸ M⁻¹, more preferably at least 10⁹ M⁻¹, and for example, at least 10¹² M⁻¹. Any K_{D} value greater than 10⁻⁴ M is generally considered nonspecific binding. The specific binding of an antibody to an antigen or antigenic determinant can be determined using any suitable known detection method, including, for example, Scatchard analysis and/or competitive binding assays such as radioimmunoassay (RIA), enzyme immunoassay (EIA), biolayer interferometry (BLI), and sandwich competitive assays, and their various variants known in the field.

The term "kₒₙ" or "kₐ" refers to the association rate constant of an antibody associating with an antigen to form an antibody/antigen complex. This rate can be determined using standard assays, such as Biacore or ELISA.

The term "k_{off}" or "kₐ" refers to the dissociation rate constant of an antibody dissociating from an antibody/antigen complex. This rate can be determined using standard assays, such as Biacore or ELISA.

The term "K_{D}" refers to the equilibrium dissociation constant of a specific antibody-antigen interaction. K_{D} is calculated as kₐ/k_{d}. The rate can be measured using standard assays, such as Biacore or ELISA. The antibody or its antigen-binding fragment in the conjugates of the present application can bind to the target antigen with an equilibrium dissociation constant of K_{D} ≤ 1 mM, ≤ 100 nM, or ≤ 10 nM, or any value within these ranges.

As used herein, the term "sequence identity" refers to the degree to which two sequences (amino acids) have identical residues at the same positions in the alignment. For example, "an amino acid sequence is X% identical to SEQ ID NO: Y" means that the amino acid sequence has X% identity with SEQ ID NO: Y, specifically indicating that X% of the residues in the amino acid sequence are identical to the residues in the sequence disclosed in SEQ ID NO: Y. Typically, computer programs are used for such calculations. Exemplary programs for comparing and aligning sequence pairs include ALIGN (Myers and Miller, 1988), FASTA (Pearson and Lipman, 1988; Pearson, 1990), gapped BLAST (Altschul et al., 1997), BLASTP, BLASTN, or GCG (Devereux et al., 1984).

Moreover, when determining the degree of sequence identity between two amino acid sequences, those skilled in the art may consider so-called "conservative" amino acid substitutions, which are typically described as amino acid substitutions in which one amino acid residue is substituted with another amino acid having a similar chemical structure, which causes little or substantially no impact on the function, activity, or other biological properties of the polypeptide. Such conservative amino acid substitutions are well known in the art.

Particularly preferred conservative substitutions are as follows: substituting Ala with Gly or Ser; substituting Arg with Lys; substituting Asn with Gln or His; substituting Asp with Glu; substituting Cys with Ser; substituting Gln with Asn; substituting Glu with Asp; substituting Gly with Ala or Pro; substituting His with Asn or Gln; substituting Ile with Leu or Val; substituting Leu with Ile or Val; substituting Lys with Arg, Gln, or Glu; substituting Met with Leu, Tyr, or Ile; substituting Phe with Met, Leu, or Tyr; substituting Ser with Thr; substituting Thr with Ser; substituting Trp with Tyr; substituting Tyr with Trp; and/or substituting Phe with Val, Ile, or Leu.

The term "MSLN antibody," "anti-MSLN antibody," or "antibody that specifically binds to MSLN" refers to any form of antibody or a fragment thereof that specifically binds to MSLN and covers monoclonal antibodies (including full-length monoclonal antibodies), polyclonal antibodies, and biologically functional antibody fragments, as long as the fragment specifically binds to MSLN.

As used herein with reference to an antibody or antigen-binding fragment, "internalization" refers to the ability of the antibody or antigen-binding fragment to pass through the lipid bilayer membrane of a cell into an internal compartment (i.e., "internalized") upon binding to the cell, preferably into a degradative compartment in the cell.

The term "therapeutic agent," "drug," or "drug moiety" refers to an agent capable of modulating a biological process and/or exhibiting biological activity.

The term "cytotoxic agent" refers to a substance that induces cell death primarily by interfering with the expression activity and/or function of a cell. Examples of cytotoxic agents include (but are not limited to) antimitotic agents such as eribulin and auristatins (e.g., monomethyl auristatin E (MMAE)).

The term "antibody : drug ratio" or "drug-to-antibody ratio" or "DAR" refers to the number of drug moieties conjugated to each antibody, i.e., the drug payload.

As used herein, the term "treatment" refers to the administration of an agent or the execution of a procedure to achieve an effect. This action may be prophylactic, to completely or partially prevent a disease or its symptoms, and/or therapeutic, to achieve the partial or complete cure of a disease and/or its symptoms. As used herein, "treatment" may include the treatment of diseases or disorders (e.g., cancer) in mammals, particularly humans, and encompasses: (a) preventing the occurrence of a disease or disease symptoms in a subject who is predisposed to the disease but has not yet been diagnosed with it (e.g., including diseases that may be associated with or caused by a primary disease); (b) suppressing the disease, i.e., hindering its progression; and (c) alleviating the disease, i.e., causing its regression. Treatment may refer to any measure of success in cancer therapy, improvement, or prevention, including any objective or subjective parameters such as remission, symptom relief, or improving the patient's ability to endure the disease condition. Thus, the term "treatment" includes the administration of the ADCs or compositions disclosed herein to prevent, delay, alleviate, stop, or inhibit the development of symptoms or conditions associated with a disease (e.g., cancer). The term "therapeutic effect" refers to the reduction, elimination, or prevention of a subject's disease, disease symptoms, or side effects of the disease.

The term "cancer" refers to a pathological condition characterized by unregulated cell growth in a group of cells within a mammal.

"Tumor-associated antigen" refers to an antigen that is differentially expressed in cancer cells compared to normal cells. Tumor-associated antigens can therefore be used to target cancer cells.

"Pharmaceutical composition" refers to a formulation that allows for administration and subsequently provides the desired biological activity of the active ingredient and/or achieves a therapeutic effect without containing other components that have toxicity levels unacceptable for administration to the subject. Pharmaceutical compositions may be sterile.

The term "pharmaceutical excipient" includes substances such as adjuvants, carriers, pH adjusters and buffering agents, tonicity adjusters, wetting agents, and preservatives.

The term "pharmaceutically acceptable" refers to a substance that, within the bounds of sound medical judgment, is suitable for contact with human and lower animal tissues without causing excessive toxicity, irritation, allergic reactions, or other adverse effects, and is commensurate with reasonable benefit-to-risk ratios.

As disclosed herein, an "effective amount" of the ADC is an amount sufficient to achieve the specifically described purposes, such as producing a therapeutic effect after administration, for example, reducing the rate of tumor growth or tumor volume, alleviating cancer symptoms, or some other signs of therapeutic efficacy. An effective amount can be determined in a conventional manner with respect to the stated purposes. The term "therapeutically effective amount" refers to the amount of the ADC effective in treating a subject's disease or condition. In the case of cancer, a therapeutically effective amount of the ADC may reduce the number of cancer cells, decrease tumor size, inhibit (e.g., slow down or stop) tumor metastasis, inhibit (e.g., slow down or stop) tumor growth, and/or alleviate one or more symptoms. A "prophylactically effective amount" refers to the amount necessary to effectively achieve a preventative outcome within the required dosage and time period. Typically, because prophylactic doses are used in a subject before the onset of disease or in the early stages of disease, the prophylactically effective amount will be less than the therapeutically effective amount.

Some embodiments of the present invention are now described in greater detail, with examples illustrated by the accompanying structural formulas and chemical formulas. The invention is intended to encompass all alternatives, modifications, and equivalent technical solutions, all of which are included within the scope of the present invention as defined by the claims. Those skilled in the art will recognize that numerous methods and materials similar or equivalent to those described herein can be used to implement the present invention. The present invention is by no means limited to the methods and materials described herein. In cases where one or more incorporated pieces of literature, patents, or similar materials differ from or conflict with the present application (including but not limited to definitions of terms, term uses, described techniques, etc.), the present application shall prevail.

It should further be understood that certain features of the present invention, which are described in the context of separate embodiments for clarity, can also be provided in combination within a single embodiment. Conversely, various features of the present invention, which are described in the context of a single embodiment for brevity, can also be provided separately or in any suitable sub-combination.

Unless otherwise stated, all technical terms used in the present invention have the same meaning as commonly understood by those skilled in the art to which the present invention relates. All patents and publications referenced in the present invention are incorporated herein in their entirety by reference.

Unless otherwise specified, the following definitions should apply as used herein. For the purposes of the present invention, chemical elements align with the CAS version of the Periodic Table of Elements and the Handbook of Chemistry and Physics, 75th Edition, 1994. Additionally, for the general principles of organic chemistry, reference can be made to "Organic Chemistry", Thomas Sorrell, University Science Books, Sausalito: 1999, and "March's Advanced Organic Chemistry" by Michael B. Smith and Jerry March, John Wiley & Sons, New York: 2007, the entire contents of which are incorporated herein by reference.

### Specific Embodiments

The following embodiments are provided to further illustrate the present invention. It should be understood that these embodiments are provided solely for the purpose of exemplifying the present invention and are not intended to limit the scope of the present invention.

In one embodiment, the present invention relates to an MSLN antibody-drug conjugate, which is a compound of formula (I) or a tautomer, stereoisomer, or pharmaceutically acceptable salt thereof:

Ab-[S-X-(Y)n-Z-E-D]q (I),

wherein,
Ab is an antibody that specifically binds to mesothelin (MSLN) or an antigen-binding fragment thereof;
S is a sulfur bond linking Ab and X;
X is selected from C₃-₁₀ cycloalkyl, a 5-10-membered heterocyclic group, C₆₋₁₀ aryl, or a 5-10-membered heteroaryl group;
X is optionally substituted with 1, 2, 3, 4, or 5 instance(s) of R*;
R* is selected from H, deuterium, halogen, CN, NO₂, OH, C₁-₆ alkyl, C₁-₆ haloalkyl, C₂-₆ alkenyl, or C₂-₆ alkynyl;
Y is -Y₁-Y₂-Y₃-, Y being linked to X via Y₁ and to Z via Y₃,
wherein,
Y₁ is selected from -C(O)-, -C(O)O-, -OC(O)-, -C(O)NH-, C₂-₆ alkenyl, C₂-₆ alkynyl, a 3-10-membered cycloalkyl, a 5-10-membered heterocyclic group, C₆₋₁₀ aryl, or a 5-10-membered heteroaryl group;
Y₂ is a bond or C₁-₆ alkylene;
Y₃ is selected from -C(O)-, -OC(O)-, or -NHC(O)-;
Y is optionally substituted with 1, 2, 3, 4, or 5 instance(s) of R#;
R# is selected from H, deuterium, halogen, CN, NO₂, OH, C₁-₆ alkyl, C₁-₆ haloalkyl, C₂-₆ alkenyl, or C₂-₆ alkynyl;
Z is a peptide containing 2 to 8 amino acids, preferably a dipeptide, tripeptide, or tetrapeptide;
E is absent or is a spacer unit optionally linking Z and D;
D is a biologically active molecule drug or a derivative thereof;
n is selected from 0, 1, 2, 3, 4, or 5, preferably 0, 1, 2, or 3;
q is an integer selected from 1 to 10, preferably 8.

### Ab

In one specific embodiment, Ab is an MSLN antibody. In another specific embodiment, Ab is an MSLN antigen-binding fragment.

### X

In one specific embodiment, X is C₃-₁₀ cycloalkyl; in another specific embodiment, X is a 5-10-membered heterocyclic group; in another specific embodiment, X is C₆₋₁₀ aryl; in another specific embodiment, X is a 5-10-membered heteroaryl group; in another specific embodiment, X is a 5-6-membered heteroaryl group.

In one specific embodiment, X is pyrimidinyl, for example,

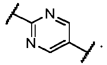

In one specific embodiment, X is unsubstituted; in another specific embodiment, X is substituted with one instance of R*; in another specific embodiment, X is substituted with two instances of R*; in another specific embodiment, X is substituted with three instances of R*; in another specific embodiment, X is substituted with four instances of R*; and in another specific embodiment, X is substituted with five instances of R*.

### Y

In one specific embodiment, Y is -Y₁-Y₂-Y₃-, Y being linked to X via Y₁ and to Z via Y₃.

In another specific embodiment, Y is

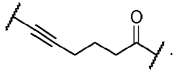

In one specific embodiment, Y is unsubstituted; in another specific embodiment, Y is substituted with one instance of R#; in another specific embodiment, Y is substituted with two instances of R#; in another specific embodiment, Y is substituted with three instances of R#; in another specific embodiment, Y is substituted with four instances of R#; in another specific embodiment, Y is substituted with five instances of R#.

### Y₁

In one specific embodiment, Y₁ is -C(O)-; in another specific embodiment, Y₁ is - C(O)O-; in another specific embodiment, Y₁ is -OC(O)-; in another specific embodiment, Y₁ is -C(O)NH-; in another specific embodiment, Y₁ is C₂-₆ alkenyl; in another specific embodiment, Y₁ is C₂-₄ alkenyl; in another specific embodiment, Y₁ is C₂-₆ alkynyl; in another specific embodiment, Y₁ is C₂-₄ alkynyl; in another specific embodiment, Y₁ is C₃-₁₀ cycloalkyl; in another specific embodiment, Y₁ is a 5-10-membered heterocyclic group; in another specific embodiment, Y₁ is C₆₋₁₀ aryl; in another specific embodiment, Y₁ is a 5-10-membered heteroaryl group.

### Y₂

In one specific embodiment, Y₂ is a bond; in another specific embodiment, Y₂ is C₁-₆ alkylene; in another specific embodiment, Y₂ is C₁-₄ alkylene.

### Y₃

In one specific embodiment, Y₃ is -C(O)-; in another specific embodiment, Y₃ is - OC(O)-; in another specific embodiment, Y₃ is -NHC(O)-.

### Z

In one specific embodiment, Z is a linker peptide containing a peptide of 2 to 8 amino acids; preferably, it is a dipeptide, tripeptide, or tetrapeptide.

In some embodiments, Z is a cleavable peptide. In some embodiments, the cleavable peptide can be cleaved by enzymes. In some embodiments, the cleavable peptide can be cleaved by tumor-associated proteases. In some embodiments, the cleavable peptide can be cleaved by lysosomal enzymes. In some embodiments, the cleavable peptide can be cleaved by cathepsins. In some embodiments, the cleavable peptide can be cleaved by lysosomal cysteine cathepsins (e.g., cathepsin B, C, F, H, K, L, O, S, V, X, or W). In some embodiments, the cleavable peptide can be cleaved by cathepsin B. An exemplary dipeptide cleavable by cathepsin B is valine-citrulline (Val-Cit) (Dubowchik et al., (2002), Bioconjugate Chemistry, 13:855-69).

In one specific embodiment, Z is selected from val-cit, val-cit-gly, gly-gly, gly-gly-gly, gly-gly-gly-gly, val-gly-gly, val-gln-gly, val-glu-gly, phe-lys-gly, leu-lys-gly, gly-val-lys-gly, val-lys-gly-gly, val-lys-gly, val-lys-ala, val-lys-leu, leu-leu-gly, gly-gly-phe-gly, gly-gly-phe-gly-gly, val-gly, val-lys-β-ala, or the aforementioned dipeptides, tripeptides, and tetrapeptides, after undergoing modification by means of substitution.

In one specific embodiment, Z is 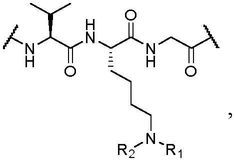 wherein R₁ and R₂ are independently selected from H, deuterium, C₁-₆ alkyl, C₁-₆ haloalkyl, C₂-₆ alkenyl, or C₂-₆ alkynyl.

In one specific embodiment, Z is 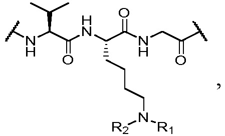 wherein R₁ and R₂ are independently selected from H, deuterium, halogen, or C₁-₆ alkyl, preferably H or C₁-₄ alkyl.

### E

E is absent or is a spacer unit optionally connecting Z and D; E is absent or selected from -NH-C₁-₆ alkylene- or -O-C₁-₆ alkylene-, wherein the -NH-C₁-₆ alkylene- and -O-C₁-₆ alkylene- may optionally be substituted with one, two, or three substituents selected from H, halogen, C₁-₆ alkyl, and C₁-₆ haloalkyl.

Preferably, E is absent or is -NH-C₁-₄ alkylene-, wherein the -NH-C₁-₄ alkylene- may optionally be substituted with one, two, or three substituents selected from H, halogen, C₁-₄ alkyl, and C₁-₄ haloalkyl.

More preferably, E is -NH-(CH₂)₁-₄-, for example, -NH-CH₂-.

In some embodiments, E may serve as a spacer unit connecting Z and D. The spacer may be a functional group that facilitates the attachment of the X-Y-Z to a camptothecin derivative or provide additional structural components to further facilitate the release of the camptothecin derivative from the conjugate.

### D

In one specific embodiment, D is a biologically active molecule drug or a derivative thereof.

In one specific embodiment, D is selected from: metal complexes; glycopeptide antibiotics; DNA topoisomerase inhibitors; drugs that interfere with DNA synthesis; drugs targeting structural proteins; tumor signaling pathway inhibitors; proteasome inhibitors; histone deacetylase inhibitors; tumor angiogenesis inhibitors; cyclin inhibitors; serine/threonine kinase inhibitors, tyrosine kinase inhibitors, aspartate kinase inhibitors, or histidine kinase inhibitors; and other active substances that inhibit tumor cell growth, or promote tumor cell apoptosis or induce necrosis.

In one specific embodiment, D is selected from: oxaliplatin; bleomycin or pingyangmycin; camptothecin, camptothecin derivatives (e.g., hydroxycamptothecin, 9-aminocamptothecin, etc.), SN-38, irinotecan, topotecan, belotecan, or rubitecan; actinomycin D, doxorubicin, duocarmycin, daunorubicin, mitoxantrone, podophyllotoxin, or etoposide; methotrexate, 5-fluorouracil, cytarabine, gemcitabine, mercaptopurine, pentostatin, fludarabine, cladribine, or nelarabine; vinca alkaloids, vincristine, vinblastine, paclitaxel, docetaxel, or cabazitaxel; maytansinoid derivatives; calicheamicin derivatives; auristatin derivatives; pyrrolobenzodiazepine dimer derivatives; melphalan; mitomycin C; or chlorambucil.

In one specific embodiment, D is 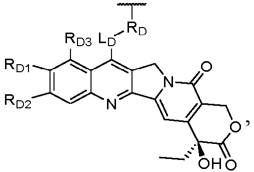 wherein,
R_{D} is absent or selected from -NH- or -O-;
L_{D} is selected from -C₀₋₆alkylene-O-C₀₋₆alkylene- or -C₀₋₆alkylene-O-C₀₋₆alkylene-C(O)NH-CHR_{D4}-;
R_{D1} and R_{D2} are independently selected from H, halogen, alkyl, haloalkyl or alkoxy; or, R_{D1} and R_{D2}, together with the carbon atom to which they are connected, form C₃₋₇ cycloalkyl or a 3-7-membered heterocyclic group, preferably a 5-6-membered heterocyclic group;
R_{D3} and R_{D4} are independently selected from H, halogen, alkyl, haloalkyl or alkoxy; or R_{D3} and R_{D4}, together with the carbon atom to which they are connected, form C₅₋₁₀ cycloalkyl or a C₅₋₁₀ heterocyclic group, preferably C₅₋₆ cycloalkyl.

In a specific embodiment, D is selected from:

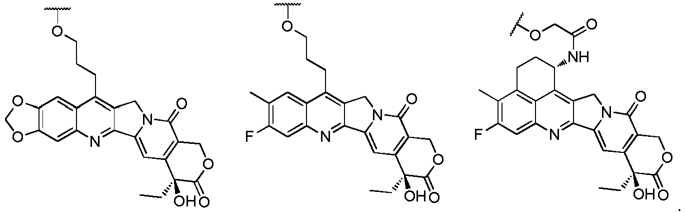

### R*

In a specific embodiment, R* is H; in another specific embodiment, R* is deuterium; in another specific embodiment, R* is halogen; in another specific embodiment, R* is CN; in another specific embodiment, R* is NO₂; in another specific embodiment, R* is OH; in another specific embodiment, R* is C₁-₆ alkyl; in another specific embodiment, R* is C₁-₄ alkyl; in another specific embodiment, R* is C₁-₆ haloalkyl; in another specific embodiment, R* is C₁-₄ haloalkyl; in another specific embodiment, R* is C₂-₆ alkenyl; in another specific embodiment, R* is C₂-₄ alkenyl; in another specific embodiment, R* is C₂-₆ alkynyl; in another specific embodiment, R* is C₂-₄ alkynyl.

### R#

In a specific embodiment, R# is H; in another specific embodiment, R# is deuterium; in another specific embodiment, R# is halogen; in another specific embodiment, R# is CN; in another specific embodiment, R# is NO₂; in another specific embodiment, R# is OH; in another specific embodiment, R# is C₁-₆ alkyl; in another specific embodiment, R# is C₁-₄ alkyl; in another specific embodiment, R# is C₁-₆ haloalkyl; in another specific embodiment, R# is C₁-₄ haloalkyl; in another specific embodiment, R# is C₂-₆ alkenyl; in another specific embodiment, R# is C₂-₄ alkenyl; in another specific embodiment, R# is C₂-₆ alkynyl; in another specific embodiment, R# is C₂-₄ alkynyl.

### n

In a specific embodiment, n is 0; in another specific embodiment, n is 1; in another specific embodiment, n is 2; in another specific embodiment, n is 3; in another specific embodiment, n is 4; in another specific embodiment, n is 5.

### q

In a specific embodiment, q is an integer selected from 1 to 10; in another specific embodiment, q is 1; in another specific embodiment, q is 2; in another specific embodiment, q is 3; in another specific embodiment, q is 4; in another specific embodiment, q is 5; in another specific embodiment, q is 6; in another specific embodiment, q is 7; in another specific embodiment, q is 8; in another specific embodiment, q is 9; in another specific embodiment, q is 10.

Any technical solution or any combination thereof in any of the specific embodiments described above can be combined with any technical solution or any combination thereof in other specific embodiments. For example, any technical solution regarding Ab or any combination thereof can be combined with any technical solution or any combination thereof regarding X, Y, Y₁, Y₂, Y₃, Z, E, D, R*, R#, n, and q. The present invention is intended to include all such combinations of technical solutions, and for simplicity, they are not listed individually.

In a more specific embodiment, the present invention provides the MSLN antibody-drug conjugate of formula (I) described above, wherein,
X is selected from C₆-₁₀ aryl or a 5-10-membered heteroaryl group;
X is optionally substituted with 1, 2, 3, 4, or 5 instance(s) of R*;
R* is selected from H, deuterium, halogen, CN, NO₂, OH, or C₁-₆ alkyl;
preferably,
X is a 5-6-membered heteroaryl group;
X is optionally substituted with 1, 2, or 3 instance(s) of R*;
R* is selected from H, deuterium, halogen, or C₁-₄ alkyl;
more preferably,
X is pyrimidinyl, for example,

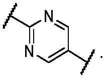

In a more specific embodiment, the present invention provides the MSLN antibody-drug conjugate of formula (I) described above, wherein,
Y is -Y₁-Y₂-Y₃-, Y being linked to X via Y₁ and to Z via Y₃,
wherein,
Y₁ is selected from C₂-₆ alkenyl or C₂-₆ alkynyl;
Y₂ is a bond, or C₁-₆ alkylene;
Y₃ is selected from -C(O)- or -NHC(O)-;
Y is optionally substituted with 1, 2, 3, 4, or 5 instance(s) of R#;
R# is selected from H, deuterium, halogen, CN, NO₂, OH, or C₁-₆ alkyl;
preferably,
Y is -Y₁-Y₂-Y₃-, Y being linked to X via Y₁ and to Z via Y₃,
wherein,
Y₁ is selected from C₂-₄ alkenyl or C₂-₄ alkynyl;
Y₂ is C₁-₄ alkylene;
Y₃ is -C(O)-;
Y is optionally substituted with 1, 2, or 3 instance(s) of R#;
R# is selected from H, deuterium, halogen, or C₁-₄ alkyl;
more preferably,
Y is

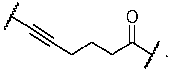

In a more specific embodiment, the present invention provides the MSLN antibody-drug conjugate of formula (I) described above, wherein,
Z is selected from val-cit, val-cit-gly, gly-gly, gly-gly-gly, gly-gly-gly-gly, val-gly-gly, val-gln-gly, val-glu-gly, phe-lys-gly, leu-lys-gly, gly-val-lys-gly, val-lys-gly-gly, val-lys-gly, val-lys-ala, val-lys-leu, leu-leu-gly, gly-gly-phe-gly, gly-gly-phe-gly-gly, val-gly, val-lys-β-ala, or the aforementioned dipeptides, tripeptides, or tetrapeptides, after undergoing modification by means of substitution;
preferably,
Z is 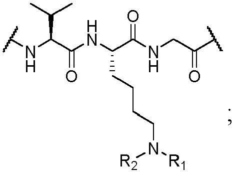
R₁ and R₂ are independently selected from H, deuterium, C₁-₆ alkyl, C₁-₆ haloalkyl, C₂-₆ alkenyl, or C₂-₆ alkynyl;
more preferably,
Z is 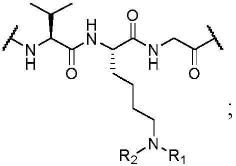
R₁ and R₂ are independently selected from H, deuterium, halogen, or C₁-₆ alkyl, preferably H or C₁-₄ alkyl.

In a more specific embodiment, the present invention provides the MSLN antibody-drug conjugate of the above formula (I), wherein,
E is absent or is selected from -NH-C₁-₆ alkylene- or -O-C₁-₆ alkylene-, wherein the - NH-C₁-₆ alkylene- and -O-C₁-₆ alkylene- may optionally be substituted with 1, 2, or 3 substituents selected from H, halogen, C₁-₆ alkyl, and C₁-₆ haloalkyl;
preferably, E is absent or is -NH-C₁-₄ alkylene-, wherein the -NH-C₁-₄ alkylene- may optionally be substituted with 1, 2, or 3 substituents selected from H, halogen, C₁-₄ alkyl, and C₁-₄ haloalkyl;
more preferably, E is -NH-(CH₂)₁-₄-, for example, -NH-CH₂-.

In a more specific embodiment, the present invention provides the MSLN antibody-drug conjugate of the above formula (I), wherein,
D is selected from metal complexes; glycopeptide antibiotics; DNA topoisomerase inhibitors; drugs that interfere with DNA synthesis; drugs targeting structural proteins; tumor signaling pathway inhibitors; proteasome inhibitors; histone deacetylase inhibitors; tumor angiogenesis inhibitors; cyclin inhibitors; serine/threonine kinase inhibitors, tyrosine kinase inhibitors, aspartate kinase inhibitors, or histidine kinase inhibitors; and other active substances that inhibit tumor cell growth, promote tumor cell apoptosis or necrosis;
preferably,
D is selected from oxaliplatin; bleomycin or pingyangmycin; camptothecin, camptothecin derivatives (e.g., hydroxycamptothecin, 9-aminocamptothecin, etc.), SN-38, irinotecan, topotecan, belotecan, or rubitecan; actinomycin D, doxorubicin, duocarmycin, daunorubicin, mitoxantrone, podophyllotoxin, or etoposide; methotrexate, 5-fluorouracil, cytarabine, gemcitabine, mercaptopurine, pentostatin, fludarabine, cladribine, or nelarabine; vinca alkaloids, vincristine, vinblastine, paclitaxel, docetaxel, or cabazitaxel; maytansinoid derivatives; calicheamicin derivatives; auristatin derivatives; pyrrolobenzodiazepine dimer derivatives; melphalan; mitomycin C; or chlorambucil;
more preferably,
D is 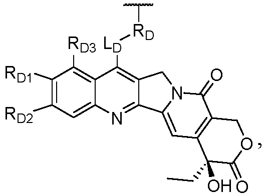
wherein,
R_{D} is absent or selected from -NH- or -O-;
L_{D} is selected from -C₀₋₆alkylene-O-C₀₋₆alkylene- or -C₀₋₆alkylene-O-C₀₋₆alkylene-C(O)NH-CHR_{D4}-;
R_{D1} and R_{D2} are independently selected from H, halogen, C₁-₆ alkyl, C₁₋₆ haloalkyl or C₁₋₆ alkoxy; or, R_{D1} and R_{D2}, together with the carbon atom to which they are connected, form a C₃₋₇ cycloalkyl or a 3-7-membered heterocyclic group, preferably a 5-6-membered heterocyclic group;
R_{D3} and R_{D4} are independently selected from H, halogen, C₁₋₆ alkyl, C₁₋₆ haloalkyl or C₁₋₆ alkoxy; or R_{D3} and R_{D4}, together with the carbon atom to which they are connected, form C₅₋₁₀ cycloalkyl or C₅₋₁₀ heterocyclic, preferably C₅₋₆ cycloalkyl;
more preferably,
D is selected from: 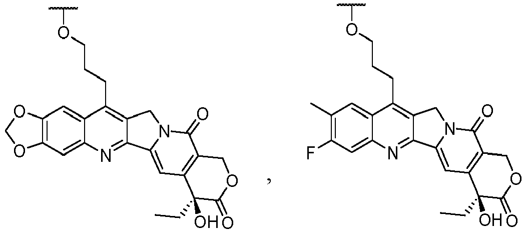 or

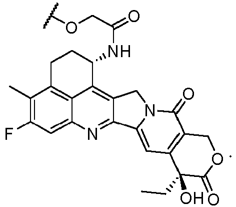

In a more specific embodiment, the present invention provides the MSLN antibody-drug conjugate of formula (I) described above, wherein,
X is selected from C₆-₁₀ aryl or a 5-10-membered heteroaryl group;
X is optionally substituted with 1, 2, 3, 4, or 5 instance(s) of R*;
R* is selected from H, deuterium, halogen, CN, NO₂, OH, or C₁-₆ alkyl;
Y is -Y₁-Y₂-Y₃-, Y being linked to X via Y₁ and to Z via Y₃,
wherein,
Y₁ is selected from C₂-₆ alkenyl or C₂-₆ alkynyl;
Y₂ is a bond or C₁-₆ alkylene;
Y₃ is selected from -C(O)- or -NHC(O)-;
Y is optionally substituted with 1, 2, 3, 4, or 5 instance(s) of R#;
R# is selected from H, deuterium, halogen, CN, NO₂, OH, or C₁-₆ alkyl;
Z is selected from val-cit, val-cit-gly, gly-gly, gly-gly-gly, gly-gly-gly-gly, val-gly-gly, val-gln-gly, val-glu-gly, phe-lys-gly, leu-lys-gly, gly-val-lys-gly, val-lys-gly-gly, val-lys-gly, val-lys-ala, val-lys-leu, leu-leu-gly, gly-gly-phe-gly, gly-gly-phe-gly-gly, val-gly, val-lys-β-ala, or the aforementioned dipeptides, tripeptides, or tetrapeptides, after undergoing modification by means of substitution;
E is absent or selected from -NH-C₁-₆ alkylene- or -O-C₁-₆ alkylene-, wherein the -NH-C₁-₆ alkylene- and -O-C₁-₆ alkylene- may optionally be substituted with 1, 2, or 3 substituents selected from H, halogen, C₁-₆ alkyl, and C₁-₆ haloalkyl;
D is selected from metal complexes; glycopeptide antibiotics; DNA topoisomerase inhibitors; drugs that interfere with DNA synthesis; drugs targeting structural proteins; tumor signaling pathway inhibitors; proteasome inhibitors; histone deacetylase inhibitors; tumor angiogenesis inhibitors; cyclin inhibitors; serine/threonine kinase inhibitors, tyrosine kinase inhibitors, aspartate kinase inhibitors, or histidine kinase inhibitors; and other active substances that inhibit tumor cell growth, or promote tumor cell apoptosis or necrosis.

In a more specific embodiment, the present invention provides the MSLN antibody-drug conjugate of the above formula (I), wherein,
X is a 5-6-membered heteroaryl group;
X is optionally substituted with 1, 2, or 3 instance(s) of R*;
R* is selected from H, deuterium, halogen, or C₁₋₄ alkyl;
Y is -Y₁-Y₂-Y₃-, Y being linked to X via Y₁ and to Z via Y₃;
wherein,
Y₁ is selected from C₂-₄ alkenyl or C₂-₄ alkynyl;
Y₂ is a C₁₋₄ alkylene;
Y₃ is -C(O)-;
Y is optionally substituted with 1, 2, or 3 instances of R#;
R# is selected from H, deuterium, halogen, or C₁₋₄ alkyl;
Z is 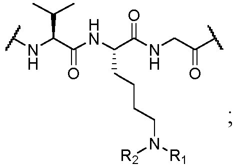
E is absent or is -NH-C₁₋₄alkydene-, wherein the -NH-C₁₋₄alkylene- may optionally be substituted with 1, 2, or 3 substituents selected from H, halogen, C₁₋₄ alkyl and C₁₋₄ haloalkyl;
R₁ and R₂ are independently selected from H, deuterium, halogen, C₁₋₆ alkyl, C₁₋₆ haloalkyl, C₂₋₆ alkenyl or C₂₋₆ alkynyl;
D is selected from oxaliplatin; bleomycin or pingyangmycin; camptothecin, camptothecin derivatives (e.g., hydroxycamptothecin, 9-aminocamptothecin, etc.), SN-38, irinotecan, topotecan, belotecan, or rubitecan; actinomycin D, doxorubicin, duocarmycin, daunorubicin, mitoxantrone, podophyllotoxin, or etoposide; methotrexate, 5-fluorouracil, cytarabine, gemcitabine, mercaptopurine, pentostatin, fludarabine, cladribine, or nelarabine; vinca alkaloids, vincristine, vinblastine, paclitaxel, docetaxel, or cabazitaxel; maytansinoid derivatives; calicheamicin derivatives; auristatin derivatives; pyrrolobenzodiazepine dimer derivatives; melphalan; mitomycin C; or chlorambucil;
preferably,
D is 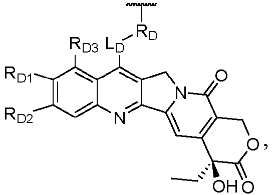
wherein,
R_{D} is absent or selected from -NH- or -O-;
L_{D} is selected from -C₀₋₆alkylene-O-C₀₋₆alkylene- or -C₀₋₆alkylene-O-C₀₋₆alkylidene-C(O)NH-CHR_{D4}-;
R_{D1} and R_{D2} are independently selected from H, halogen, C₁₋₆ alkyl, C₁₋₆ haloalkyl or C₁₋₆ alkoxy; or, R_{D1} and R_{D2}, together with the carbon atom to which they are connected, form a C₃₋₇ cycloalkyl or a 3-7-membered heterocyclic group, preferably a 5-6-membered heterocyclic group;
R_{D3} and R_{D4} are independently selected from H, halogen, C₁₋₆ alkyl, C₁₋₆ haloalkyl or C₁₋₆ alkoxy; or R_{D3} and R_{D4}, together with the carbon atom to which they are connected, form C₅₋₁₀ cycloalkyl or a C₅₋₁₀ heterocyclic group, preferably C₅₋₆ cycloalkyl.

In a more specific embodiment, the present invention provides the MSLN antibody-drug conjugate of the above formula (I), wherein,
X is pyrimidinyl, preferably 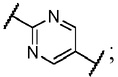
Y is 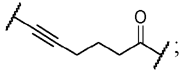
Z is 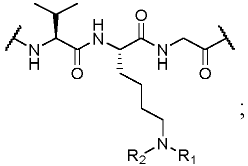
R₁ and R₂ are independently selected from H, deuterium, halogen, C₁-₆ alkyl, preferably H or C₁-₄ alkyl;
E is -NH-CH₂-;
D is selected from: 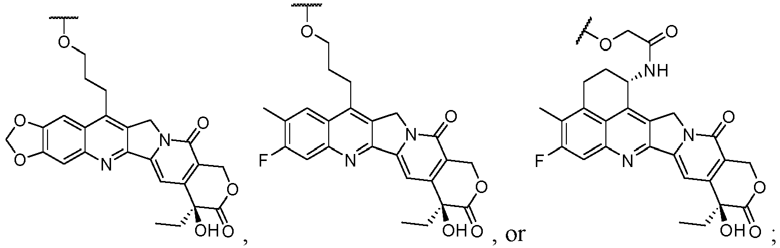
n is selected from 1, 2, or 3, preferably 1;
q is selected from 4, 5, 6, 7, 8, 9, or 10, preferably 8.

In a more specific embodiment, the present invention provides an MSLN antibody-drug conjugate, wherein the MSLN antibody-drug conjugate is a compound of formula (II) or a tautomer, stereoisomer, or pharmaceutically acceptable salt thereof: 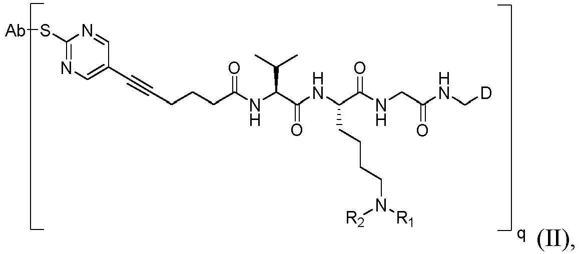 wherein,
Ab is an anti-MSLN antibody;
R₁ and R₂ are independently selected from C₁-₆ alkyl, preferably C₁-₄ alkyl;
D is selected from: 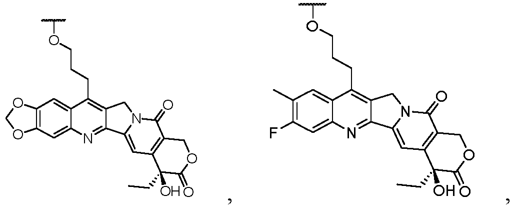 or 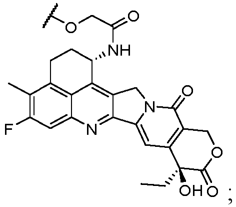
q is an integer selected from 1 to 10, preferably selected from 4, 5, 6, 7, 8, 9, or 10, and most preferably 8.

### Anti-MSLN Antibody

The antibody specifically binding to MSLN in the antibody-drug conjugate of the present invention comprises a heavy chain variable region (VH) and a light chain variable region (VL), wherein,
(1) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 27;
(2) the VH comprises HCDR1, HCDR2, and HCDR3 of the VH with the amino acid sequence shown in SEQ ID NO: 25, and the VL comprises LCDR1, LCDR2, and LCDR3 of the VL with the amino acid sequence shown in SEQ ID NO: 28;
(3) the VH comprises HCDR1, HCDR2, and HCDR3 of the VH with the amino acid sequence shown in SEQ ID NO: 26, and the VL comprises LCDR1, LCDR2, and LCDR3 of the VL with the amino acid sequence shown in SEQ ID NO: 27;
(4) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 29;
(5) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 30;
(6) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 31;
(7) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 32;
(8) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 33;
(9) the VH comprises HCDR1, HCDR2, and HCDR3 of the VH with the amino acid sequence shown in SEQ ID NO: 25, and the VL comprises LCDR1, LCDR2, and LCDR3 of the VL with the amino acid sequence shown in SEQ ID NO: 29;
(10) the VH comprises HCDR1, HCDR2, and HCDR3 of the VH with the amino acid sequence shown in SEQ ID NO: 63, and the VL comprises LCDR1, LCDR2, and LCDR3 of the VL with the amino acid sequence shown in SEQ ID NO: 67;
(11) the VH comprises HCDR1, HCDR2, and HCDR3 of the VH with the amino acid sequence shown in SEQ ID NO: 64, and the VL comprises LCDR1, LCDR2, and LCDR3 of the VL with the amino acid sequence shown in SEQ ID NO: 67;
(12) the VH comprises HCDR1, HCDR2, and HCDR3 of the VH with the amino acid sequence shown in SEQ ID NO: 65, and the VL comprises LCDR1, LCDR2, and LCDR3 of the VL with the amino acid sequence shown in SEQ ID NO: 67;
(13) the VH comprises HCDR1, HCDR2, and HCDR3 of the VH with the amino acid sequence shown in SEQ ID NO: 65, and the VL comprises LCDR1, LCDR2, and LCDR3 of the VL with the amino acid sequence shown in SEQ ID NO: 68;
(14) the VH comprises HCDR1, HCDR2, and HCDR3 of the VH with the amino acid sequence shown in SEQ ID NO: 63, and the VL comprises LCDR1, LCDR2, and LCDR3 of the VL with the amino acid sequence shown in SEQ ID NO: 68;
(15) the VH comprises HCDR1, HCDR2, and HCDR3 of the VH with the amino acid sequence shown in SEQ ID NO: 66, and the VL comprises LCDR1, LCDR2, and LCDR3 of the VL with the amino acid sequence shown in SEQ ID NO: 67;
(16) the VH comprises HCDR1, HCDR2, and HCDR3 of the VH with the amino acid sequence shown in SEQ ID NO: 64, and the VL comprises LCDR1, LCDR2, and LCDR3 of the VL with the amino acid sequence shown in SEQ ID NO: 69;
(17) the VH comprises HCDR1, HCDR2, and HCDR3 of the VH with the amino acid sequence shown in SEQ ID NO: 66, and the VL comprises LCDR1, LCDR2, and LCDR3 of the VL with the amino acid sequence shown in SEQ ID NO: 69;
(18) the VH comprises HCDR1, HCDR2, and HCDR3 of the VH with the amino acid sequence shown in SEQ ID NO: 63, and the VL comprises LCDR1, LCDR2, and LCDR3 of the VL with the amino acid sequence shown in SEQ ID NO: 69; or
(19) the VH comprises HCDR1, HCDR2, and HCDR3 of the VH with the amino acid sequence shown in SEQ ID NO: 65, and the VL comprises LCDR1, LCDR2, and LCDR3 of the VL with the amino acid sequence shown in SEQ ID NO: 69.

In some embodiments, LCDR 1-3 and HCDR 1-3 are defined by the EU Kabat definition/numbering system. In some embodiments, LCDR 1-3 and HCDR 1-3 are defined by the Chothia definition/numbering system. In some embodiments, LCDR 1-3 and HCDR 1-3 are defined by the AbM definition/numbering system. In some preferred embodiments, LCDR 1-3 and HCDR 1-3 are defined by the combined Kabat and Chothia numbering system.

In some embodiments, the Ab in the antibody-drug conjugate of the present invention is an antibody that specifically binds to mesothelin (MSLN) or an antigen-binding fragment thereof, wherein the antibody comprises a heavy chain variable region (VH) and a light chain variable region (VL), wherein,
(1) the VH comprises HCDR1, HCDR2, and HCDR3, HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 13;
(2) the VH comprises HCDR1, HCDR2, and HCDR3, HCDR1 comprising the amino acid sequence shown in SEQ ID NO: 15, HCDR2 comprising the amino acid sequence shown in SEQ ID NO: 16, and HCDR3 comprising the amino acid sequence shown in SEQ ID NO: 6, and the VL comprises LCDR1, LCDR2, and LCDR3, LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 18;
(3) the VH comprises HCDR1, HCDR2, and HCDR3, HCDR1 comprising the amino acid sequence shown in SEQ ID NO: 15, HCDR2 comprising the amino acid sequence shown in SEQ ID NO: 17, and HCDR3 comprising the amino acid sequence shown in SEQ ID NO: 6, and the VL comprises LCDR1, LCDR2, and LCDR3, LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 13;
(4) the VH comprises HCDR1, HCDR2, and HCDR3, HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 19;
(5) the VH comprises HCDR1, HCDR2, and HCDR3, HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 20;
(6) the VH comprises HCDR1, HCDR2, and HCDR3, HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 21;
(7) the VH comprises HCDR1, HCDR2, and HCDR3, HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 22;
(8) the VH comprises HCDR1, HCDR2, and HCDR3, HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 23;
(9) the VH comprises HCDR1, HCDR2, and HCDR3, HCDR1 comprising the amino acid sequence shown in SEQ ID NO: 15, HCDR2 comprising the amino acid sequence shown in SEQ ID NO: 16, and HCDR3 comprising the amino acid sequence shown in SEQ ID NO: 6, and the VL comprises LCDR1, LCDR2, and LCDR3, LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 19;
(10) the VH comprises HCDR1, HCDR2, and HCDR3, HCDR1 comprising the amino acid sequence shown in SEQ ID NO: 45, HCDR2 comprising the amino acid sequence shown in SEQ ID NO: 47, and HCDR3 comprising the amino acid sequence shown in SEQ ID NO: 49, and the VL comprises LCDR1, LCDR2, and LCDR3, LCDR1 comprising the amino acid sequence shown in SEQ ID NO: 52, LCDR2 comprising the amino acid sequence shown in SEQ ID NO: 54, and LCDR3 comprising the amino acid sequence shown in SEQ ID NO: 56;
(11) the VH comprises HCDR1, HCDR2, and HCDR3, HCDR1 comprising the amino acid sequence shown in SEQ ID NO: 58, HCDR2 comprising the amino acid sequence shown in SEQ ID NO: 47, and HCDR3 comprising the amino acid sequence shown in SEQ ID NO: 49, and the VL comprises LCDR1, LCDR2, and LCDR3, LCDR1 comprising the amino acid sequence shown in SEQ ID NO: 52, LCDR2 comprising the amino acid sequence shown in SEQ ID NO: 54, and LCDR3 comprising the amino acid sequence shown in SEQ ID NO: 56;
(12) the VH comprises HCDR1, HCDR2, and HCDR3, HCDR1 comprising the amino acid sequence shown in SEQ ID NO: 58, HCDR2 comprising the amino acid sequence shown in SEQ ID NO: 59, and HCDR3 comprising the amino acid sequence shown in SEQ ID NO: 49, and the VL comprises LCDR1, LCDR2, and LCDR3, LCDR1 comprising the amino acid sequence shown in SEQ ID NO: 52, LCDR2 comprising the amino acid sequence shown in SEQ ID NO: 54, and LCDR3 comprising the amino acid sequence shown in SEQ ID NO: 56;
(13) the VH comprises HCDR1, HCDR2, and HCDR3, HCDR1 comprising the amino acid sequence shown in SEQ ID NO: 58, HCDR2 comprising the amino acid sequence shown in SEQ ID NO: 59, and HCDR3 comprising the amino acid sequence shown in SEQ ID NO: 49, and the VL comprises LCDR1, LCDR2, and LCDR3, LCDR1 comprising the amino acid sequence shown in SEQ ID NO: 61, LCDR2 comprising the amino acid sequence shown in SEQ ID NO: 54, and LCDR3 comprising the amino acid sequence shown in SEQ ID NO: 56;
(14) the VH comprises HCDR1, HCDR2, and HCDR3, HCDR1 comprising the amino acid sequence shown in SEQ ID NO: 45, HCDR2 comprising the amino acid sequence shown in SEQ ID NO: 47, and HCDR3 comprising the amino acid sequence shown in SEQ ID NO: 49, and the VL comprises LCDR1, LCDR2, and LCDR3, LCDR1 comprising the amino acid sequence shown in SEQ ID NO: 61, LCDR2 comprising the amino acid sequence shown in SEQ ID NO: 54, and LCDR3 comprising the amino acid sequence shown in SEQ ID NO: 56;
(15) the VH comprises HCDR1, HCDR2, and HCDR3, HCDR1 comprising the amino acid sequence shown in SEQ ID NO: 58, HCDR2 comprising the amino acid sequence shown in SEQ ID NO: 60, and HCDR3 comprising the amino acid sequence shown in SEQ ID NO: 49, and the VL comprises LCDR1, LCDR2, and LCDR3, LCDR1 comprising the amino acid sequence shown in SEQ ID NO: 52, LCDR2 comprising the amino acid sequence shown in SEQ ID NO: 54, and LCDR3 comprising the amino acid sequence shown in SEQ ID NO: 56;
(16) the VH comprises HCDR1, HCDR2, and HCDR3, HCDR1 comprising the amino acid sequence shown in SEQ ID NO: 58, HCDR2 comprising the amino acid sequence shown in SEQ ID NO: 47, and HCDR3 comprising the amino acid sequence shown in SEQ ID NO: 49, and the VL comprises LCDR1, LCDR2, and LCDR3, LCDR1 comprising the amino acid sequence shown in SEQ ID NO: 62, LCDR2 comprising the amino acid sequence shown in SEQ ID NO: 54, and LCDR3 comprising the amino acid sequence shown in SEQ ID NO: 56;
(17) the VH comprises HCDR1, HCDR2, and HCDR3, HCDR1 comprising the amino acid sequence shown in SEQ ID NO: 58, HCDR2 comprising the amino acid sequence shown in SEQ ID NO: 60, and HCDR3 comprising the amino acid sequence shown in SEQ ID NO: 49, and the VL comprises LCDR1, LCDR2, and LCDR3, LCDR1 comprising the amino acid sequence shown in SEQ ID NO: 62, LCDR2 comprising the amino acid sequence shown in SEQ ID NO: 54, and LCDR3 comprising the amino acid sequence shown in SEQ ID NO: 56;
(18) the VH comprises HCDR1, HCDR2, and HCDR3, HCDR1 comprising the amino acid sequence shown in SEQ ID NO: 45, HCDR2 comprising the amino acid sequence shown in SEQ ID NO: 47, and HCDR3 comprising the amino acid sequence shown in SEQ ID NO: 49, and the VL comprises LCDR1, LCDR2, and LCDR3, LCDR1 comprising the amino acid sequence shown in SEQ ID NO: 62, LCDR2 comprising the amino acid sequence shown in SEQ ID NO: 54, and LCDR3 comprising the amino acid sequence shown in SEQ ID NO: 56; or
(19) the VH comprises HCDR1, HCDR2, and HCDR3, HCDR1 comprising the amino acid sequence shown in SEQ ID NO: 58, HCDR2 comprising the amino acid sequence shown in SEQ ID NO: 59, and HCDR3 comprising the amino acid sequence shown in SEQ ID NO: 49, and the VL comprises LCDR1, LCDR2, and LCDR3, LCDR1 comprising the amino acid sequence shown in SEQ ID NO: 62, LCDR2 comprising the amino acid sequence shown in SEQ ID NO: 54, and LCDR3 comprising the amino acid sequence shown in SEQ ID NO: 56.

In some embodiments, the CDR is determined using the combined Kabat Chothia system.

In some embodiments, the Ab in the antibody-drug conjugate of the present invention is an antibody that specifically binds to mesothelin (MSLN) or an antigen-binding fragment thereof, wherein the antibody comprises VL and VH, wherein,
(1) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 27;
(2) the VH comprises an amino acid sequence with at least 80%, at least 85%, at least 90%, at least 95%, at least 98%, at least 99%, or 100% sequence identity to SEQ ID NO: 25, and the VL comprises an amino acid sequence with at least 80%, at least 85%, at least 90%, at least 95%, at least 98%, at least 99%, or 100% sequence identity to SEQ ID NO: 28;
(3) the VH comprises an amino acid sequence with at least 80%, at least 85%, at least 90%, at least 95%, at least 98%, at least 99%, or 100% sequence identity to SEQ ID NO: 26, and the VL comprises an amino acid sequence with at least 80%, at least 85%, at least 90%, at least 95%, at least 98%, at least 99%, or 100% sequence identity to SEQ ID NO: 27;
(4) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 29;
(5) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 30;
(6) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 31;
(7) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 32;
(8) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 33;
(9) the VH comprises an amino acid sequence with at least 80%, at least 85%, at least 90%, at least 95%, at least 98%, at least 99%, or 100% sequence identity to SEQ ID NO: 25, and the VL comprises an amino acid sequence with at least 80%, at least 85%, at least 90%, at least 95%, at least 98%, at least 99%, or 100% sequence identity to SEQ ID NO: 29;
(10) the VH comprises an amino acid sequence with at least 80%, at least 85%, at least 90%, at least 95%, at least 98%, at least 99%, or 100% sequence identity to SEQ ID NO: 63, and the VL comprises an amino acid sequence with at least 80%, at least 85%, at least 90%, at least 95%, at least 98%, at least 99%, or 100% sequence identity to SEQ ID NO: 67;
(11) the VH comprises an amino acid sequence with at least 80%, at least 85%, at least 90%, at least 95%, at least 98%, at least 99%, or 100% sequence identity to SEQ ID NO: 64, and the VL comprises an amino acid sequence with at least 80%, at least 85%, at least 90%, at least 95%, at least 98%, at least 99%, or 100% sequence identity to SEQ ID NO: 67;
(12) the VH comprises an amino acid sequence with at least 80%, at least 85%, at least 90%, at least 95%, at least 98%, at least 99%, or 100% sequence identity to SEQ ID NO: 65, and the VL comprises an amino acid sequence with at least 80%, at least 85%, at least 90%, at least 95%, at least 98%, at least 99%, or 100% sequence identity to SEQ ID NO: 67;
(13) the VH comprises an amino acid sequence with at least 80%, at least 85%, at least 90%, at least 95%, at least 98%, at least 99%, or 100% sequence identity to SEQ ID NO: 65, and the VL comprises an amino acid sequence with at least 80%, at least 85%, at least 90%, at least 95%, at least 98%, at least 99%, or 100% sequence identity to SEQ ID NO: 68;
(14) the VH comprises an amino acid sequence with at least 80%, at least 85%, at least 90%, at least 95%, at least 98%, at least 99%, or 100% sequence identity to SEQ ID NO: 63, and the VL comprises an amino acid sequence with at least 80%, at least 85%, at least 90%, at least 95%, at least 98%, at least 99%, or 100% sequence identity to SEQ ID NO: 68;
(15) the VH comprises an amino acid sequence with at least 80%, at least 85%, at least 90%, at least 95%, at least 98%, at least 99%, or 100% sequence identity to SEQ ID NO: 66, and the VL comprises an amino acid sequence with at least 80%, at least 85%, at least 90%, at least 95%, at least 98%, at least 99%, or 100% sequence identity to SEQ ID NO: 67;
(16) the VH comprises an amino acid sequence with at least 80%, at least 85%, at least 90%, at least 95%, at least 98%, at least 99%, or 100% sequence identity to SEQ ID NO: 64, and the VL comprises an amino acid sequence with at least 80%, at least 85%, at least 90%, at least 95%, at least 98%, at least 99%, or 100% sequence identity to SEQ ID NO: 69;
(17) the VH comprises an amino acid sequence with at least 80%, at least 85%, at least 90%, at least 95%, at least 98%, at least 99%, or 100% sequence identity to SEQ ID NO: 66, and the VL comprises an amino acid sequence with at least 80%, at least 85%, at least 90%, at least 95%, at least 98%, at least 99%, or 100% sequence identity to SEQ ID NO: 69;
(18) the VH comprises an amino acid sequence with at least 80%, at least 85%, at least 90%, at least 95%, at least 98%, at least 99%, or 100% sequence identity to SEQ ID NO: 63, and the VL comprises an amino acid sequence with at least 80%, at least 85%, at least 90%, at least 95%, at least 98%, at least 99%, or 100% sequence identity to SEQ ID NO: 69; or
(19) the VH comprises an amino acid sequence with at least 80%, at least 85%, at least 90%, at least 95%, at least 98%, at least 99%, or 100% sequence identity to SEQ ID NO: 65, and the VL comprises an amino acid sequence with at least 80%, at least 85%, at least 90%, at least 95%, at least 98%, at least 99%, or 100% sequence identity to SEQ ID NO: 69.

In some embodiments, the VL comprises a functional variant of any one of the amino acid sequences shown in SEQ ID NOs: 27-33 and 67-69, formed by inserting, deleting, and/or substituting one or more of the amino acids therein, provided that the antibody containing the VL with the functional variant retains the ability to bind to MSLN. In some embodiments, the VH comprises a functional variant of any one of the amino acid sequences shown in SEQ ID NOs: 24-26 and 63-66, formed by inserting, deleting, and/or substituting one or more of the amino acids therein, provided that the antibody containing the VH with the functional variant retains the ability to bind to MSLN.

A functional variant comprises or consists of an amino acid sequence with at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence homology with the amino acid sequence of the parent polypeptide. For example, a functional variant of any one of SEQ ID NOs: 27-33 and 67-69 comprises or consists of an amino acid sequence with at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to SEQ ID NOs: 27-33 and 67-69, respectively. A functional variant of any one of SEQ ID NOs: 24-26 and 63-66 comprises or consists of an amino acid sequence with at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to SEQ ID NOs: 24-26 and 63-66, respectively.

In the context of functional variants, the number of amino acids inserted, deleted, and/or substituted preferably does not exceed 40%, more preferably does not exceed 35%, more preferably does not exceed 1-33%, more preferably does not exceed 5-30%, more preferably does not exceed 10-25%, and more preferably does not exceed 15-20% of the total number of amino acids in the parent amino acid sequence. For example, the number of amino acids inserted, deleted, and/or substituted can be 1-20, preferably 1-10, more preferably 1-7, more preferably 1-5, and most preferably 1-2. In preferred embodiments, the number of amino acids inserted, deleted, and/or substituted is 1, 2, 3, 4, 5, 6, or 7.

In some embodiments, the insertion, deletion, and/or substitution can be performed in the framework (FR) regions, for example, in FR1, FR2, FR3, and/or FR4.

In a preferred embodiment, the VL comprises the amino acid sequence shown in SEQ ID NO: 27, and the VH comprises the amino acid sequence shown in SEQ ID NO: 24; or the VL comprises the amino acid sequence shown in SEQ ID NO: 28, and the VH comprises the amino acid sequence shown in SEQ ID NO: 25; or the VL comprises the amino acid sequence shown in SEQ ID NO: 27, and the VH comprises the amino acid sequence shown in SEQ ID NO: 26; or the VL comprises the amino acid sequence shown in SEQ ID NO: 29, and the VH comprises the amino acid sequence shown in SEQ ID NO: 24; or the VL comprises the amino acid sequence shown in SEQ ID NO: 30, and the VH comprises the amino acid sequence shown in SEQ ID NO: 24; or the VL comprises the amino acid sequence shown in SEQ ID NO: 31, and the VH comprises the amino acid sequence shown in SEQ ID NO: 24; or the VL comprises the amino acid sequence shown in SEQ ID NO: 32, and the VH comprises the amino acid sequence shown in SEQ ID NO: 24; or the VL comprises the amino acid sequence shown in SEQ ID NO: 33, and the VH comprises the amino acid sequence shown in SEQ ID NO: 24; or the VL comprises the amino acid sequence shown in SEQ ID NO: 29, and the VH comprises the amino acid sequence shown in SEQ ID NO: 25; or the VL comprises the amino acid sequence shown in SEQ ID NO: 67, and the VH comprises the amino acid sequence shown in SEQ ID NO: 63; or the VL comprises the amino acid sequence shown in SEQ ID NO: 67, and the VH comprises the amino acid sequence shown in SEQ ID NO: 64; or the VL comprises the amino acid sequence shown in SEQ ID NO: 67, and the VH comprises the amino acid sequence shown in SEQ ID NO: 65; or the VL comprises the amino acid sequence shown in SEQ ID NO: 68, and the VH comprises the amino acid sequence shown in SEQ ID NO: 65; or the VL comprises the amino acid sequence shown in SEQ ID NO: 68, and the VH comprises the amino acid sequence shown in SEQ ID NO: 63; or the VL comprises the amino acid sequence shown in SEQ ID NO: 67, and the VH comprises the amino acid sequence shown in SEQ ID NO: 66; or the VL comprises the amino acid sequence shown in SEQ ID NO: 69, and the VH comprises the amino acid sequence shown in SEQ ID NO: 64; or the VL comprises the amino acid sequence shown in SEQ ID NO: 69, and the VH comprises the amino acid sequence shown in SEQ ID NO: 66; or the VL comprises the amino acid sequence shown in SEQ ID NO: 69, and the VH comprises the amino acid sequence shown in SEQ ID NO: 63; or the VL comprises the amino acid sequence shown in SEQ ID NO: 69, and the VH comprises the amino acid sequence shown in SEQ ID NO: 65.

In some embodiments, the Ab in the antibody-drug conjugate of the present invention is an antibody that specifically binds to mesothelin (MSLN) or an antigen-binding fragment thereof, wherein the antibody includes a heavy chain (HC) and a light chain (LC), wherein,
(1) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 37;
(2) the HC comprises an amino acid sequence with at least 80%, at least 85%, at least 90%, at least 95%, at least 98%, at least 99%, or 100% sequence identity to SEQ ID NO: 35, and the LC comprises an amino acid sequence with at least 80%, at least 85%, at least 90%, at least 95%, at least 98%, at least 99%, or 100% sequence identity to SEQ ID NO: 38;
(3) the HC comprises an amino acid sequence with at least 80%, at least 85%, at least 90%, at least 95%, at least 98%, at least 99%, or 100% sequence identity to SEQ ID NO: 36, and the LC comprises an amino acid sequence with at least 80%, at least 85%, at least 90%, at least 95%, at least 98%, at least 99%, or 100% sequence identity to SEQ ID NO: 37;
(4) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 39;
(5) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 40;
(6) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 41;
(7) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 42;
(8) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 43;
(9) the HC comprises an amino acid sequence with at least 80%, at least 85%, at least 90%, at least 95%, at least 98%, at least 99%, or 100% sequence identity to SEQ ID NO: 35, and the LC comprises an amino acid sequence with at least 80%, at least 85%, at least 90%, at least 95%, at least 98%, at least 99%, or 100% sequence identity to SEQ ID NO: 39;
(10) the HC comprises an amino acid sequence with at least 80%, at least 85%, at least 90%, at least 95%, at least 98%, at least 99%, or 100% sequence identity to SEQ ID NO: 70, and the LC comprises an amino acid sequence with at least 80%, at least 85%, at least 90%, at least 95%, at least 98%, at least 99%, or 100% sequence identity to SEQ ID NO: 74;
(11) the HC comprises an amino acid sequence with at least 80%, at least 85%, at least 90%, at least 95%, at least 98%, at least 99%, or 100% sequence identity to SEQ ID NO: 71, and the LC comprises an amino acid sequence with at least 80%, at least 85%, at least 90%, at least 95%, at least 98%, at least 99%, or 100% sequence identity to SEQ ID NO: 74;
(12) the HC comprises an amino acid sequence with at least 80%, at least 85%, at least 90%, at least 95%, at least 98%, at least 99%, or 100% sequence identity to SEQ ID NO: 72, and the LC comprises an amino acid sequence with at least 80%, at least 85%, at least 90%, at least 95%, at least 98%, at least 99%, or 100% sequence identity to SEQ ID NO: 74;
(13) the HC comprises an amino acid sequence with at least 80%, at least 85%, at least 90%, at least 95%, at least 98%, at least 99%, or 100% sequence identity to SEQ ID NO: 72, and the LC comprises an amino acid sequence with at least 80%, at least 85%, at least 90%, at least 95%, at least 98%, at least 99%, or 100% sequence identity to SEQ ID NO: 75;
(14) the HC comprises an amino acid sequence with at least 80%, at least 85%, at least 90%, at least 95%, at least 98%, at least 99%, or 100% sequence identity to SEQ ID NO: 70, and the LC comprises an amino acid sequence with at least 80%, at least 85%, at least 90%, at least 95%, at least 98%, at least 99%, or 100% sequence identity to SEQ ID NO: 75;
(15) the HC comprises an amino acid sequence with at least 80%, at least 85%, at least 90%, at least 95%, at least 98%, at least 99%, or 100% sequence identity to SEQ ID NO: 73, and the LC comprises an amino acid sequence with at least 80%, at least 85%, at least 90%, at least 95%, at least 98%, at least 99%, or 100% sequence identity to SEQ ID NO: 74;
(16) the HC comprises an amino acid sequence with at least 80%, at least 85%, at least 90%, at least 95%, at least 98%, at least 99%, or 100% sequence identity to SEQ ID NO: 71, and the LC comprises an amino acid sequence with at least 80%, at least 85%, at least 90%, at least 95%, at least 98%, at least 99%, or 100% sequence identity to SEQ ID NO: 76;
(17) the HC comprises an amino acid sequence with at least 80%, at least 85%, at least 90%, at least 95%, at least 98%, at least 99%, or 100% sequence identity to SEQ ID NO: 73, and the LC comprises an amino acid sequence with at least 80%, at least 85%, at least 90%, at least 95%, at least 98%, at least 99%, or 100% sequence identity to SEQ ID NO: 76;
(18) the HC comprises an amino acid sequence with at least 80%, at least 85%, at least 90%, at least 95%, at least 98%, at least 99%, or 100% sequence identity to SEQ ID NO: 70, and the LC comprises an amino acid sequence with at least 80%, at least 85%, at least 90%, at least 95%, at least 98%, at least 99%, or 100% sequence identity to SEQ ID NO: 76; or
(19) the HC comprises an amino acid sequence with at least 80%, at least 85%, at least 90%, at least 95%, at least 98%, at least 99%, or 100% sequence identity to SEQ ID NO: 72, and the LC comprises an amino acid sequence with at least 80%, at least 85%, at least 90%, at least 95%, at least 98%, at least 99%, or 100% sequence identity to SEQ ID NO: 76.

In some embodiments, the light chain comprises a functional variant of any one of the amino acid sequences shown in SEQ ID NOs: 37-43 and 74-76, formed by inserting, deleting, and/or substituting one or more of the amino acids therein, provided that the antibody containing the light chain with the functional variant retains the ability to bind to MSLN. In some embodiments, the heavy chain comprises a functional variant of any one of the amino acid sequences shown in SEQ ID NOs: 34-36 and 70-73, formed by inserting, deleting, and/or substituting one or more of the amino acids therein, provided that the antibody containing the VH with the functional variant retains the ability to bind to MSLN.

For example, a functional variant of any one of SEQ ID NOs: 37-43 and 74-76 comprises or consists of an amino acid sequence with at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to SEQ ID NOs: 37-43 and 74-76. For example, a functional variant of any one of SEQ ID NOs: 34-36 and 70-73 comprises or consists of an amino acid sequence with at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to SEQ ID NOs: 34-36 and 70-73.

In some embodiments, the number of inserted, deleted, and/or substituted amino acids preferably does not exceed 40%, more preferably does not exceed 35%, more preferably does not exceed 1-33%, more preferably does not exceed 5-30%, more preferably does not exceed 10-25%, and even more preferably does not exceed 15-20% of the total number of amino acids in the parent amino acid sequence. For example, the number of inserted, deleted, and/or substituted amino acids may be 1-50, preferably 1-20, more preferably 1-10, and even more preferably 1-5. In preferred embodiments, the number of inserted, deleted, and/or substituted amino acids is 1, 2, 3, 4, 5, 6, or 7.

In some embodiments, the insertion, deletion, and/or substitution may be performed in the framework (FR) regions, for example, in FR1, FR2, FR3, and/or FR4; and/or in the constant regions, for example, CL, CH1, CH2, and/or CH3.

In some embodiments, the substitutions of one or more amino acids may involve the conservative substitution of one or more amino acids. Examples of conservative substitutions are as described above.

In preferred embodiments, the light chain comprises the amino acid sequence shown in SEQ ID NO: 37, and the heavy chain comprises the amino acid sequence shown in SEQ ID NO: 34; or the light chain comprises the amino acid sequence shown in SEQ ID NO: 38, and the heavy chain comprises the amino acid sequence shown in SEQ ID NO: 35; or the light chain comprises the amino acid sequence shown in SEQ ID NO: 37, and the heavy chain comprises the amino acid sequence shown in SEQ ID NO: 36; or the light chain comprises the amino acid sequence shown in SEQ ID NO: 39, and the heavy chain comprises the amino acid sequence shown in SEQ ID NO: 34; or the light chain comprises the amino acid sequence shown in SEQ ID NO: 40, and the heavy chain comprises the amino acid sequence shown in SEQ ID NO: 34; or the light chain comprises the amino acid sequence shown in SEQ ID NO: 41, and the heavy chain comprises the amino acid sequence shown in SEQ ID NO: 34; or the light chain comprises the amino acid sequence shown in SEQ ID NO: 42, and the heavy chain comprises the amino acid sequence shown in SEQ ID NO: 34; or the light chain comprises the amino acid sequence shown in SEQ ID NO: 43, and the heavy chain comprises the amino acid sequence shown in SEQ ID NO: 34; or the light chain comprises the amino acid sequence shown in SEQ ID NO: 39, and the heavy chain comprises the amino acid sequence shown in SEQ ID NO: 35; or the light chain comprises the amino acid sequence shown in SEQ ID NO: 74, and the heavy chain comprises the amino acid sequence shown in SEQ ID NO: 70; or the light chain comprises the amino acid sequence shown in SEQ ID NO: 74, and the heavy chain comprises the amino acid sequence shown in SEQ ID NO: 71; or the light chain comprises the amino acid sequence shown in SEQ ID NO: 74, and the heavy chain comprises the amino acid sequence shown in SEQ ID NO: 72; or the light chain comprises the amino acid sequence shown in SEQ ID NO: 75, and the heavy chain comprises the amino acid sequence shown in SEQ ID NO: 72; or the light chain comprises the amino acid sequence shown in SEQ ID NO: 75, and the heavy chain comprises the amino acid sequence shown in SEQ ID NO: 70; or the light chain comprises the amino acid sequence shown in SEQ ID NO: 74, and the heavy chain comprises the amino acid sequence shown in SEQ ID NO: 73; or the light chain comprises the amino acid sequence shown in SEQ ID NO: 76, and the heavy chain comprises the amino acid sequence shown in SEQ ID NO: 71; or the light chain comprises the amino acid sequence shown in SEQ ID NO: 76, and the heavy chain comprises the amino acid sequence shown in SEQ ID NO: 73; or the light chain comprises the amino acid sequence shown in SEQ ID NO: 76, and the heavy chain comprises the amino acid sequence shown in SEQ ID NO: 70; or the light chain comprises the amino acid sequence shown in SEQ ID NO: 76, and the heavy chain comprises the amino acid sequence shown in SEQ ID NO: 72.

In some embodiments, the antibody includes an Fc region. In some embodiments, the Fc region can be of any isotype, including but not limited to IgG1, IgG2, IgG3, and IgG4, and may include one or more mutations or modifications. In one embodiment, the Fc region is of the IgG1 isotype or derived therefrom, optionally having one or more mutations or modifications. In one embodiment, the Fc region is a human IgG1 Fc.

In one embodiment, the Fc region lacks effector functions. For example, the Fc region can be of the IgG1 isotype or a non-IgG1 isotype, such as IgG2, IgG3, or IgG4, that has been mutated to reduce or eliminate its ability to mediate effector functions (e.g., ADCC). For example, see Dall'Acqua WF et al., The Journal of Immunology, 177(2):1129-1138 (2006), and Hezareh M, Journal of Virology, 75(24):12161-12168 (2001). In some embodiments, the Fc region of the antibody comprises a wild-type IgG1 Fc with L234A, L235A, and G237A mutations.

In some embodiments, the antibody is mutated at one or more post-translational modification sites. In one embodiment, the Fc region includes mutations to remove Asn-linked glycosylation receptor sites or is engineered to eliminate the effector functions of the antibody.

Post-translational modifications (PTMs) are widely present in proteins expressed by mammalian cells. In addition to conserved PTM sites in antibodies, such as the conserved N-glycosylation site in the CH2 structure domain of IgG1 antibodies, other PTM sites that occur within the antigen-binding regions (i.e., CDR regions) of antibodies may reduce antigen-binding activity or decrease chemical stability. For example, deamidation or isomerization may lead to molecular instability and heterogeneity. To reduce sequence susceptibility, PTM motifs can be removed through mutation. The VH or VL sequence can be scanned for the presence of PTM motifs, such as isomerization motifs (e.g., DG). Then, "hotspot" residues (e.g., D or G in the DG motif) can be mutated into corresponding residues in the germline sequence or into other residues with similar biophysical properties.

In some embodiments, the antibody-drug conjugate of the present invention comprises an antibody that specifically binds to mesothelin (MSLN), the antibody being a chimeric antibody, a humanized antibody or a human antibody. In some embodiments, the antibody-drug conjugate of the present invention comprises an antibody that specifically binds to mesothelin (MSLN), the antibody being of an isotype selected from IgG, IgA, IgM, IgE, and IgD. In some embodiments, the antibody-drug conjugate of the present invention comprises an antibody that specifically binds to mesothelin (MSLN), the antibody being of a subclass selected from IgG1, IgG2, IgG3, and IgG4.

In some embodiments, the antibody-drug conjugate of the present invention comprises an antigen-binding fragment that specifically binds to mesothelin, wherein the antigen-binding fragment is selected from Fab, Fab', F(ab')₂, Fd, Fd', Fv, scFv, ds-scFv, and dAb.

In some embodiments, the antibody-drug conjugate of the present invention comprises an antibody that specifically binds to mesothelin or an antigen-binding fragment thereof, wherein the antibody is a monoclonal antibody, a bispecific antibody, or a multispecific antibody.

In some embodiments, the antibody-drug conjugate of the present invention comprises an antibody that specifically binds to mesothelin or an antigen-binding fragment thereof, wherein the antibody is monovalent, bivalent, or multivalent.

In some embodiments, the antibody-drug conjugate of the present invention comprises an antibody that specifically binds to mesothelin or an antigen-binding fragment thereof, wherein the antibody or antigen-binding fragment is conjugated to a fluorescent label, a radioactive label, or a cytotoxic agent.

In some embodiments, the antibody-drug conjugate of the present invention comprises an antibody that specifically binds to mesothelin or an antigen-binding fragment thereof, wherein the antibody is a bispecific antibody, the bispecific antibody further comprising a second antigen-binding region, which specifically binds to a tumor-associated antigen, an immune cell antigen, or an immune checkpoint molecule.

In some embodiments, the antibody-drug conjugate of the present invention is the following compound or a tautomer, stereoisomer, or pharmaceutically acceptable salt thereof: 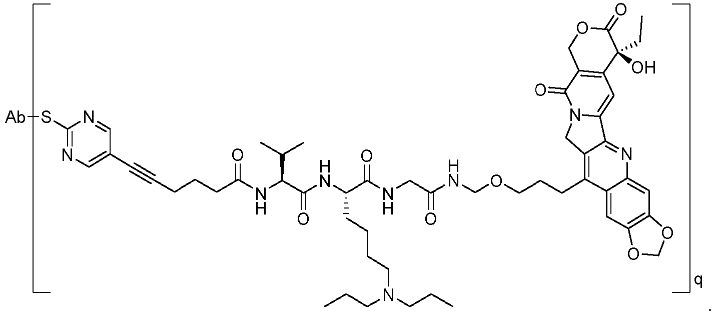
Ab is an anti-MSLN antibody;
q is an integer selected from 1 to 10, for example, 4, 5, 6, 7, 8, 9, or 10, preferably 8.

In some embodiments, the antibody-drug conjugate of the present invention is the following compound or a tautomer, stereoisomer, or pharmaceutically acceptable salt thereof: 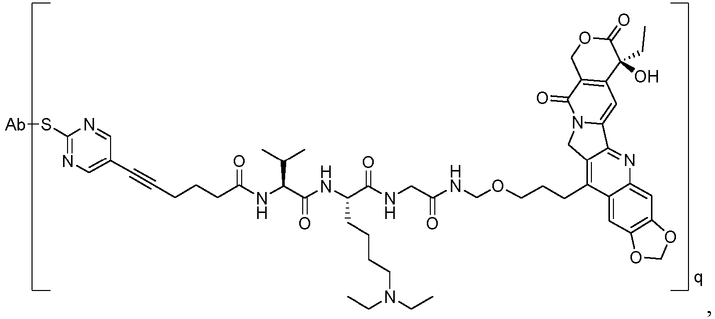
Ab is an anti-MSLN antibody;
q is an integer selected from 1 to 10, for example, 4, 5, 6, 7, 8, 9, or 10, preferably 8.

In some embodiments, the antibody-drug conjugate of the present invention is the following compound or a tautomer, stereoisomer, or pharmaceutically acceptable salt thereof: 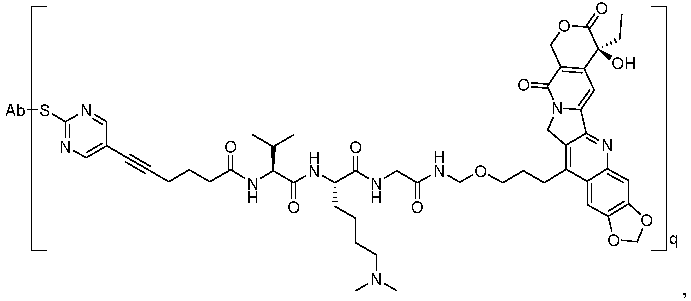
Ab is an anti-MSLN antibody;
q is an integer selected from 1 to 10, for example, 4, 5, 6, 7, 8, 9, or 10, preferably 8.

In some embodiments, the antibody-drug conjugate of the present invention is a compound or a tautomer, stereoisomer, or pharmaceutically acceptable salt thereof: 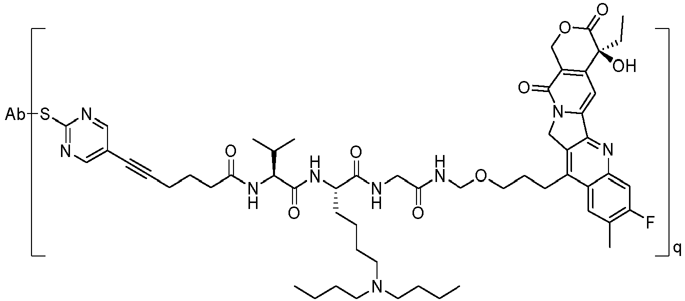
Ab is an anti-MSLN antibody;
q is an integer selected from 1 to 10, for example, 4, 5, 6, 7, 8, 9, or 10, preferably 8.

In some embodiments, the antibody-drug conjugate of the present invention is the following compound or a tautomer, stereoisomer, or pharmaceutically acceptable salt thereof: 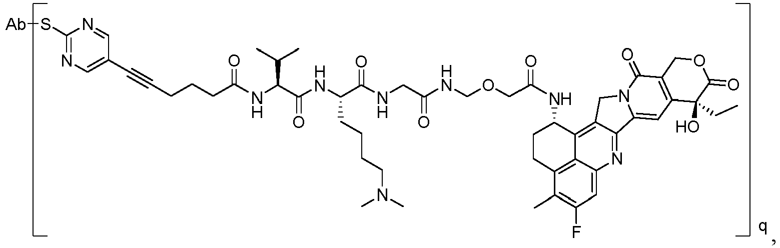
wherein, Ab is an anti-MSLN antibody;
q is an integer selected from 1 to 10, for example, 4, 5, 6, 7, 8, 9, or 10, preferably 8.

In some preferred embodiments, the MSLN antibody comprises a heavy chain (HC) and a light chain (LC), wherein,
(1) the HC comprises the amino acid sequence shown in SEQ ID NO: 34, and the LC comprises the amino acid sequence shown in SEQ ID NO: 37;
(2) the HC comprises the amino acid sequence shown in SEQ ID NO: 34, and the LC comprises the amino acid sequence shown in SEQ ID NO: 43;
(3) the HC comprises the amino acid sequence shown in SEQ ID NO: 70, and the LC comprises the amino acid sequence shown in SEQ ID NO: 74; or
(4) the HC comprises the amino acid sequence shown in SEQ ID NO: 72, and the LC comprises the amino acid sequence shown in SEQ ID NO: 76.

### Bispecific Antibodies

In some embodiments, the antibody in the antibody-drug conjugate of the present invention may be a bispecific or multispecific antibody. In some embodiments, the antibody in the antibody-drug conjugate of the present invention is a bispecific antibody, which comprises an antibody that specifically binds to MSLN or an antigen-binding fragment thereof and further comprises a second antigen-binding domain that binds to a second antigen. In some embodiments, the second antigen may be a tumor-associated antigen, an immune checkpoint molecule, or an immune cell antigen.

Many tumor-associated antigens related to specific cancers have been identified in the art. In some embodiments, the tumor-associated antigen is an antigen capable of stimulating a significant tumor-specific immune response. Some of these antigens are encoded by normal cells but are not necessarily expressed in normal cells. These antigens may be described as antigens that are typically silenced (i.e., not expressed) in normal cells, antigens expressed only at certain stages of differentiation, or antigens expressed over time, such as embryonic and fetal antigens. Other cancer antigens are encoded by mutated cellular genes, such as oncogenes (e.g., activated ras oncogenes), suppressor genes (e.g., mutated p53), and fusion proteins resulting from internal deficiency or chromosomal translocations. Additional cancer antigens may be encoded by viral genes, such as RNA- and DNA-carrying tumor viruses. Many other tumor-associated antigens and antibodies against them are known and/or commercially available and may also be produced by those skilled in the art.

In some embodiments, the bispecific antibody comprises a single polypeptide chain containing a first antigen-binding region, a second antigen-binding region, and optionally an Fc region.

### Pharmaceutical Composition

In a second aspect, the present invention provides a pharmaceutical composition comprising the antibody-drug conjugate described in the first aspect of the present invention, and optionally a pharmaceutically acceptable carrier or excipient.

The present invention provides a pharmaceutical composition that comprises the antibody-drug conjugate of the present invention. In some embodiments, the pharmaceutical composition further comprises a pharmaceutically acceptable carrier. The term "pharmaceutically acceptable carrier" encompasses any and all solvents, buffers, dispersing media, coatings, antibacterial and antifungal agents, isotonic agents, absorption-delaying agents, etc. that are physiologically compatible. Preferably, the carrier is suitable for intravenous, intramuscular, subcutaneous, parenteral, spinal, or topical administration (e.g., by injection or infusion). For instance, in some embodiments, the composition for intravenous administration is typically a solution in a sterile isotonic aqueous buffer.

The pharmaceutical composition of the present invention is formulated to be compatible with the intended route of administration. Examples of administration routes include parenteral administration, such as intravenous injection, intradermal administration, subcutaneous administration, and oral administration (e.g., inhalation), transdermal administration (i.e., local administration), transmucosal administration, and rectal administration. A solution or suspension used for parenteral, intradermal, or subcutaneous applications can include the following components: sterile diluents such as water for injection, saline, fixed oils, polyethylene glycol, glycerin, propylene glycol, or other synthetic solvents; antibacterial agents such as benzyl alcohol or methylparaben; antioxidants such as ascorbic acid or sodium bisulfite; chelating agents such as ethylenediaminetetraacetic acid (EDTA); buffers such as acetates, citrates, or phosphates; and agents for adjusting tonicity, such as sodium chloride or glucose. The pH can be adjusted using acids or bases, such as hydrochloric acid or sodium hydroxide. Parenteral preparations may be packaged in ampoules, disposable syringes, or multi-dose vials made of glass or plastic.

### Treatment Method

In a third aspect, the present invention provides a method for treating cancer in a subject, comprising administering to the subject an effective amount of the antibody-drug conjugate described in the first aspect of the present invention or the pharmaceutical composition described in the second aspect of the present invention. The antibody-drug conjugate provided herein can be used to slow down or inhibit the progression of mesothelin-positive cancers or inhibit the metastasis of mesothelin-positive cancers. In these applications, a therapeutically effective amount of the composition is administered to the subject, in an amount sufficient to inhibit the growth, replication, or metastasis of cancer cells, or to suppress the signs or symptoms of cancer. Suitable subjects may include those diagnosed with cancers expressing mesothelin, such as colon cancer, mesothelioma, ovarian cancer, lung cancer, breast cancer, pancreatic cancer, gastric cancer, prostate cancer, squamous cell carcinoma, and cholangiocarcinoma.

The antibody-drug conjugate disclosed herein may also be administered in combination with other anticancer agents or therapeutic treatments (e.g., the surgical resection of tumors). Any suitable anticancer agents may be administered in combination with the antibody-drug conjugate disclosed herein. Examples of anticancer agents include, but are not limited to, chemotherapeutic agents such as mitotic inhibitors, alkylating agents, antimetabolites, growth factor inhibitors, cell cycle inhibitors, enzymes, topoisomerase inhibitors, anti-survival agents, biological response modifiers, anti-hormonal agents (e.g., anti-androgens), and anti-angiogenic agents. Other anticancer treatments include radiation therapy and other antibodies targeting cancer cells.

Another common treatment method for certain types of cancer is surgical treatment, such as the surgical resection of metastatic tumors. Another example of treatment is radiation therapy, such as the application of radioactive substances or energy (e.g., external beam therapy) to the tumor site to help eradicate or shrink the tumor prior to surgical removal.

In a fourth aspect, the present invention provides the antibody-drug conjugate or pharmaceutical composition of the present invention, wherein same is used for treating cancer. In some embodiments, the cancer is selected from colon cancer, mesothelioma, ovarian cancer, lung cancer, breast cancer, pancreatic cancer, gastric cancer, prostate cancer, squamous cell carcinoma, and cholangiocarcinoma. In some embodiments, the antibody or pharmaceutical composition of the present invention is used in combination with a second therapeutic agent. In some embodiments, the second therapeutic agent is selected from antibodies, chemotherapeutic agents, siRNA, antisense oligonucleotides, polypeptides, and small molecule drugs.

In a fifth aspect, the present invention provides the use of the antibody-drug conjugate or pharmaceutical composition of the present invention in the preparation of a drug for treating cancer. In some embodiments, the cancer is selected from colon cancer, mesothelioma, ovarian cancer, lung cancer, breast cancer, pancreatic cancer, gastric cancer, prostate cancer, squamous cell carcinoma, and cholangiocarcinoma. In some embodiments, the drug is used in combination with a second therapeutic agent. In some embodiments, the second therapeutic agent is selected from antibodies, chemotherapeutic agents, siRNA, antisense oligonucleotides, polypeptides, and small molecule drugs.

### Examples

### Example 1. Preparation of Initial Antibodies and Variants

To obtain MSLN-specific antibodies, Harbour H2L2 transgenic mice (https://harbourantibodies.com/) were immunized using various methods. The immunization produced many antibodies that bind to the MSLN extracellular domain (ECD) protein and MSLN-expressing cells, such as COV644 cells (a mucinous epithelial ovarian carcinoma cell line expressing MSLN).

Specifically, recombinant human MSLN ECD His-tag protein (Acro Biosystem, Catalog #MSN-H5223) or recombinant cynomolgus monkey MSLN ECD His-tag protein (with the amino acid sequence shown in SEQ ID NO: 77) was used as the immunogen to immunize the Harbour H2L2 transgenic mice.

A screening for single B cells was conducted using the Beacon^{®} Optofluidic System, and the heavy chain and light chain sequences of antibodies were obtained from single plasma cells. The general procedure involved extracting and purifying total RNA from single plasma cell lysates, performing reverse transcription to synthesize cDNA, amplifying and purifying the cDNA, amplifying DNA sequences encoding the antibody heavy and light chains, cloning and transfection, and Sanger sequencing. The obtained sequences were subjected to uniqueness and clustering analysis, followed by the synthesis of DNA sequences encoding paired heavy and light chains of the antibody. Initial antibodies PR300159 and PR300186 were obtained.

The VH and VL sequences of the anti-MSLN antibodies PR300159 and PR300186 were further optimized through a PTM (post-translational modification) removal procedure. The designed variants obtained by removing PTMs from PR300159 and PR300186 are shown in Table 2. The amino acid sequences of these anti-MSLN antibodies are shown in Tables 3 to 6. PR300159-1, PR300159-3, PR300159-4, PR300159-5, PR300159-6, PR300159-7, PR300159-8, and PR300159-9 are PTM-removed antibodies derived from PR300159. PR300186-2, PR300186-3, PR300186-4, PR300186-5, PR300186-6, PR300186-7, PR300186-8, PR300186-9, and PR300186-10 are PTM-removed antibodies derived from PR300186.

**Table 2. PTM Variants Derived from PR300159 and PR300186**

| Parent Antibody | Variant Antibody | Mutation Sites | Fc |
|---|---|---|---|
| PR300159 | PR300159-1 | Heavy chain G33A and D54E Light chain N92S | hIgG1 Fc |
| | PR300159-3 | Heavy chain G33A and G55A | hIgG1 Fc |
| | PR300159-4 | Light chain S93A | hIgG1 Fc |
| | PR300159-5 | Light chain N92Q | hIgG1 Fc |
| | PR300159-6 | Light chain S93T | hIgG1 Fc |
| | PR300159-7 | Light chain N92T | hIgG1 Fc |
| | PR300159-8 | Light chain N92A | hIgG1 Fc |
| | PR300159-9 | Heavy chain G33A and D54E Light chain S93A | hIgG1 Fc |
| PR300186 | PR300186-2 | Heavy chain N30Q | hIgG1 Fc |
| | PR300186-3 | Heavy chain N30Q and N58Q | hIgG1 Fc |
| | PR300186-4 | Heavy chain N30Q and N58Q Light chain N30Q | hIgG1 Fc |
| | PR300186-5 | Light chain N30Q | hIgG1 Fc |
| | PR300186-6 | Heavy chain N30Q and S59A | hIgG1 Fc |
| | PR300186-7 | Heavy chain N30Q Light chain S31A | hIgG1 Fc |
| | PR300186-8 | Heavy chain N30Q and S59A Light chain S31A | hIgG1 Fc |
| | PR300186-9 | Light chain S31A | hIgG1 Fc |
| | PR300186-10 | Heavy chain N30Q and N58Q Light chain S31A | hIgG1 Fc |

**Table 3. Amino Acid Sequences of Heavy Chain and Light Chain of Antibodies**

| Clone | Heavy Chain | SEQ ID NO | Light Chain | SEQ ID NO |
|---|---|---|---|---|
| PR3001 59 | QVQLVESGGGVVQPGRSLRLSCAAS GFTFSSDGMNWVRQAPGKGLEWVA VIWNDGSNKYYADSVKGRFTISRDN SKNTLYLQMNSLRAEDTAVYYCAR DRGPLWPLDYWGQGTLVTVSSAST KGPSVFPLAPSSKSTSGGTAALGCLV KDYFPEPVTVSWNSGALTSGVHTFP AVLQSSGLYSLSSVVTVPSSSLGTQT YICNVNHKPSNTKVDKKVEPKSCDK THTCPPCPAPELLGGPSVFLFPPKPKD TLMISRTPEVTCVVVDVSHEDPEVKF NWYVDGVEVHNAKTKPREEQYNST YRVVSVLTVLHQDWLNGKEYKCKV SNKALPAPIEKTISKAKGQPREPQVY TLPPSREEMTKNQVSLTCLVKGFYPS DIAVEWESNGQPENNYKTTPPVLDS DGSFFLYSKLTVDKSRWQQGNVFSC SVMHEALHNHYTQKSLSLSPGK | 34 | DIQMTQSPSTLSASVGDRVTITCRASQ SISRWLAWYQQKPGKAPKLLIYKASSL ESGVPSRFSGSGSGTEFTLTISNLQPDD FATFYCQQYNSYSRTFGQGTKVEIKRT VAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 37 |
| PR3001 59-1 | QVQLVESGGGVVQPGRSLRLSCAAS GFTFSSDAMNWVRQAPGKGLEWVA VIWNEGSNKYYADSVKGRFTISRDN SKNTLYLQMNSLRAEDTAVYYCAR DRGPLWPLDYWGQGTLVTVSSAST KGPSVFPLAPSSKSTSGGTAALGCLV KDYFPEPVTVSWNSGALTSGVHTFP AVLQSSGLYSLSSVVTVPSSSLGTQT YICNVNHKPSNTKVDKKVEPKSCDK THTCPPCPAPELLGGPSVFLFPPKPKD TLMISRTPEVTCVVVDVSHEDPEVKF NWYVDGVEVHNAKTKPREEQYNST YRVVSVLTVLHQDWLNGKEYKCKV SNKALPAPIEKTISKAKGQPREPQVY TLPPSREEMTKNQVSLTCLVKGFYPS DIAVEWESNGQPENNYKTTPPVLDS DGSFFLYSKLTVDKSRWQQGNVFSC SVMHEALHNHYTQKSLSLSPGK | 35 | DIQMTQSPSTLSASVGDRVTITCRASQ SISRWLAWYQQKPGKAPKLLIYKASSL ESGVPSRFSGSGSGTEFTLTISNLQPDD FATFYCQQYSSYSRTFGQGTKVEIKRT VAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 38 |
| PR3001 59-3 | QVQLVESGGGVVQPGRSLRLSCAAS GFTFSSDAMNWVRQAPGKGLEWVA VIWNDASNKYYADSVKGRFTISRDN SKNTLYLQMNSLRAEDTAVYYCAR DRGPLWPLDYWGQGTLVTVSSAST KGPSVFPLAPSSKSTSGGTAALGCLV KDYFPEPVTVSWNSGALTSGVHTFP AVLQSSGLYSLSSVVTVPSSSLGTQT YICNVNHKPSNTKVDKKVEPKSCDK THTCPPCPAPELLGGPSVFLFPPKPKD TLMISRTPEVTCVVVDVSHEDPEVKF NWYVDGVEVHNAKTKPREEQYNST YRVVSVLTVLHQDWLNGKEYKCKV SNKALPAPIEKTISKAKGQPREPQVY TLPPSREEMTKNQVSLTCLVKGFYPS DIAVEWESNGQPENNYKTTPPVLDS DGSFFLYSKLTVDKSRWQQGNVFSC SVMHEALHNHYTQKSLSLSPGK | 36 | DIQMTQSPSTLSASVGDRVTITCRASQ SISRWLAWYQQKPGKAPKLLIYKASSL ESGVPSRFSGSGSGTEFTLTISNLQPDD FATFYCQQYNSYSRTFGQGTKVEIKRT VAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 37 |
| PR3001 59-4 | QVQLVESGGGVVQPGRSLRLSCAAS GFTFSSDGMNWVRQAPGKGLEWVA VIWNDGSNKYYADSVKGRFTISRDN SKNTLYLQMNSLRAEDTAVYYCAR DRGPLWPLDYWGQGTLVTVSSAST KGPSVFPLAPSSKSTSGGTAALGCLV KDYFPEPVTVSWNSGALTSGVHTFP AVLQSSGLYSLSSVVTVPSSSLGTQT YICNVNHKPSNTKVDKKVEPKSCDK THTCPPCPAPELLGGPSVFLFPPKPKD TLMISRTPEVTCVVVDVSHEDPEVKF NWYVDGVEVHNAKTKPREEQYNST YRVVSVLTVLHQDWLNGKEYKCKV SNKALPAPIEKTISKAKGQPREPQVY TLPPSREEMTKNQVSLTCLVKGFYPS DIAVEWESNGQPENNYKTTPPVLDS DGSFFLYSKLTVDKSRWQQGNVFSC SVMHEALHNHYTQKSLSLSPGK | 34 | DIQMTQSPSTLSASVGDRVTITCRASQ SISRWLAWYQQKPGKAPKLLIYKASSL ESGVPSRFSGSGSGTEFTLTISNLQPDD FATFYCQQYNAYSRTFGQGTKVEIKR TVAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 39 |
| PR3001 59-5 | QVQLVESGGGVVQPGRSLRLSCAAS GFTFSSDGMNWVRQAPGKGLEWVA VIWNDGSNKYYADSVKGRFTISRDN SKNTLYLQMNSLRAEDTAVYYCAR DRGPLWPLDYWGQGTLVTVSSAST KGPSVFPLAPSSKSTSGGTAALGCLV KDYFPEPVTVSWNSGALTSGVHTFP AVLQSSGLYSLSSVVTVPSSSLGTQT YICNVNHKPSNTKVDKKVEPKSCDK THTCPPCPAPELLGGPSVFLFPPKPKD TLMISRTPEVTCVVVDVSHEDPEVKF NWYVDGVEVHNAKTKPREEQYNST YRVVSVLTVLHQDWLNGKEYKCKV SNKALPAPIEKTISKAKGQPREPQVY TLPPSREEMTKNQVSLTCLVKGFYPS DIAVEWESNGQPENNYKTTPPVLDS DGSFFLYSKLTVDKSRWQQGNVFSC SVMHEALHNHYTQKSLSLSPGK | 34 | DIQMTQSPSTLSASVGDRVTITCRASQ SISRWLAWYQQKPGKAPKLLIYKASSL ESGVPSRFSGSGSGTEFTLTISNLQPDD FATFYCQQYQSYSRTFGQGTKVEIKRT VAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 40 |
| PR3001 59-6 | QVQLVESGGGVVQPGRSLRLSCAAS GFTFSSDGMNWVRQAPGKGLEWVA VIWNDGSNKYYADSVKGRFTISRDN SKNTLYLQMNSLRAEDTAVYYCAR DRGPLWPLDYWGQGTLVTVSSAST KGPSVFPLAPSSKSTSGGTAALGCLV KDYFPEPVTVSWNSGALTSGVHTFP AVLQSSGLYSLSSVVTVPSSSLGTQT YICNVNHKPSNTKVDKKVEPKSCDK THTCPPCPAPELLGGPSVFLFPPKPKD TLMISRTPEVTCVVVDVSHEDPEVKF NWYVDGVEVHNAKTKPREEQYNST YRVVSVLTVLHQDWLNGKEYKCKV SNKALPAPIEKTISKAKGQPREPQVY TLPPSREEMTKNQVSLTCLVKGFYPS DIAVEWESNGQPENNYKTTPPVLDS DGSFFLYSKLTVDKSRWQQGNVFSC SVMHEALHNHYTQKSLSLSPGK | 34 | DIQMTQSPSTLSASVGDRVTITCRASQ SISRWLAWYQQKPGKAPKLLIYKASSL ESGVPSRFSGSGSGTEFTLTISNLQPDD FATFYCQQYNTYSRTFGQGTKVEIKRT VAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 41 |
| PR3001 59-7 | QVQLVESGGGVVQPGRSLRLSCAAS GFTFSSDGMNWVRQAPGKGLEWVA VIWNDGSNKYYADSVKGRFTISRDN SKNTLYLQMNSLRAEDTAVYYCAR DRGPLWPLDYWGQGTLVTVSSAST KGPSVFPLAPSSKSTSGGTAALGCLV KDYFPEPVTVSWNSGALTSGVHTFP AVLQSSGLYSLSSVVTVPSSSLGTQT YICNVNHKPSNTKVDKKVEPKSCDK THTCPPCPAPELLGGPSVFLFPPKPKD TLMISRTPEVTCVVVDVSHEDPEVKF NWYVDGVEVHNAKTKPREEQYNST YRVVSVLTVLHQDWLNGKEYKCKV SNKALPAPIEKTISKAKGQPREPQVY TLPPSREEMTKNQVSLTCLVKGFYPS DIAVEWESNGQPENNYKTTPPVLDS DGSFFLYSKLTVDKSRWQQGNVFSC SVMHEALHNHYTQKSLSLSPGK | 34 | DIQMTQSPSTLSASVGDRVTITCRASQ SISRWLAWYQQKPGKAPKLLIYKASSL ESGVPSRFSGSGSGTEFTLTISNLQPDD FATFYCQQYTSYSRTFGQGTKVEIKRT VAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 42 |
| PR3001 59-8 | QVQLVESGGGVVQPGRSLRLSCAAS GFTFSSDGMNWVRQAPGKGLEWVA VIWNDGSNKYYADSVKGRFTISRDN SKNTLYLQMNSLRAEDTAVYYCAR DRGPLWPLDYWGQGTLVTVSSAST KGPSVFPLAPSSKSTSGGTAALGCLV KDYFPEPVTVSWNSGALTSGVHTFP AVLQSSGLYSLSSVVTVPSSSLGTQT YICNVNHKPSNTKVDKKVEPKSCDK THTCPPCPAPELLGGPSVFLFPPKPKD TLMISRTPEVTCVVVDVSHEDPEVKF NWYVDGVEVHNAKTKPREEQYNST YRVVSVLTVLHQDWLNGKEYKCKV SNKALPAPIEKTISKAKGQPREPQVY TLPPSREEMTKNQVSLTCLVKGFYPS DIAVEWESNGQPENNYKTTPPVLDS DGSFFLYSKLTVDKSRWQQGNVFSC SVMHEALHNHYTQKSLSLSPGK | 34 | DIQMTQSPSTLSASVGDRVTITCRASQ SISRWLAWYQQKPGKAPKLLIYKASSL ESGVPSRFSGSGSGTEFTLTISNLQPDD FATFYCQQYASYSRTFGQGTKVEIKRT VAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 43 |
| PR3001 59-9 | QVQLVESGGGVVQPGRSLRLSCAAS GFTFSSDAMNWVRQAPGKGLEWVA VIWNEGSNKYYADSVKGRFTISRDN SKNTLYLQMNSLRAEDTAVYYCAR DRGPLWPLDYWGQGTLVTVSSAST KGPSVFPLAPSSKSTSGGTAALGCLV KDYFPEPVTVSWNSGALTSGVHTFP AVLQSSGLYSLSSVVTVPSSSLGTQT YICNVNHKPSNTKVDKKVEPKSCDK THTCPPCPAPELLGGPSVFLFPPKPKD TLMISRTPEVTCVVVDVSHEDPEVKF NWYVDGVEVHNAKTKPREEQYNST YRVVSVLTVLHQDWLNGKEYKCKV SNKALPAPIEKTISKAKGQPREPQVY TLPPSREEMTKNQVSLTCLVKGFYPS DIAVEWESNGQPENNYKTTPPVLDS DGSFFLYSKLTVDKSRWQQGNVFSC SVMHEALHNHYTQKSLSLSPGK | 35 | DIQMTQSPSTLSASVGDRVTITCRASQ SISRWLAWYQQKPGKAPKLLIYKASSL ESGVPSRFSGSGSGTEFTLTISNLQPDD FATFYCQQYNAYSRTFGQGTKVEIKR TVAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 39 |
| PR3001 86 | QVQLQESGPGLVKPSETLSLTCTVSG GSINNYYWSWVRQPPGKGLEWIGYI FYSGSTNSNPSLKSRVIISVDTSKNQF SLKLSSVTAADTAVYYCAREVGRIA VAGWFFDLWGRGTLVTVSSASTKGP SVFPLAPSSKSTSGGTAALGCLVKDY FPEPVTVSWNSGALTSGVHTFPAVL QSSGLYSLSSVVTVPSSSLGTQTYICN VNHKPSNTKVDKKVEPKSCDKTHTC PPCPAPELLGGPSVFLFPPKPKDTLMI SRTPEVTCVVVDVSHEDPEVKFNWY VDGVEVHNAKTKPREEQYNSTYRV VSVLTVLHQDWLNGKEYKCKVSNK ALPAPIEKTISKAKGQPREPQVYTLPP SREEMTKNQVSLTCLVKGFYPSDIA VEWESNGQPENNYKTTPPVLDSDGS FFLYSKLTVDKSRWQQGNVFSCSVM HEALHNHYTQKSLSLSPGK | 70 | EIVMTQSPATLSVSPGERATLSCRASQ SVNSNLAWYQQKPGQAPRLLIYGAST RATGIPARFSGSGSGTEFTLTISSLQSED FAVYYCQQYNNWPLTFGQGTRLEIKR TVAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 74 |
| PR3001 86-2 | QVQLQESGPGLVKPSETLSLTCTVSG GSIQNYYWSWVRQPPGKGLEWIGYI FYSGSTNSNPSLKSRVIISVDTSKNQF SLKLSSVTAADTAVYYCAREVGRIA VAGWFFDLWGRGTLVTVSSASTKGP SVFPLAPSSKSTSGGTAALGCLVKDY FPEPVTVSWNSGALTSGVHTFPAVL QSSGLYSLSSVVTVPSSSLGTQTYICN VNHKPSNTKVDKKVEPKSCDKTHTC PPCPAPELLGGPSVFLFPPKPKDTLMI SRTPEVTCVVVDVSHEDPEVKFNWY VDGVEVHNAKTKPREEQYNSTYRV VSVLTVLHQDWLNGKEYKCKVSNK ALPAPIEKTISKAKGQPREPQVYTLPP SREEMTKNQVSLTCLVKGFYPSDIA VEWESNGQPENNYKTTPPVLDSDGS FFLYSKLTVDKSRWQQGNVFSCSVM HEALHNHYTQKSLSLSPGK | 71 | EIVMTQSPATLSVSPGERATLSCRASQ SVNSNLAWYQQKPGQAPRLLIYGAST RATGIPARFSGSGSGTEFTLTISSLQSED FAVYYCQQYNNWPLTFGQGTRLEIKR TVAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 74 |
| PR3001 86-3 | QVQLQESGPGLVKPSETLSLTCTVSG GSIQNYYWSWVRQPPGKGLEWIGYI FYSGSTQSNPSLKSRVIISVDTSKNQF SLKLSSVTAADTAVYYCAREVGRIA VAGWFFDLWGRGTLVTVSSASTKGP SVFPLAPSSKSTSGGTAALGCLVKDY FPEPVTVSWNSGALTSGVHTFPAVL QSSGLYSLSSVVTVPSSSLGTQTYICN VNHKPSNTKVDKKVEPKSCDKTHTC PPCPAPELLGGPSVFLFPPKPKDTLMI SRTPEVTCVVVDVSHEDPEVKFNWY VDGVEVHNAKTKPREEQYNSTYRV VSVLTVLHQDWLNGKEYKCKVSNK ALPAPIEKTISKAKGQPREPQVYTLPP SREEMTKNQVSLTCLVKGFYPSDIA VEWESNGQPENNYKTTPPVLDSDGS FFLYSKLTVDKSRWQQGNVFSCSVM HEALHNHYTQKSLSLSPGK | 72 | EIVMTQSPATLSVSPGERATLSCRASQ SVNSNLAWYQQKPGQAPRLLIYGAST RATGIPARFSGSGSGTEFTLTISSLQSED FAVYYCQQYNNWPLTFGQGTRLEIKR TVAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 74 |
| PR3001 86-4 | QVQLQESGPGLVKPSETLSLTCTVSG GSIQNYYWSWVRQPPGKGLEWIGYI FYSGSTQSNPSLKSRVIISVDTSKNQF SLKLSSVTAADTAVYYCAREVGRIA VAGWFFDLWGRGTLVTVSSASTKGP SVFPLAPSSKSTSGGTAALGCLVKDY FPEPVTVSWNSGALTSGVHTFPAVL QSSGLYSLSSVVTVPSSSLGTQTYICN VNHKPSNTKVDKKVEPKSCDKTHTC PPCPAPELLGGPSVFLFPPKPKDTLMI SRTPEVTCVVVDVSHEDPEVKFNWY VDGVEVHNAKTKPREEQYNSTYRV VSVLTVLHQDWLNGKEYKCKVSNK ALPAPIEKTISKAKGQPREPQVYTLPP SREEMTKNQVSLTCLVKGFYPSDIA VEWESNGQPENNYKTTPPVLDSDGS FFLYSKLTVDKSRWQQGNVFSCSVM HEALHNHYTQKSLSLSPGK | 72 | EIVMTQSPATLSVSPGERATLSCRASQ SVQSNLAWYQQKPGQAPRLLIYGAST RATGIPARFSGSGSGTEFTLTISSLQSED FAVYYCQQYNNWPLTFGQGTRLEIKR TVAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 75 |
| PR3001 86-5 | QVQLQESGPGLVKPSETLSLTCTVSG GSINNYYWSWVRQPPGKGLEWIGYI FYSGSTNSNPSLKSRVIISVDTSKNQF SLKLSSVTAADTAVYYCAREVGRIA VAGWFFDLWGRGTLVTVSSASTKGP SVFPLAPSSKSTSGGTAALGCLVKDY FPEPVTVSWNSGALTSGVHTFPAVL QSSGLYSLSSVVTVPSSSLGTQTYICN VNHKPSNTKVDKKVEPKSCDKTHTC PPCPAPELLGGPSVFLFPPKPKDTLMI SRTPEVTCVVVDVSHEDPEVKFNWY VDGVEVHNAKTKPREEQYNSTYRV VSVLTVLHQDWLNGKEYKCKVSNK ALPAPIEKTISKAKGQPREPQVYTLPP SREEMTKNQVSLTCLVKGFYPSDIA VEWESNGQPENNYKTTPPVLDSDGS FFLYSKLTVDKSRWQQGNVFSCSVM HEALHNHYTQKSLSLSPGK | 70 | EIVMTQSPATLSVSPGERATLSCRASQ SVQSNLAWYQQKPGQAPRLLIYGAST RATGIPARFSGSGSGTEFTLTISSLQSED FAVYYCQQYNNWPLTFGQGTRLEIKR TVAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 75 |
| PR3001 86-6 | QVQLQESGPGLVKPSETLSLTCTVSG GSIQNYYWSWVRQPPGKGLEWIGYI FYSGSTNANPSLKSRVIISVDTSKNQF SLKLSSVTAADTAVYYCAREVGRIA VAGWFFDLWGRGTLVTVSSASTKGP SVFPLAPSSKSTSGGTAALGCLVKDY FPEPVTVSWNSGALTSGVHTFPAVL QSSGLYSLSSVVTVPSSSLGTQTYICN VNHKPSNTKVDKKVEPKSCDKTHTC PPCPAPELLGGPSVFLFPPKPKDTLMI SRTPEVTCVVVDVSHEDPEVKFNWY VDGVEVHNAKTKPREEQYNSTYRV VSVLTVLHQDWLNGKEYKCKVSNK ALPAPIEKTISKAKGQPREPQVYTLPP SREEMTKNQVSLTCLVKGFYPSDIA VEWESNGQPENNYKTTPPVLDSDGS FFLYSKLTVDKSRWQQGNVFSCSVM HEALHNHYTQKSLSLSPGK | 73 | EIVMTQSPATLSVSPGERATLSCRASQ SVNSNLAWYQQKPGQAPRLLIYGAST RATGIPARFSGSGSGTEFTLTISSLQSED FAVYYCQQYNNWPLTFGQGTRLEIKR TVAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 74 |
| PR3001 86-7 | QVQLQESGPGLVKPSETLSLTCTVSG GSIQNYYWSWVRQPPGKGLEWIGYI FYSGSTNSNPSLKSRVIISVDTSKNQF SLKLSSVTAADTAVYYCAREVGRIA VAGWFFDLWGRGTLVTVSSASTKGP SVFPLAPSSKSTSGGTAALGCLVKDY FPEPVTVSWNSGALTSGVHTFPAVL QSSGLYSLSSVVTVPSSSLGTQTYICN VNHKPSNTKVDKKVEPKSCDKTHTC PPCPAPELLGGPSVFLFPPKPKDTLMI SRTPEVTCVVVDVSHEDPEVKFNWY VDGVEVHNAKTKPREEQYNSTYRV VSVLTVLHQDWLNGKEYKCKVSNK ALPAPIEKTISKAKGQPREPQVYTLPP SREEMTKNQVSLTCLVKGFYPSDIA VEWESNGQPENNYKTTPPVLDSDGS FFLYSKLTVDKSRWQQGNVFSCSVM HEALHNHYTQKSLSLSPGK | 71 | EIVMTQSPATLSVSPGERATLSCRASQ SVNANLAWYQQKPGQAPRLLIYGAST RATGIPARFSGSGSGTEFTLTISSLQSED FAVYYCQQYNNWPLTFGQGTRLEIKR TVAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 76 |
| PR3001 86-8 | QVQLQESGPGLVKPSETLSLTCTVSG GSIQNYYWSWVRQPPGKGLEWIGYI FYSGSTNANPSLKSRVIISVDTSKNQF SLKLSSVTAADTAVYYCAREVGRIA VAGWFFDLWGRGTLVTVSSASTKGP SVFPLAPSSKSTSGGTAALGCLVKDY FPEPVTVSWNSGALTSGVHTFPAVL QSSGLYSLSSVVTVPSSSLGTQTYICN VNHKPSNTKVDKKVEPKSCDKTHTC PPCPAPELLGGPSVFLFPPKPKDTLMI SRTPEVTCVVVDVSHEDPEVKFNWY VDGVEVHNAKTKPREEQYNSTYRV VSVLTVLHQDWLNGKEYKCKVSNK ALPAPIEKTISKAKGQPREPQVYTLPP SREEMTKNQVSLTCLVKGFYPSDIA VEWESNGQPENNYKTTPPVLDSDGS FFLYSKLTVDKSRWQQGNVFSCSVM HEALHNHYTQKSLSLSPGK | 73 | EIVMTQSPATLSVSPGERATLSCRASQ SVNANLAWYQQKPGQAPRLLIYGAST RATGIPARFSGSGSGTEFTLTISSLQSED FAVYYCQQYNNWPLTFGQGTRLEIKR TVAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 76 |
| PR3001 86-9 | QVQLQESGPGLVKPSETLSLTCTVSG GSINNYYWSWVRQPPGKGLEWIGYI FYSGSTNSNPSLKSRVIISVDTSKNQF SLKLSSVTAADTAVYYCAREVGRIA VAGWFFDLWGRGTLVTVSSASTKGP SVFPLAPSSKSTSGGTAALGCLVKDY FPEPVTVSWNSGALTSGVHTFPAVL QSSGLYSLSSVVTVPSSSLGTQTYICN VNHKPSNTKVDKKVEPKSCDKTHTC PPCPAPELLGGPSVFLFPPKPKDTLMI SRTPEVTCVVVDVSHEDPEVKFNWY VDGVEVHNAKTKPREEQYNSTYRV VSVLTVLHQDWLNGKEYKCKVSNK ALPAPIEKTISKAKGQPREPQVYTLPP SREEMTKNQVSLTCLVKGFYPSDIA VEWESNGQPENNYKTTPPVLDSDGS FFLYSKLTVDKSRWQQGNVFSCSVM HEALHNHYTQKSLSLSPGK | 70 | EIVMTQSPATLSVSPGERATLSCRASQ SVNANLAWYQQKPGQAPRLLIYGAST RATGIPARFSGSGSGTEFTLTISSLQSED FAVYYCQQYNNWPLTFGQGTRLEIKR TVAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 76 |
| PR3001 86-10 | QVQLQESGPGLVKPSETLSLTCTVSG GSIQNYYWSWVRQPPGKGLEWIGYI FYSGSTQSNPSLKSRVIISVDTSKNQF SLKLSSVTAADTAVYYCAREVGRIA VAGWFFDLWGRGTLVTVSSASTKGP SVFPLAPSSKSTSGGTAALGCLVKDY FPEPVTVSWNSGALTSGVHTFPAVL QSSGLYSLSSVVTVPSSSLGTQTYICN VNHKPSNTKVDKKVEPKSCDKTHTC PPCPAPELLGGPSVFLFPPKPKDTLMI SRTPEVTCVVVDVSHEDPEVKFNWY VDGVEVHNAKTKPREEQYNSTYRV VSVLTVLHQDWLNGKEYKCKVSNK ALPAPIEKTISKAKGQPREPQVYTLPP SREEMTKNQVSLTCLVKGFYPSDIA VEWESNGQPENNYKTTPPVLDSDGS FFLYSKLTVDKSRWQQGNVFSCSVM HEALHNHYTQKSLSLSPGK | 72 | EIVMTQSPATLSVSPGERATLSCRASQ SVNANLAWYQQKPGQAPRLLIYGAST RATGIPARFSGSGSGTEFTLTISSLQSED FAVYYCQQYNNWPLTFGQGTRLEIKR TVAAPSVFIFPPSDEQLKSGTASVVCLL NNFYPREAKVQWKVDNALQSGNSQE SVTEQDSKDSTYSLSSTLTLSKADYEK HKVYACEVTHQGLSSPVTKSFNRGEC | 76 |

**Table 4. Amino Acid Sequences of Heavy Chain Variable Regions and Light Chain Variable Regions of Antibodies of the Present Invention**

| Clone | Heavy Chain Variable Region | SEQ ID NO | Light Chain Variable Region | SEQ ID NO |
|---|---|---|---|---|
| PR3001 59 | QVQLVESGGGVVQPGRSLRLSCAASGFT FSSDGMNWVRQAPGKGLEWVAVIWND GSNKYYADSVKGRFTISRDNSKNTLYLQ MNSLRAEDTAVYYCARDRGPLWPLDY WGQGTLVTVSS | 24 | DIQMTQSPSTLSASVGDRVTITCRAS QSISRWLAWYQQKPGKAPKLLIYKA SSLESGVPSRFSGSGSGTEFTLTISNL QPDDFATFYCQQYNSYSRTFGQGTK VEIK | 27 |
| PR3001 59-1 | QVQLVESGGGVVQPGRSLRLSCAASGFT FSSDAMNWVRQAPGKGLEWVAVIWNE GSNKYYADSVKGRFTISRDNSKNTLYLQ MNSLRAEDTAVYYCARDRGPLWPLDY WGQGTLVTVSS | 25 | DIQMTQSPSTLSASVGDRVTITCRAS QSISRWLAWYQQKPGKAPKLLIYKA SSLESGVPSRFSGSGSGTEFTLTISNL QPDDFATFYCQQYSSYSRTFGQGTK VEIK | 28 |
| PR3001 59-3 | QVQLVESGGGVVQPGRSLRLSCAASGFT FSSDAMNWVRQAPGKGLEWVAVIWND ASNKYYADSVKGRFTISRDNSKNTLYLQ MNSLRAEDTAVYYCARDRGPLWPLDY WGQGTLVTVSS | 26 | DIQMTQSPSTLSASVGDRVTITCRAS QSISRWLAWYQQKPGKAPKLLIYKA SSLESGVPSRFSGSGSGTEFTLTISNL QPDDFATFYCQQYNSYSRTFGQGTK VEIK | 27 |
| PR3001 59-4 | QVQLVESGGGVVQPGRSLRLSCAASGFT FSSDGMNWVRQAPGKGLEWVAVIWND GSNKYYADSVKGRFTISRDNSKNTLYLQ MNSLRAEDTAVYYCARDRGPLWPLDY WGQGTLVTVSS | 24 | DIQMTQSPSTLSASVGDRVTITCRAS QSISRWLAWYQQKPGKAPKLLIYKA SSLESGVPSRFSGSGSGTEFTLTISNL QPDDFATFYCQQYNAYSRTFGQGTK VEIK | 29 |
| PR3001 59-5 | QVQLVESGGGVVQPGRSLRLSCAASGFT FSSDGMNWVRQAPGKGLEWVAVIWND GSNKYYADSVKGRFTISRDNSKNTLYLQ MNSLRAEDTAVYYCARDRGPLWPLDY WGQGTLVTVSS | 24 | DIQMTQSPSTLSASVGDRVTITCRAS QSISRWLAWYQQKPGKAPKLLIYKA SSLESGVPSRFSGSGSGTEFTLTISNL QPDDFATFYCQQYQSYSRTFGQGTK VEIK | 30 |
| PR3001 59-6 | QVQLVESGGGVVQPGRSLRLSCAASGFT FSSDGMNWVRQAPGKGLEWVAVIWND GSNKYYADSVKGRFTISRDNSKNTLYLQ MNSLRAEDTAVYYCARDRGPLWPLDY WGQGTLVTVSS | 24 | DIQMTQSPSTLSASVGDRVTITCRAS QSISRWLAWYQQKPGKAPKLLIYKA SSLESGVPSRFSGSGSGTEFTLTISNL QPDDFATFYCQQYNTYSRTFGQGTK VEIK | 31 |
| PR3001 59-7 | QVQLVESGGGVVQPGRSLRLSCAASGFT FSSDGMNWVRQAPGKGLEWVAVIWND GSNKYYADSVKGRFTISRDNSKNTLYLQ MNSLRAEDTAVYYCARDRGPLWPLDY WGQGTLVTVSS | 24 | DIQMTQSPSTLSASVGDRVTITCRAS QSISRWLAWYQQKPGKAPKLLIYKA SSLESGVPSRFSGSGSGTEFTLTISNL QPDDFATFYCQQYTSYSRTFGQGTK VEIK | 32 |
| PR3001 59-8 | QVQLVESGGGVVQPGRSLRLSCAASGFT FSSDGMNWVRQAPGKGLEWVAVIWND GSNKYYADSVKGRFTISRDNSKNTLYLQ MNSLRAEDTAVYYCARDRGPLWPLDY WGQGTLVTVSS | 24 | DIQMTQSPSTLSASVGDRVTITCRAS QSISRWLAWYQQKPGKAPKLLIYKA SSLESGVPSRFSGSGSGTEFTLTISNL QPDDFATFYCQQYASYSRTFGQGTK VEIK | 33 |
| PR3001 59-9 | QVQLVESGGGVVQPGRSLRLSCAASGFT FSSDAMNWVRQAPGKGLEWVAVIWNE GSNKYYADSVKGRFTISRDNSKNTLYLQ MNSLRAEDTAVYYCARDRGPLWPLDY WGQGTLVTVSS | 25 | DIQMTQSPSTLSASVGDRVTITCRAS QSISRWLAWYQQKPGKAPKLLIYKA SSLESGVPSRFSGSGSGTEFTLTISNL QPDDFATFYCQQYNAYSRTFGQGTK VEIK | 29 |
| PR3001 86 | QVQLQESGPGLVKPSETLSLTCTVSGGSI NNYYWSWVRQPPGKGLEWIGYIFYSGS TNSNPSLKSRVIISVDTSKNQFSLKLSSVT AADTAVYYCAREVGRIAVAGWFFDLW GRGTLVTVSS | 63 | EIVMTQSPATLSVSPGERATLSCRAS QSVNSNLAWYQQKPGQAPRLLIYGA STRATGIPARFSGSGSGTEFTLTISSL QSEDFAVYYCQQYNNWPLTFGQGT RLEIK | 67 |
| PR3001 86-2 | QVQLQESGPGLVKPSETLSLTCTVSGGSI QNYYWSWVRQPPGKGLEWIGYIFYSGS TNSNPSLKSRVIISVDTSKNQFSLKLSSVT AADTAVYYCAREVGRIAVAGWFFDLW GRGTLVTVSS | 64 | EIVMTQSPATLSVSPGERATLSCRAS QSVNSNLAWYQQKPGQAPRLLIYGA STRATGIPARFSGSGSGTEFTLTISSL QSEDFAVYYCQQYNNWPLTFGQGT RLEIK | 67 |
| PR3001 86-3 | QVQLQESGPGLVKPSETLSLTCTVSGGSI QNYYWSWVRQPPGKGLEWIGYIFYSGS TQSNPSLKSRVIISVDTSKNQFSLKLSSVT AADTAVYYCAREVGRIAVAGWFFDLW GRGTLVTVSS | 65 | EIVMTQSPATLSVSPGERATLSCRAS QSVNSNLAWYQQKPGQAPRLLIYGA STRATGIPARFSGSGSGTEFTLTISSL QSEDFAVYYCQQYNNWPLTFGQGT RLEIK | 67 |
| PR3001 86-4 | QVQLQESGPGLVKPSETLSLTCTVSGGSI QNYYWSWVRQPPGKGLEWIGYIFYSGS TQSNPSLKSRVIISVDTSKNQFSLKLSSVT AADTAVYYCAREVGRIAVAGWFFDLW GRGTLVTVSS | 65 | EIVMTQSPATLSVSPGERATLSCRAS QSVQSNLAWYQQKPGQAPRLLIYGA STRATGIPARFSGSGSGTEFTLTISSL QSEDFAVYYCQQYNNWPLTFGQGT RLEIK | 68 |
| PR3001 86-5 | QVQLQESGPGLVKPSETLSLTCTVSGGSI NNYYWSWVRQPPGKGLEWIGYIFYSGS TNSNPSLKSRVIISVDTSKNQFSLKLSSVT AADTAVYYCAREVGRIAVAGWFFDLW GRGTLVTVSS | 63 | EIVMTQSPATLSVSPGERATLSCRAS QSVQSNLAWYQQKPGQAPRLLIYGA STRATGIPARFSGSGSGTEFTLTISSL QSEDFAVYYCQQYNNWPLTFGQGT RLEIK | 68 |
| PR3001 86-6 | QVQLQESGPGLVKPSETLSLTCTVSGGSI QNYYWSWVRQPPGKGLEWIGYIFYSGS TNANPSLKSRVIISVDTSKNQFSLKLSSV TAADTAVYYCAREVGRIAVAGWFFDL WGRGTLVTVSS | 66 | EIVMTQSPATLSVSPGERATLSCRAS QSVNSNLAWYQQKPGQAPRLLIYGA STRATGIPARFSGSGSGTEFTLTISSL QSEDFAVYYCQQYNNWPLTFGQGT RLEIK | 67 |
| PR3001 86-7 | QVQLQESGPGLVKPSETLSLTCTVSGGSI QNYYWSWVRQPPGKGLEWIGYIFYSGS TNSNPSLKSRVIISVDTSKNQFSLKLSSVT AADTAVYYCAREVGRIAVAGWFFDLW GRGTLVTVSS | 64 | EIVMTQSPATLSVSPGERATLSCRAS QSVNANLAWYQQKPGQAPRLLIYG ASTRATGIPARFSGSGSGTEFTLTISS LQSEDFAVYYCQQYNNWPLTFGQG TRLEIK | 69 |
| PR3001 86-8 | QVQLQESGPGLVKPSETLSLTCTVSGGSI QNYYWSWVRQPPGKGLEWIGYIFYSGS TNANPSLKSRVIISVDTSKNQFSLKLSSV TAADTAVYYCAREVGRIAVAGWFFDL WGRGTLVTVSS | 66 | EIVMTQSPATLSVSPGERATLSCRAS QSVNANLAWYQQKPGQAPRLLIYG ASTRATGIPARFSGSGSGTEFTLTISS LQSEDFAVYYCQQYNNWPLTFGQG TRLEIK | 69 |
| PR3001 86-9 | QVQLQESGPGLVKPSETLSLTCTVSGGSI NNYYWSWVRQPPGKGLEWIGYIFYSGS TNSNPSLKSRVIISVDTSKNQFSLKLSSVT AADTAVYYCAREVGRIAVAGWFFDLW GRGTLVTVSS | 63 | EIVMTQSPATLSVSPGERATLSCRAS QSVNANLAWYQQKPGQAPRLLIYG ASTRATGIPARFSGSGSGTEFTLTISS LQSEDFAVYYCQQYNNWPLTFGQG TRLEIK | 69 |
| PR3001 86-10 | QVQLQESGPGLVKPSETLSLTCTVSGGSI QNYYWSWVRQPPGKGLEWIGYIFYSGS TQSNPSLKSRVIISVDTSKNQFSLKLSSVT AADTAVYYCAREVGRIAVAGWFFDLW GRGTLVTVSS | 65 | EIVMTQSPATLSVSPGERATLSCRAS QSVNANLAWYQQKPGQAPRLLIYG ASTRATGIPARFSGSGSGTEFTLTISS LQSEDFAVYYCQQYNNWPLTFGQG TRLEIK | 69 |

**Table 5. Amino Acid Sequences of HCDR1 to HCDR3 of Antibodies (According to Combined Encoding Rules)**

| Ab | SEQ ID NO: | HFWR1 | SEQ ID NO: | HCDR1 | SEQ ID NO: | HFWR2 | SEQ ID NO: | HCDR2 | SEQ ID NO: | HFWR3 | SEQ ID NO: | HCDR3 | SEQ ID NO: | HFWR4 |
|---|---|---|---|---|---|---|---|---|---|---|---|---|---|---|
| PR3001 59 | 1 | QVQLVESGGGVVQ PGRSLRLSCAAS | 2 | GFTFSS DGMN | 3 | WVRQAPGK GLEWVA | 4 | VIWNDGSNKY YADSVKG | 5 | RFTISRDNSKNTLYLQM NSLRAEDTAVYYCAR | 5 | DRGPLWP LDY | 7 | WGQGT LVTVSS |
| PR3001 59-1 | 1 | QVQLVESGGGVVQ PGRSLRLSCAAS | 15 | GFTFSS DAMN | 3 | WVRQAPGK GLEWVA | 16 | VIWNEGSNKY YADSVKG | 5 | RFTISRDNSKNTLYLQM NSLRAEDTAVYYCAR | 5 | DRGPLWP LDY | 7 | WGQGT LVTVSS |
| PR3001 59-3 | 1 | QVQLVESGGGVVQ PGRSLRLSCAAS | 15 | GFTFSS DAMN | 3 | WVRQAPGK GLEWVA | 17 | VIWNDASNKY YADSVKG | 5 | RFTISRDNSKNTLYLQM NSLRAEDTAVYYCAR | 5 | DRGPLWP LDY | 7 | WGQGT LVTVSS |
| PR3001 59-4 | 1 | QVQLVESGGGVVQ PGRSLRLSCAAS | 2 | GFTFSS DGMN | 3 | WVRQAPGK GLEWVA | 4 | VIWNDGSNKY YADSVKG | 5 | RFTISRDNSKNTLYLQM NSLRAEDTAVYYCAR | 5 | DRGPLWP LDY | 7 | WGQGT LVTVSS |
| PR3001 59-5 | 1 | QVQLVESGGGVVQ PGRSLRLSCAAS | 2 | GFTFSS DGMN | 3 | WVRQAPGK GLEWVA | 4 | VIWNDGSNKY YADSVKG | 5 | RFTISRDNSKNTLYLQM NSLRAEDTAVYYCAR | 5 | DRGPLWP LDY | 7 | WGQGT LVTVSS |
| PR3001 59-6 | 1 | QVQLVESGGGVVQ PGRSLRLSCAAS | 2 | GFTFSS DGMN | 3 | WVRQAPGK GLEWVA | 4 | VIWNDGSNKY YADSVKG | 5 | RFTISRDNSKNTLYLQM NSLRAEDTAVYYCAR | 5 | DRGPLWP LDY | 7 | WGQGT LVTVSS |
| PR3001 59-7 | 1 | QVQLVESGGGVVQ PGRSLRLSCAAS | 2 | GFTFSS DGMN | 3 | WVRQAPGK GLEWVA | 4 | VIWNDGSNKY YADSVKG | 5 | RFTISRDNSKNTLYLQM NSLRAEDTAVYYCAR | 5 | DRGPLWP LDY | 7 | WGQGT LVTVSS |
| PR3001 59-8 | 1 | QVQLVESGGGVVQ PGRSLRLSCAAS | 2 | GFTFSS DGMN | 3 | WVRQAPGK GLEWVA | 4 | VIWNDGSNKY YADSVKG | 5 | RFTISRDNSKNTLYLQM NSLRAEDTAVYYCAR | 5 | DRGPLWP LDY | 7 | WGQGT LVTVSS |
| PR3001 59-9 | 1 | QVQLVESGGGVVQ PGRSLRLSCAAS | 15 | GFTFSS DAMN | 3 | WVRQAPGK GLEWVA | 16 | VIWNEGSNKY YADSVKG | 5 | RFTISRDNSKNTLYLQM NSLRAEDTAVYYCAR | 5 | DRGPLWP LDY | 7 | WGQGT LVTVSS |
| PR3001 86 | 44 | QVQLQESGPGLVKP SETLSLTCTVS | 45 | GGSINN YYWS | 46 | WVRQPPGK GLEWIG | 47 | YIFYSGSTNSN PSLKS | 48 | RVIISVDTSKNQFSLKLS SVTAADTAVYYCAR | 49 | EVGRIAV AGWFFD L | 50 | WGRGT LVTVSS |
| PR3001 86-2 | 44 | QVQLQESGPGLVKP SETLSLTCTVS | 58 | GGSIQN YYWS | 46 | WVRQPPGK GLEWIG | 47 | YIFYSGSTNSN PSLKS | 48 | RVIISVDTSKNQFSLKLS SVTAADTAVYYCAR | 49 | EVGRIAV AGWFFD L | 50 | WGRGT LVTVSS |
| PR3001 36-3 | 44 | QVQLQESGPGLVKP SETLSLTCTVS | 58 | GGSIQN YYWS | 46 | WVRQPPGK GLEWIG | 59 | YIFYSGSTQSN PSLKS | 48 | RVIISVDTSKNQFSLKLS SVTAADTAVYYCAR | 49 | EVGRIAV AGWFFD L | 50 | WGRGT LVTVSS |
| PR3001 86-4 | 44 | QVQLQESGPGLVKP SETLSLTCTVS | 58 | GGSIQN YYWS | 46 | WVRQPPGK GLEWIG | 59 | YIFYSGSTQSN PSLKS | 48 | RVIISVDTSKNQFSLKLS SVTAADTAVYYCAR | 49 | EVGRIAV AGWFFD L | 50 | WGRGT LVTVSS |
| PR3001 86-5 | 44 | QVQLQESGPGLVKP SETLSLTCTVS | 45 | GGSINN YYWS | 46 | WVRQPPGK GLEWIG | 47 | YIFYSGSTNSN PSLKS | 48 | RVIISVDTSKNQFSLKLS SVTAADTAVYYCAR | 49 | EVGRIAV AGWFFD L | 50 | WGRGT LVTVSS |
| PR3001 86-6 | 44 | QVQLQESGPGLVKP SETLSLTCTVS | 58 | GGSIQN YYWS | 46 | WVRQPPGK GLEWIG | 60 | YIFYSGSTNAN PSLKS | 48 | RVIISVDTSKNQFSLKLS SVTAADTAVYYCAR | 49 | EVGRIAV AGWFFD L | 50 | WGRGT LVTVSS |
| PR3001 86-7 | 44 | QVQLQESGPGLVKP SETLSLTCTVS | 58 | GGSIQN YYWS | 46 | WVRQPPGK GLEWIG | 47 | YIFYSGSTNSN PSLKS | 48 | RVIISVDTSKNQFSLKLS SVTAADTAVYYCAR | 49 | EVGRIAV AGWFFD L | 50 | WGRGT LVTVSS |
| PR3001 86-8 | 44 | QVQLQESGPGLVKP SETLSLTCTVS | 58 | GGSIQN YYWS | 46 | WVRQPPGK GLEWIG | 60 | YIFYSGSTNAN PSLKS | 48 | RVIISVDTSKNQFSLKLS SVTAADTAVYYCAR | 49 | EVGRIAV AGWFFD L | 50 | WGRGT LVTVSS |
| PR3001 86-9 | 44 | QVQLQESGPGLVKP SETLSLTCTVS | 45 | GGSINN YYWS | 46 | WVRQPPGK GLEWIG | 47 | YIFYSGSTNSN PSLKS | 48 | RVIISVDTSKNQFSLKLS SVTAADTAVYYCAR | 49 | EVGRIAV AGWFFD L | 50 | WGRGT LVTVSS |
| PR3001 86-10 | 44 | QVQLQESGPGLVKP SETLSLTCTVS | 58 | GGSIQN YYWS | 46 | WVRQPPGK GLEWIG | 59 | YIFYSGSTQSN PSLKS | 48 | RVIISVDTSKNQFSLKLS SVTAADTAVYYCAR | 49 | EVGRIAV AGWFFD L | 50 | WGRGT LVTVSS |

**Table 6. Amino Acid Sequences of LCDR1 to LCDR3 of Antibodies (According to Combined Encoding Rules)**

| Ab | SEQ ID NO: | LFWR1 | SEQ ID NO: | LCDR1 | SEQ ID NO: | LFWR2 | SEQ ID NO: | LCDR2 | EQ ID NO: | LFWR3 | EQ ID NO: | LCDR3 | EQ ID NO: | LFWR4 |
|---|---|---|---|---|---|---|---|---|---|---|---|---|---|---|
| PR300159 | 8 | DIQMTQSPSTLS ASVGDRVTITC | 9 | RASQSISR WLA | 10 | WYQQKPGK APKLLIY | 11 | KASSL ES | 12 | GVPSRFSGSGSGTEFTL TISNLQPDDFATFYC | 13 | QQYNSYS RT | 14 | FGQGT KVEIK |
| PR300159-1 | 8 | DIQMTQSPSTLS ASVGDRVTITC | 9 | RASQSISR WLA | 10 | WYQQKPGK APKLLIY | 11 | KASSL ES | 12 | GVPSRFSGSGSGTEFTL TISNLQPDDFATFYC | 18 | QQYSSYSR T 14 | 14 | FGQGT KVEIK |
| PR300159-3 | 8 | DIQMTQSPSTLS ASVGDRVTITC | 9 | RASQSISR WLA | 10 | WYQQKPGK APKLLIY | 11 | KASSL ES | 12 | GVPSRFSGSGSGTEFTL TISNLQPDDFATFYC | 13 | QQYNSYS RT | 14 | FGQGT KVEIK |
| PR300159-4 | 8 | DIQMTQSPSTLS ASVGDRVTITC | 9 | RASQSISR WLA | 10 | WYQQKPGK APKLLIY | 11 | KASSL ES | 12 | GVPSRFSGSGSGTEFTL TISNLQPDDFATFYC | 19 | QQYNAYS RT | 14 | FGQGT KVEIK |
| PR 300159-5 | 8 | DIQMTQSPSTLS ASVGDRVTITC | 9 | RASQSISR WLA | 10 | WYQQKPGK APKLLIY | 11 | KASSL ES | 12 | GVPSRFSGSGSGTEFTL TISNLQPDDFATFYC | 20 | QQYQSYS RT | 14 | FGQGT KVEIK |
| PR300159-6 | 8 | DIQMTQSPSTLS ASVGDRVTITC | 9 | RASQSISR WLA | 10 | WYQQKPGK APKLLIY | 11 | KASSL ES | 12 | GVPSRFSGSGSGTEFTL TISNLQPDDFATFYC | 21 | QQYNTYS RT | 14 | FGQGT KVEIK |
| PR300159-7 | 8 | DIQMTQSPSTLS ASVGDRVTITC | 9 | RASQSISR WLA | 10 | WYQQKPGK APKLLIY | 11 | KASSL ES | 12 | GVPSRFSGSGSGTEFTL TISNLQPDDFATFYC | 22 | QQYTSYSR T | 14 | FGQGT KVEIK |
| PR300159-8 | 8 | DIQMTQSPSTLS ASVGDRVTITC | 9 | RASQSISR WLA | 10 | WYQQKPGK APKLLIY | 11 | KASSL ES | 12 | GVPSRFSGSGSGTEFTL TISNLQPDDFATFYC | 23 | QQYASYS RT | 14 | FGQGT KVEIK |
| PR300159-9 | 8 | DIQMTQSPSTLS ASVGDRVTITC | 9 | RASQSISR WLA | 10 | WYQQKPGK APKLLIY | 11 | KASSL ES | 12 | GVPSRFSGSGSGTEFTL TISNLQPDDFATFYC | 19 | QQYNAYS RT | 14 | FGQGT KVEIK |
| PR300186 | 51 | EIVMTQSPATLS VSPGERATLSC | 52 | RASQSVN SNLA | 53 | WYQQKPGQ APRLLIY | 54 | GASTR AT | 55 | GIPARFSGSGSGTEFTLT ISSLQSEDFAVYYC | 56 | QQYNNWP LT | 57 | FGQGT RLEIK |
| PR300186-2 | 51 | EIVMTQSPATLS VSPGERATLSC | 52 | RASQSVN SNLA | 53 | WYQQKPGQ APRLLIY | 54 | GASTR AT | 55 | GIPARFSGSGSGTEFTLT ISSLQSEDFAVYYC | 56 | QQYNNWP LT | 57 | FGQGT RLEIK |
| PR300186-3 | 51 | EIVMTQSPATLS VSPGERATLSC | 52 | RASQSVN SNLA | 53 | WYQQKPGQ APRLLIY | 54 | GASTR AT | 55 | GIPARFSGSGSGTEFTLT ISSLQSEDFAVYYC | 56 | QQYNNWP LT | 57 | FGQGT RLEIK |
| PR300186-4 | 51 | EIVMTQSPATLS VSPGERATLSC | 61 | RASQSVQ SNLA | 53 | WYQQKPGQ APRLLIY | 54 | GASTR AT | 55 | GIPARFSGSGSGTEFTLT ISSLQSEDFAVYYC | 56 | QQYNNWP LT | 57 | FGQGT RLEIK |
| PR300186-5 | 51 | EIVMTQSPATLS VSPGERATLSC | 61 | RASQSVQ SNLA | 53 | WYQQKPGQ APRLLIY | 54 | GASTR AT | 55 | GIPARFSGSGSGTEFTLT ISSLQSEDFAVYYC | 56 | QQYNNWP LT | 57 | FGQGT RLEIK |
| PR300186-6 | 51 | EIVMTQSPATLS VSPGERATLSC | 52 | RASQSVN SNLA | 53 | WYQQKPGQ APRLLIY | 54 | GASTR AT | 55 | GIPARFSGSGSGTEFTLT ISSLQSEDFAVYYC | 56 | QQYNNWP LT | 57 | FGQGT RLEIK |
| PR300186-7 | 51 | EIVMTQSPATLS VSPGERATLSC | 62 | RASQSVN ANLA | 53 | WYQQKPGQ APRLLIY | 54 | GASTR AT | 55 | GIPARFSGSGSGTEFTLT ISSLQSEDFAVYYC | 56 | QQYNNWP LT | 57 | FGQGT RLEIK |
| PR300186-8 | 51 | EIVMTQSPATLS VSPGERATLSC | 62 | RASQSVN ANLA | 53 | WYQQKPGQ APRLLIY | 54 | GASTR AT | 55 | GIPARFSGSGSGTEFTLT ISSLQSEDFAVYYC | 56 | QQYNNWP LT | 57 | FGQGT RLEIK |
| PR300186-9 | 51 | EIVMTQSPATLS VSPGERATLSC | 62 | RASQSVN ANLA | 53 | WYQQKPGQ APRLLIY | 54 | GASTR AT | 55 | GIPARFSGSGSGTEFTLT ISSLQSEDFAVYYC | 56 | QQYNNWP LT | 57 | FGQGT RLEIK |
| PR300186-10 | 51 | EIVMTQSPATLS VSPGERATLSC | 62 | RASQSVN ANLA | 53 | WYQQKPGQ APRLLIY | 54 | GASTR AT | 55 | GIPARFSGSGSGTEFTLT ISSLQSEDFAVYYC | 56 | QQYNNWP LT | 57 | FGQGT RLEIK |

### Example 2. Binding Activity of Antibodies to Human and Cynomolgus Monkey MSLN Proteins

Human MSLN (Acro Biosystems, Cat #: MSN-H5223) or cynomolgus monkey MSLN protein (Harbour BioMed, Batch #: 2019072202) was diluted in PBS to a concentration of 1 µg/mL. 100 µL of the diluted human or cynomolgus monkey MSLN was added to each well of an ELISA microplate and incubated overnight at 4°C. The plate was blocked with an ELISA blocking solution (containing 2% w/v BSA, 0.05% (v/v) Tween-20, and a PBS buffer of pH 7.4) at 37°C for 1 hour. The plate was then washed and incubated with diluted anti-MSLN antibodies (PR300186, PR300186-2, PR300186-3, PR300186-4, PR300186-5, PR300186-6, PR300186-7, PR300186-8, PR300186-9, and PR300186-10) (at 15 µg/mL) (100 nM, 10-fold dilutions, 8 points) at 37°C for 1 hour. The plate was then washed and incubated with HRP-conjugated goat anti-human IgG (H+L) antibody (Jackson, Cat: 109-035-088) at 37°C for 1 hour. Then, 100 µL of 3,3',5,5'-tetramethylbenzidine (TMB) substrate (Biopanda, Cat: TMB-S-003) was added, and the plate was incubated at room temperature for 15 minutes. The reaction was stopped by adding 100 µL of an ELISA stop solution (Solarbio, Cat#: C1058), and the optical density at 450 nm (OD450nm) was measured using an ELISA plate reader (Molecular Devices, SpectraMax 384 Plus).

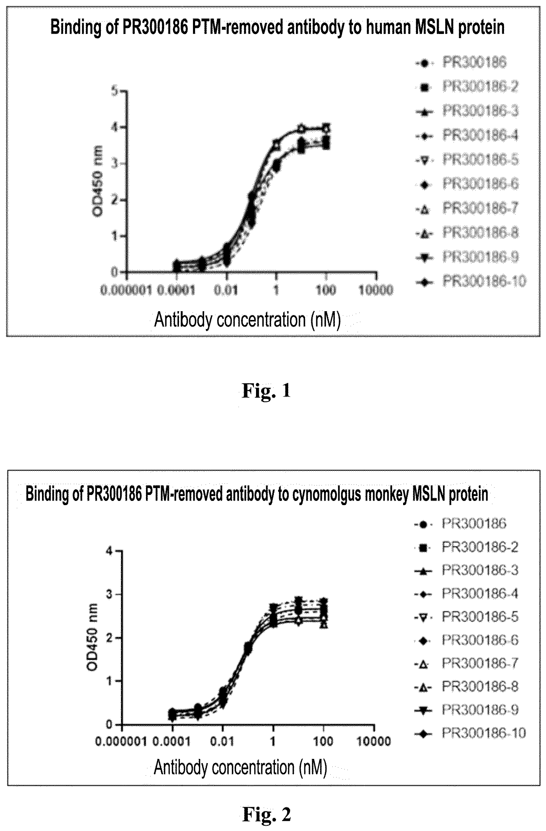

The binding activity results of PR300186, PR300186-2, PR300186-3, PR300186-4, PR300186-5, PR300186-6, PR300186-7, PR300186-8, PR300186-9, and PR300186-10 to human MSLN protein are shown in Figure 1 and Table 7, and the results of the binding activity to cynomolgus monkey MSLN protein are shown in Figure 2 and Table 7. The results indicate that all these antibodies exhibit good binding activity to both human and cynomolgus monkey MSLN proteins.

**Table 7. Binding Activity of Antibodies of the Present Invention to Human and Cynomolgus Monkey MSLN Proteins**

| Antibody | Human MSLN | | Cynomolgus Monkey MSLN | |
|---|---|---|---|---|
| | EC50 (nM) | Span | EC50 (nM) | Span |
| PR300186 | 0.12 | 3.42 | 0.05 | 2.33 |
| PR300186-2 | 0.14 | 3.36 | 0.05 | 2.43 |
| PR300186-3 | 0.10 | 3.25 | 0.05 | 2.36 |
| PR300186-4 | 0.18 | 3.47 | 0.07 | 2.53 |
| PR300186-5 | 0.22 | 3.61 | 0.07 | 2.70 |
| PR300186-6 | 0.21 | 3.66 | 0.07 | 2.67 |
| PR300186-7 | 0.14 | 3.85 | 0.04 | 2.24 |
| PR300186-8 | 0.13 | 3.87 | 0.04 | 2.18 |
| PR300186-9 | 0.11 | 3.85 | 0.05 | 2.19 |
| PR300186-10 | 0.10 | 3.71 | 0.04 | 2.19 |

### Example 3. Binding Activity of MSLN Antibodies to MSLN-Expressing Cells

The binding of recombinant anti-MSLN antibodies (PR300159, PR300159-1, PR300159-3, PR300159-5, PR300159-6, PR300159-8, PR300159-9, PR300186, PR300186-2, PR300186-3, PR300186-4, PR300186-5, PR300186-6, PR300186-7, PR300186-8, PR300186-9, or PR300186-10) to the COV644 cell line (ECACC, Cat #: 07071908) was detected by means of flow cytometry. Briefly, anti-MSLN antibodies were serially diluted in a staining buffer (PBS containing 2% FBS). 50 µL of the diluted antibody solution was added to 50 µL of a cell suspension containing 1-2 × 10⁵ cells and incubated at 4°C for 1 hour. The cells were washed twice with the staining buffer (PBS containing 2% FBS), and 100 µL of a 1:1000-diluted, fluorescently labeled anti-human IgG antibody (Alexa Fluor^{®} 488 AffiniPure Goat Anti-Human IgG (H+L), Jackson ImmunoResearch, Cat #: 109-545-088) was added to each well. After incubating at 4°C for 1 hour, the cells were washed twice with the staining buffer and subjected to flow cytometry.

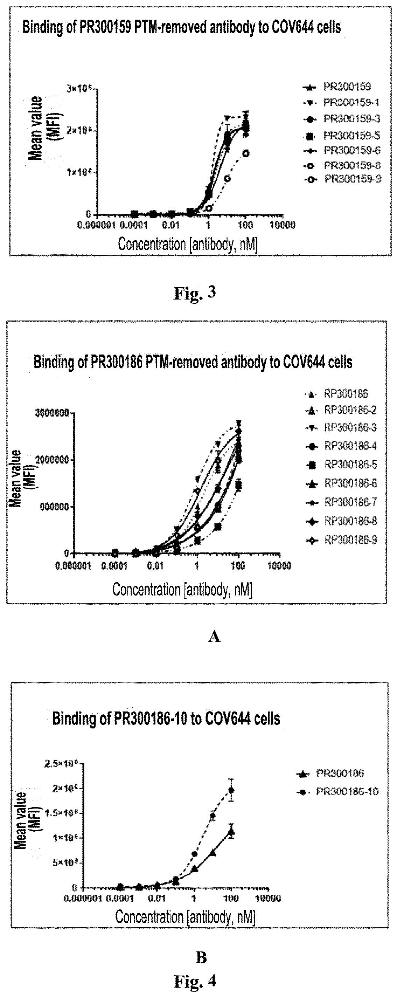

As shown in Figure 3 and Table 8, PR300159, PR300159-1, PR300159-3, PR300159-5, PR300159-6, PR300159-8, and PR300159-9 all exhibited high affinity for COV644 cells.

**Table 8. Binding Activity of Antibodies to Cell Surface MSLN as Detected by means of FACS**

| Antibody | COV644 | |
|---|---|---|
| | EC50 (nM) | TOP (MFI) |
| PR300159 | 1.99 | 2072495 |
| PR300159-1 | 1.64 | 2340478 |
| PR300159-3 | 2.14 | 2062034 |
| PR300159-5 | 2.75 | 2127765 |
| PR300159-6 | 4.07 | 2189544 |
| PR300159-8 | 2.33 | 2153079 |
| PR300159-9 | 8.25 | 1561284 |

As shown in Figure 4, PR300186, PR300186-2, PR300186-3, PR300186-4, PR300186-5, PR300186-6, PR300186-7, PR300186-8, PR300186-9, and PR300186-10 all demonstrated high affinity for COV644 cells.

### Example 4. Determination of\ Antibody Internalization by MSLN-Expressing Cells

In this example, a pHAb Amine Reactive Dye (Promega, Cat # G9841) is used to determine the internalization efficiency of anti-MSLN antibodies in various tumor cells. The pHAb dye is a pH sensor dye that exhibits very low fluorescence at pH values greater than 7 and significantly increased fluorescence as the pH value decreases. When an antibody labeled with a pHAb dye binds to an extracellular membrane under neutral pH conditions, little to no fluorescence is detected. After the antibody labeled with the pHAb dye is internalized into cells, strong fluorescence is detected in the low-pH environments of the endosomes and lysosomes of the cells.

Antibodies are labeled with pHAb dye, and a DAR value is calculated according to reagent kit instructions. The antibodies labeled with the pHAb dye are incubated with COV644 cells at 4°C (a temperature where antibody internalization activity is low, same being used as background controls) or at 37°C for 24 hours. Fluorescence is then measured at the excitation light value (Ex) of 532 nm and the emission light value (Em) of 560 nm. The way in which the internalization results of the antibodies are calculated is: the fluorescence intensity at 37°C minus the background fluorescence intensity at 4°C, divided by the DAR value of the antibody-conjugated dye. Higher fluorescence values indicate higher internalization efficiency of the antibody in the cells.

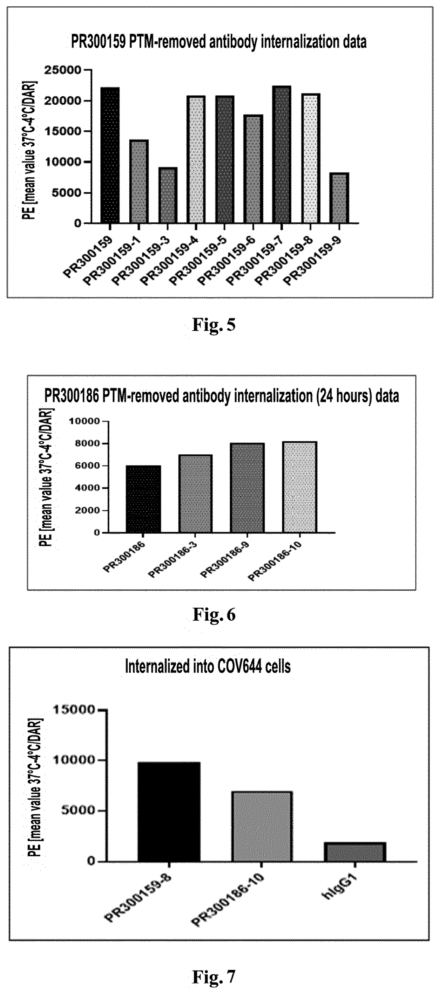

As shown by the results in Figures 5 to 6 and Table 9, PR300159, PR300159-1, PR300159-3, PR300159-4, PR300159-5, PR300159-6, PR300159-7, PR300159-8, PR300159-9, and PR300186, PR300186-3, PR300186-9, and PR300186-10 all demonstrated good internalization activity.

**Table 9. Results of Antibody Internalization by MSLN-Expressing Cells**

| Antibody | Internalization |
|---|---|
| | FLU |
| PR300159 | 22214 |
| PR300159-1 | 13720 |
| PR300159-3 | 9180 |
| PR300159-4 | 20888 |
| PR300159-5 | 20912 |
| PR300159-6 | 17753 |
| PR300159-7 | 22440 |
| PR300159-8 | 21172 |
| PR300159-9 | 8299 |
| PR300186 | 6049 |
| PR300186-3 | 7042 |
| PR300186-9 | 8069 |
| PR300186-10 | 8219 |

### Example 5. Binding Activity of PR300159-8 and PR300186-10 on Various Tumor Cells

The binding activity of antibodies PR300159-8 and PR300186-10 on the following tumor cells was evaluated using flow cytometry: breast cancer cell lines COV644 (ECACC, 07071908), OVCAR3 (ATCC, HTB-161), and SKOV3 (ATCC, HTB77); lung cancer cell lines NCI-H226 (ATCC, CRL5826) and HCl-H1568 (ATCC, CRL5876); pancreatic cancer cell lines HPAC (ATCC, CRL2199) and CAPAN2 (ATCC, HTB80); and gastric cancer cell line MKN45 (CCTCC, GDC0220).

PR300159-8 and PR300186-10 antibodies were serially diluted in a staining buffer (PBS with 2% FBS). 50 µL of the diluted antibody solution were added to a 50 µL cell suspension containing 1-2 × 10⁵ cells and incubated at 4°C for 1 hour. Cells were washed twice with the buffer (PBS with 2% fetal bovine serum), and 100 µL of fluorescently labeled anti-human IgG antibody (Alexa Fluor^{®} 488 AffiniPure Goat Anti-Human IgG (H+L), Jackson ImmunoResearch, Catalog 109-545-088) was added to each well. After incubation at 4°C for 1 hour, cells were washed twice with a buffer and analyzed using a flow cytometer.

The experiment results are shown in Table 10, and the experiment results indicate that the antibody PR300159-8 demonstrated strong binding activity across various tumor cell lines. The antibody PR300159-8 exhibited higher binding activity than PR300186-10.

**Table 10. Binding Activity of PR300159-8 and PR300186-10 on Various Tumor Cells**

| Cell Line | PR300159-8 | | PR300186-10 | |
|---|---|---|---|---|
| | EC50 (nM) | TOP (MFI) | EC50 (nM) | TOP (MFI) |
| NCIH1568 | 0.54 | 267401 | 135.60 | 168430 |
| NCIH226 | 0.20 | 835313 | 1.66 | 614535 |
| COV644 | 0.71 | 1884412 | 2.14 | 1101682 |
| OVCAR3 | 33.22 | 653501 | Weak binding | 75446 |
| SKOV3 | 0.59 | 97669 | Weak binding | 41733 |
| MKN45 | 0.1 | 173076 | Weak binding | 119565 |
| HPAC | 0.36 | 359012 | 12.45 | 239746 |
| CAPAN2 | 0.60 | 85162 | No binding | No binding |

### Example 6. Internalization Activity of PR300159-8 and PR300186-10 Antibodies on Various Tumor Cells

The internalization efficiency of antibodies PR300159-8 and PR300186-10 on the following various tumor cells was evaluated using flow cytometry: breast cancer cell line COV644 (ECACC, 07071908); lung cancer cell line NCI-H226 (ATCC, CRL5826); and pancreatic cancer cell line HPAC (ATCC, CRL2199).

In this example, a pHAb Amine Reactive Dye (Promega, Cat # G9841) was used to determine the internalization efficiency of anti-MSLN antibodies on different tumor cells. The pHAb dye is a pH sensor dye that exhibits very low fluorescence at pH values greater than 7, and its fluorescence significantly increases as the pH value of a solution decreases. When an antibody labeled with pHAb dye binds to the extracellular membrane under neutral pH conditions, little or no fluorescence is detected. After the antibody labelled with the pHAb dye is internalized into cells, strong fluorescence is detected in the low-pH environments of the endosomes and lysosomes of the cells.

Antibodies were labeled with pHAb dye, and DAR values were calculated according to reagent kit instructions. The antibodies labeled with the pHAb dye were incubated with COV644 cells at 4°C (a temperature where antibody internalization activity is low, same being used as background controls) or 37°C for 24 hours. Fluorescence was then measured at the excitation light value (Ex) of 532 nm and the emission light value (Em) of 560 nm. The way in which the internalization results of the antibodies were calculated is: fluorescence intensity at 37°C minus background fluorescence intensity at 4°C, divided by the DAR value of the antibody-conjugated dye. Higher fluorescence values indicate greater internalization efficiency of the antibody on the cells.

From the results shown in Figures 7, 8, and 9, it can be seen that PR300159-8 and PR300186-10 both exhibited good internalization activity on COV644, NCI-H226, and HPAC cells. The cell internalization activity of the antibody PR300159-8 was higher than that of PR300186-10.

### Example 7. Synthesis of Bioactive Molecules and Intermediates Used in the Process of Synthesizing "Drug-Linker Compounds"

### Example 7.1: Synthesis of (S)-7-ethyl-7-hydroxy-14-(3-hydroxypropyl)-10,13-dihydro-11H-[1,3]dioxolano[4,5-g]pyrano[3',4':6,7]indolizino[1,2-b]quinoline-8,11(7H)-dione (A1)

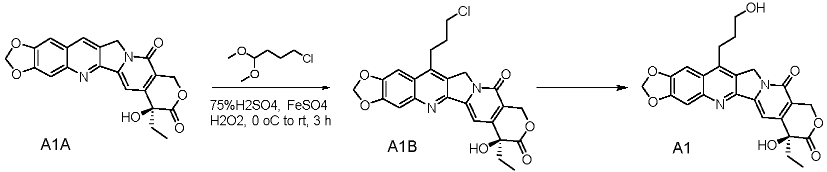

### Step 1: Synthesis of (S)-14-(3-chloropropyl)-7-ethyl-7-hydroxy-10,13-dihydro-11H-[1,3]dioxolano[4,5-g]pyrano[3',4':6,7]indolizino[1,2-b]quinoline-8,11(7H)-dione (A1B)

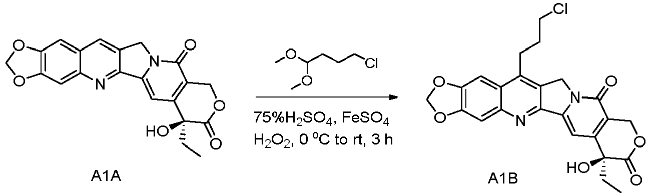

Under ice bath conditions, to the 75% sulfuric acid solution (5 mL) of compound (S)-7-ethyl-7-hydroxy-10,13-dihydro-11H-[1,3]dioxolano[4,5-g]pyrano[3',4':6,7]indolizino[1,2-b]quinoline-8,11(7H)-dione (A1A, 500 mg) were added ferrous sulfate heptahydrate (570 mg dissolved in 1 mL water) and 4,4-dimethoxychlorobutane (3.89 g). After the reaction mixture was stirred for 3 minutes, hydrogen peroxide (29%, 2.5 mL) was added dropwise. The reaction mixture was stirred and reacted at 0°C for 5 minutes and then warmed to room temperature, then stirred and reacted for 3 hours. The reaction mixture was diluted with water (50 mL) and extracted with ethyl acetate (80 mL × 2), the organic phase was washed with saturated brine, dried over anhydrous sodium sulfate, and concentrated to obtain a crude product, and the crude product was further purified using a C18 column (an aqueous solution of acetonitrile/0.05% formic acid: 5%-60%) to yield the target compound A1B (a yellow solid, 400 mg, yield: 67%).
LCMS (ESI) [M+H]⁺: 468.9;
¹H NMR (400 MHz, DMSO-d6) δ 7.65 (s, 1H), 7.51 (s, 1H), 7.24 (s, 1H), 6.50 (s, 1H), 6.30 (s, 2H), 5.42 (s, 2H), 5.26 (s, 2H), 3.81 (d, *J* = 5.9 Hz, 2H), 3.22 (s, 2H), 1.98 (d, *J* = 6.7 Hz, 4H), 0.88 (t, *J* = 7.2 Hz, 3H).

### Step 2: Synthesis of (S)-7-ethyl-7-hydroxy-14-(3-hydroxypropyl)-10,13-dihydro-11H-[1,3]dioxolano[4,5-g]pyrano[3',4':6,7]indolizino[1,2-b]quinoline-8,11(7H)-dione (A1)

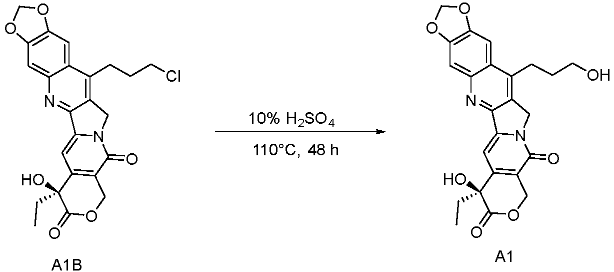

Compound (S)-14-(3-chloropropyl)-7-ethyl-7-hydroxy-10,13-dihydro-11H-[1,3]dioxolano[4,5-g]pyrano[3',4':6,7]indolizino[1,2-b]quinoline-8,11(7H)-dione (A1B, 100 mg, 0.213 mmol) was dissolved in a solution of 10% sulfuric acid (5 mL) and reacted at 110°C for 48 hours. A saturated sodium bicarbonate solution (mL) was added to the reaction mixture and extraction was performed using dichloromethane (10 mL × 5), followed by drying over anhydrous sodium sulfate, filtration, and concentration under reduced pressure to obtain a crude product. The crude product was purified using preparative high-performance liquid chromatography (acetonitrile/water containing 0.05% formic acid) to yield (S)-7-ethyl-7-hydroxy-14-(3-hydroxypropyl)-10,13-dihydro-11H-[1,3]dioxolano[4,5-g]pyrano[3',4':6,7]indolizino[1,2-b]quinoline-8,11(7H)-dione (A1, 1.78 mg).
LCMS (ESI) [M+H]⁺: 451.0;
¹H NMR (400 MHz, DMSO-*d6*) δ 7.63 (s, 1H), 7.50 (s, 1H), 7.24 (s, 1H), 6.48 (s, 1H), 6.28 (s, 2H), 5.47-5.37 (m, 2H), 5.32-5.19 (m, 2H), 3.51-3.46 (m, 2H), 3.17-3.13 (m, 2H), 1.92-1.76 (m, 4H), 0.90-0.84 (m, 3H).

### Example 7.2: Synthesis of (S)-4-ethyl-8-fluoro-4-hydroxy-11-(3-hydroxypropyl)-9-methyl-1,12-dihydro-14H-pyrano[3',4':6,7]indolizino[1,2-b]quinoline-3,14(4H)-dione (A2)

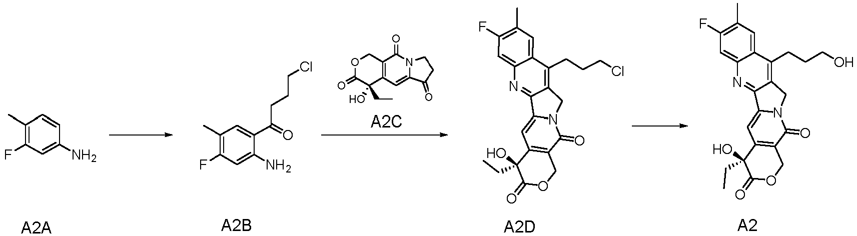

### Step 1:

At 0°C, to a solution of compound A2A (10 g) in 1,2-dichloroethane (200 mL) was added dropwise 1 mol/L boron trichloride (96 mL) and 4-chlorobutyronitrile (9.9 g). Same was stirred and reacted at 80°C for 2 hours. After the reaction mixture cooled to room temperature, 2 mol/L hydrochloric acid (90 mL) was added and refluxed and stirred at 80°C for 0.5 hours. The reaction mixture was cooled to room temperature, diluted with a small amount of water, and extracted with dichloromethane (200 mL × 3). The organic phase was dried over anhydrous sodium sulfate, filtered, and concentrated to obtain a crude product, and the crude product was separated and purified by means of column chromatography (petroleum ether: ethyl acetate = 10:1) to yield the target compound A2B (4 g).

LCMS (ESI) [M+H]⁺: 230.0.

### Step 2:

To the compound A2B (50 mg) was added (S)-4-ethyl-4-hydroxy-7,8-dihydro-1H-pyrano[3,4-f]indolo-3,6,10(4H)-trione (A2C, 35 mg), p-toluenesulfonic acid monohydrate (41.4 mg), and dichloromethane (30 mL). After clarifying and mixing, the solution was concentrated under reduced pressure and vacuumed using an oil pump. The reaction was carried out under vacuum at 120°C for 3 hours. LCMS showed the reaction was complete. After the reaction mixture was cooled to room temperature, water (20 mL) was added, and the mixture was extracted with dichloromethane (20 mL × 3). The combined organic phases were sequentially dried over anhydrous sodium sulfate, filtered, and concentrated under reduced pressure to obtain a crude product. The crude product was separated and purified by means of column chromatography (dichloromethane:methanol = 20:1) to yield the target compound A2D (80 mg) as a white solid.

LCMS (ESI) [M+H]⁺: 457.0.

### Step 3:

The compound A2D (75 mg) was dissolved in hexamethylphosphoramide, and pure water (0.8 mL) was added, and the reaction mixture was stirred at 100°C for 72 hours. LCMS indicated the completion of the reaction. The crude product was purified by preparative chromatography (0.01% TFA in water, MeCN) to yield the target compound A2 (10 mg).
LCMS (ESI) [M+H]⁺: 439.2;
¹H NMR (400 MHz, DMSO-d6) δ 8.22 (d, *J* = 8.4 Hz, 1H), 7.87 (d, *J* = 10.9 Hz, 1H), 7.31 (s, 1H), 6.50 (s, 1H), 5.43 (s, 2H), 5.30 (s, 2H), 4.67 (t, *J* = 4.9 Hz, 1H), 3.55-3.47 (m, 2H), 3.28-3.20 (m, 2H), 2.51 (s, 3H), 1.93-1.81 (m, 4H), 0.88 (t, *J =* 7.3 Hz, 3H).

### Example 7.3: Synthesis of (S)-2-amino-N-((3-(7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)propoxy)methyl)acetamide (B1)

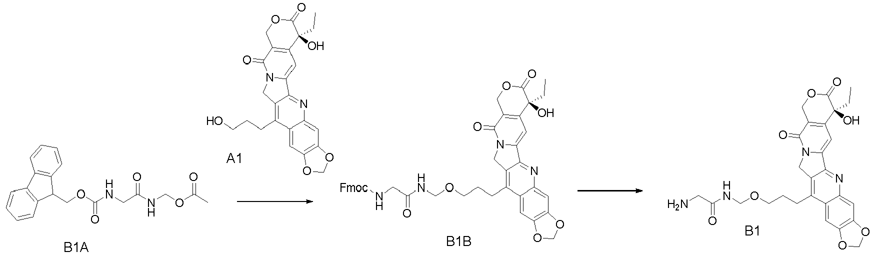

Step 1: The compound B1A (368 mg), compound A1 (440 mg), and pyridinium p-toluenesulfonate (PPTS, 25 mg) were refluxed in dichloromethane (20 mL) for 20 hours. The reaction mixture was then washed with an aqueous sodium bicarbonate solution and an aqueous hydrochloric acid solution, respectively. The organic solvent was removed under reduced pressure to yield a crude product, and the crude product was separated and purified by means of column chromatography (dichloromethane:methanol = 10:1) to yield the target compound B1B (240 mg).

LCMS (ESI) [M+H]⁺: 759.5.

Step 2: Compound B1B (240 mg) was dissolved in DMF (5 mL), and piperidine (1 mL) was added. The compound was stirred for 20 minutes, and dissolved at reduced pressure to remove low-boiling-point components. The residue was used directly in the next step for synthesis. A small amount of the crude product was purified by means of reverse-phase chromatography (acetonitrile/0.05% FA aqueous solution: from 5% to 50%) to yield the target compound B1.
ESI-MS (m/z): 537.4 [M+H]⁺;
¹H NMR (400 MHz, DMSO-d6) δ 9.13 (t, 1H), 8.04 (br, 2H), 7.58 (s, 1H), 7.51 (s, 1H), 7.25 (s, 1H), 6.29 (s, 2H), 5.43 (S, 2H), 5.21 (s, 2H), 4.65 (d, 2H), 3.63 (m, 2H), 3.53 (m, 2H), 3.11 (m, 2H), 1.87 (m, 4H), 0.88 (t, 3H).

### Example 7.4: Synthesis of (S)-2-amino-N-((3-(4-ethyl-8-fluoro-4-hydroxy-9-methyl-3,14-dioxo-3,4,12,14-tetrahydro-1H-pyrano[3',4':6,7]indolizino[1,2-b]quinolin-11-yl)propoxy)methyl)acetamide (B2)

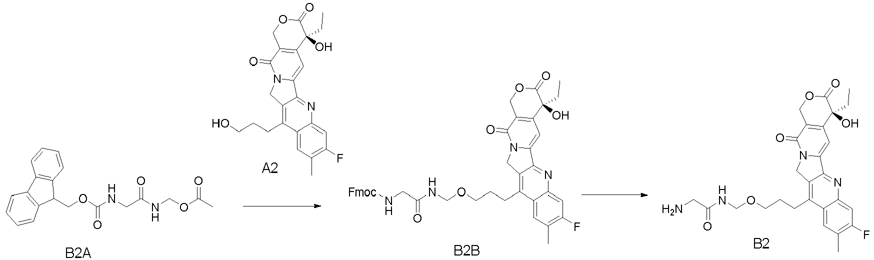

### Step 1:

The compound A2 (160 mg, 0.365 mmol) was dissolved in N,N-dimethylformamide (DMF, 3 mL), and (2-((((9H-fluoren-9-yl)methoxy)carbonyl)amino)acetamido)methyl acetate (B2A, 672 mg, 1.83 mmol) was added, and then HCl-ethyl acetate (0.073 mL, 3M) was added to the reaction mixture, which was stirred at room temperature overnight. Completion of the reaction was detected by LCMS. The reaction mixture was directly purified by means of reverse-phase chromatography (acetonitrile/0.05% FA aqueous solution: from 5% to 50%) to yield the target compound B2B as a white solid (80 mg, yield 29.0%).
LCMS (ESI) [M+H]⁺ = 747.4;
¹H NMR (400 MHz, DMSO-d6) δ 8.74 (t, *J =* 6.4 Hz, 1H), 8.28-8.17 (m, 1H), 7.99-7.88 (m, 3H), 7.73 (d, *J =* 7.3 Hz, 2H), 7.63 (t, *J =* 5.7 Hz, 1H), 7.44 (t, *J =* 7.4 Hz, 2H), 7.35 (t, *J* = 7.2 Hz, 3H), 6.58 (s, 1H), 5.48 (s, 2H), 5.29 (s, 2H), 4.66 (d, *J =* 6.3 Hz, 2H), 4.32 (d, *J* = 6.9 Hz, 2H), 4.26 (d, *J* = 6.1 Hz, 1H), 3.71 (d, *J* = 5.8 Hz, 2H), 3.57 (t, *J* = 5.7 Hz, 2H), 3.29-3.20 (m, 2H), 2.55 (s, 3H), 2.00-1.86 (m, 4H), 0.93 (t, *J =* 7.2 Hz, 3H).

Step 2: B2B (240 mg) was dissolved in DMF (5 mL), and piperidine (1 mL) was added. The compound was stirred for 20 minutes, and dissolved at reduced pressure to remove low-boiling-point components. The residue was directly used for the next step for synthesis.

ESI-MS (m/z): 525.2 [M+H]⁺.

A small amount of the crude product was purified by means of reverse-phase chromatography (acetonitrile/0.05% FA aqueous solution: from 5% to 50%) to yield the target compound B2.
ESI-MS (m/z): 525.1 [M+H]⁺;
¹H NMR (400 MHz, DMSO) δ 9.13 (t, *J* = 6.6 Hz, 1H), 8.21 (d, *J* = 8.1 Hz, 1H), 8.02 (brs, 2H), 7.89 (d, *J* = 10.8 Hz, 1H), 7.32 (s, 1H), 6.54 (s, 1H), 5.44 (s, 2H), 5.28 (s, 2H), 4.66 (d, *J =* 6.5 Hz, 2H), 3.64 (s, 2H), 3.53 (t, *J =* 6.1 Hz, 2H), 3.25-3.18 (m, 2H), 2.52 (s, 3H), 1.98-1.84 (m, 4H), 0.88 (t, *J =* 7.3 Hz, 3H).

### Example 7.5: Synthesis of N⁶,N⁶-Dimethyl-N²-((6-(2-(methanesulfonyl)pyrimidin-5-yl)hex-5-ynoyl)-L-valine)-L-lysine (C1)

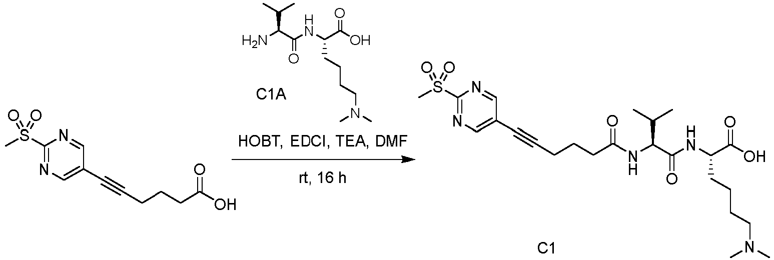

6-(2-(methanesulfonyl)pyrimidin-5-yl)hex-5-ynoic acid (268 mg), compound C1A (328 mg), and triethylamine (322 mg) were dissolved in N,N-dimethylformamide (5 mL). 1-hydroxybenzotriazole (HOBT, 162 mg) and 1-(3-dimethylaminopropyl)-3-ethylcarbodiimide hydrochloride (EDCI, 229 mg) were then added. The reaction mixture was stirred at room temperature for 16 hours. The reaction mixture was directly purified using a C18 reverse-phase column (acetonitrile and 0.05% formic acid aqueous solution system) to obtain the target compound N⁶,N⁶-dimethyl-N²-((6-(2-(methanesulfonyl)pyrimidin-5-yl)hex-5-ynoyl)-L-valine)-L-lysine (C1, a white solid, 327 mg).

LCMS (ESI) [M+H]⁺: 524.4.

¹H NMR (400 MHz, ) δ 9.13 (s, 2H), 7.95 (t, *J* = 8.8 Hz, 2H), 4.21 (dd, *J* = 8.8, 6.9 Hz, 1H), 4.08-4.03 (m, 1H), 3.41 (s, 3H), 2.55 (t, *J =* 7.0 Hz, 2H), 2.42-2.32 (m, 4H), 2.27 (s, 6H), 1.98 (dd, *J* = 13.6, 6.8 Hz, 1H), 1.86-1.77 (m, 2H), 1.74-1.55 (m, 2H), 1.47-1.37 (m, 2H), 1.31-1.23 (m, 2H), 0.85 (dd, *J =* 12.8, 6.8 Hz, 6H).

### Example 7.6: Synthesis of N⁶,N⁶-diethyl-N²-((6-(2-(methanesulfonyl)pyrimidin-5-yl)hex-5-ynoyl)-L-valine)-L-lysine (C2)

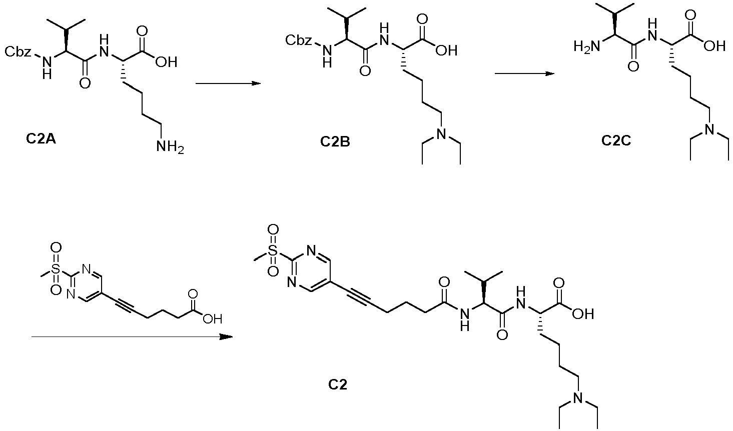

### Step 1:

The compound C2A (5.0 g, 12.05 mmol) was dissolved in dichloromethane (100 mL). Acetaldehyde (3.2 g, 72.3 mmol) was added to the reaction mixture and same was stirred and reacted at room temperature for 10 minutes. Then, sodium triacetoxyborohydride (12.8 g, 60.25 mmol) was added to the reaction mixture and same was stirred and reacted at room temperature for 1 hour. LCMS indicated the reaction was complete. A saturated ammonium chloride aqueous solution was added to the reaction mixture and same was stirred for 1 hour. Same was spin-dried and filtered, then the filtrate was separated and purified using a C18 reverse-phase column (acetonitrile and 0.05% formic acid aqueous solution: 5% to 55%) to obtain the target compound C2B (4.57 g, yield 82%) as a white solid.
LCMS (ESI) [M+H]⁺ = 436.4;
¹H NMR (400 MHz, DMSO-d6) δ 7.70 (d, *J* = 7.0 Hz, 1H), 7.41 (d, *J* = 9.0 Hz, 1H), 7.38-7.26 (m, 5H), 5.08-4.99 (m, 2H), 4.00 (dd, *J =* 12.6, 6.5 Hz, 1H), 3.86 (dd, *J =* 8.6, 6.8 Hz, 1H), 2.74 (dd, *J* = 14.0, 6.9 Hz, 4H), 2.64-2.54 (m, 2H), 2.05-1.94 (m, 1H), 1.72-1.52 (m, 2H), 1.52-1.38 (m, 2H), 1.38-1.18 (m, 2H), 1.04 (t, *J =* 7.1 Hz, 6H), 0.87-0.81 (m, 6H).

### Step 2:

The compound C2B (1.6 g, 3.68 mmol) was dissolved at room temperature in methanol (80 mL), Pd/C (0.16 g) was then added to the reaction mixture, and same was stirred under hydrogen gas at room temperature for 12 hours. LCMS indicated the reaction was complete. The reaction mixture was filtered, and the filtrate was concentrated under reduced pressure to obtain the target compound C2C (900 mg, yield 82%) as an off-white solid.

¹H NMR (400 MHz, DMSO-d6) δ 8.04 (br s, 1H), 4.02 - 3.99 (m, 1H), 3.10 (d, *J* = 4.5 Hz, 1H), 2.65 (q, *J* = 7.1 Hz, 4H), 2.55-2.51 (m, 2H), 2.06-1.93 (m, 1H), 1.73-1.54 (m, 2H), 1.47-1.38 (m, 2H), 1.30-1.21 (m, 2H), 1.01 (t, *J =* 7.1 Hz, 6H), 0.89 (d, *J =* 6.9 Hz, 3H), 0.79 (d, *J* = 6.8 Hz, 3H).

### Step 3:

The compound 6-(2-(methanesulfonyl)pyrimidin-5-yl)hex-5-ynoic acid (268 mg, 1 mmol) in DMF (8 mL), HATU (380 mg, 1 mmol) and triethylamine (322 mg, 2.5 mmol) were sequentially added, and same were stirred at room temperature for 20 minutes. The compound C2C (301 mg, 1 mmol) was then added, and same continued to be stirred at room temperature for 30 minutes. After confirming the completion of the reaction by means of LCMS, the reaction mixture was directly purified using a C18 reverse-phase column (acetonitrile and 0.05% formic acid aqueous solution system) to obtain the target compound C2 (280 mg, yield 51%) as a white solid.
LCMS (ESI) [M+H]⁺ = 552.3;
¹H NMR (400 MHz, DMSO-d6) δ 9.13 (s, 2H), 7.95 (d, *J* = 8.9 Hz, 1H), 7.86 (d, *J* = 7.2 Hz, 1H), 4.18 (dd, *J* = 8.8, 6.8 Hz, 1H), 4.02 (dd, *J =* 12.8, 7.2 Hz, 1H), 3.41 (s, 3H), 2.74-2.69 (m, 4H), 2.62-2.52 (m, 4H), 2.44-2.29 (m, 2H), 2.04-1.94 (m, 1H), 1.86-1.77 (m, 2H), 1.72-1.54 (m, 2H), 1.51-1.39 (m, 2H), 1.33-1.23 (m, 2H), 1.02 (t, *J* = 7.2 Hz, 6H), 0.87-0.82 (m, 6H).

### Example 7.7: Synthesis of N⁶,N⁶-dipropyl-N²-((6-(2-(methanesulfonyl)pyrimidin-5-yl)hex-5-ynoyl)-L-valine)-L-lysine (C3)

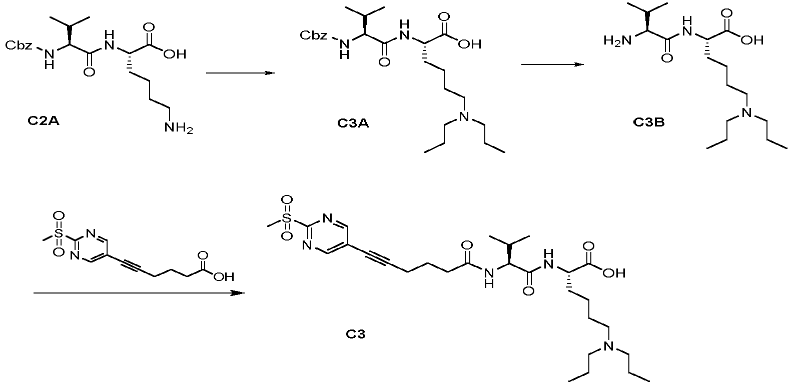

### Step 1:

The compound C2A (5.0 g, 12 mmol) was dissolved in dichloromethane (100 mL). n-propionaldehyde (4.2 g, 72.3 mmol) was added to the reaction mixture, and same were stirred and reacted at room temperature for 10 minutes. Sodium triacetoxyborohydride (12.8 g, 60.25 mmol) was then added to the reaction mixture, and same were stirred and reacted at room temperature for 1 hour. LCMS indicated the reaction was complete. A saturated ammonium chloride aqueous solution was added to the reaction mixture, and same were stirred for 1 hour. Same were spin-dried and filtered, and then and the filtrate was separated and purified using a C18 reverse-phase column (acetonitrile and 0.05% formic acid aqueous solution: 5% to 55%) to obtain the target compound C3A (4.57 g, yield 82.0%) as a white solid.
LCMS (ESI) [M+H]⁺ = 464.0;
¹H NMR (400 MHz, DMSO-d6) δ 7.81 (d, *J* = 7.3 Hz, 1H), 7.38-7.28 (m, 5H), 5.04 (d, *J* = 1.7 Hz, 2H), 4.12-4.02 (m, 1H), 3.94-3.82 (m, 1H), 2.65-2.52 (m, 6H), 2.06-1.94 (m, 1H), 1.76-1.64 (m, 1H), 1.64-1.53 (m, 1H), 1.52-1.40 (m, 6H), 1.34-1.18 (m, 2H), 0.92-0.80 (m, 12H).

### Step 2:

The compound C3A (2.0 g, 4.32 mmol) was dissolved in methanol (80 mL) at room temperature. Pd/C (0.16 g) was then added to the reaction mixture, and same were stirred and reacted under hydrogen gas at room temperature for 12 hours. LCMS indicated the reaction was complete. The reaction mixture was filtered and the filtrate was concentrated under reduced pressure to obtain the target compound C3B (1.2 g, yield 85.5%) as a white solid.

### Step 3:

The compound 6-(2-(methanesulfonyl)pyrimidin-5-yl)hex-5-ynoic acid (100 mg, 0.373 mmol) was dissolved in N,N-dimethylformamide (1 mL). HATU (142 mg, 0.373 mmol) and N,N-diisopropylethylamine (120 mg, 0.93 mmol) were then added, and the system was stirred for 30 minutes. The compound C3B (122 mg, 0.371 mmol) was then added, and the reaction mixture was stirred at room temperature for 1 hour. After LCMS detected the reaction was complete, the reaction mixture was directly purified using a C18 reverse-phase column (acetonitrile and 0.05% formic acid aqueous solution system) to obtain the target compound N⁶,N⁶-dipropyl-N²-((6-(2-(methanesulfonyl)pyrimidin-5-yl)hex-5-ynoyl)-L-valine)-L-lysine (C3, 50 mg, yield 28%) as a pale yellow solid.
LCMS (ESI) [M+H]⁺ = 580.0;
¹H NMR (400 MHz, DMSO-d6) δ 8.24 (s, 2H), 7.98-7.93 (m, 2H), 4.24-4.16 (m, 1H), 4.10 (d, *J =* 5.2 Hz, 1H), 3.41 (s, 3H), 2.79-2.64 (m, 6H), 2.55 (t, *J =* 7.1 Hz, 2H), 2.45-2.26 (m, 2H), 2.06-1.91 (m, 1H), 1.89-1.78 (m, 2H), 1.76-1.66 (m, 1H), 1.64-1.57 (m, 1H), 1.57-1.42 (m, 6H), 1.37-1.24 (m, 2H), 0.93-0.78 (m, 12H).

### Example 7.8: Synthesis of N⁶,N⁶-dibutyl-N²-((6-(2-(methanesulfonyl)pyrimidin-5-yl)hex-5-ynoyl)-L-valine)-L-lysine (C4)

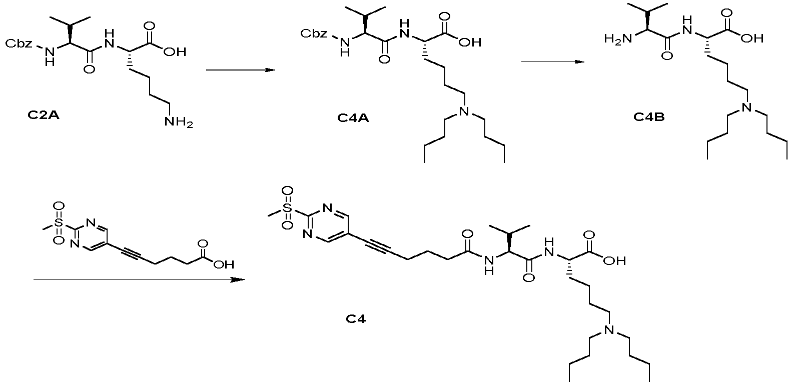

### Step 1:

The compound C2A (10.0 g) was dissolved in a dichloromethane solution (200 mL). n-butyraldehyde (10.4 g) was added to the reaction mixture, and same were stirred and reacted at room temperature for 10 minutes. Then, sodium triacetoxyborohydride (25.6 g) was added to the reaction mixture in batches, and same were stirred and reacted at room temperature for 1 hour. LCMS indicated the reaction was complete. A saturated ammonium chloride aqueous solution was added to the reaction mixture, and same were stirred for 1 hour. Same were spin-dried and filtered, and the filtrate was then separated and purified using a C18 reverse-phase column (acetonitrile and 0.05% formic acid aqueous solution, 5% to 55%) to obtain the target compound C4A (4.9 g) as a white solid.
LCMS (ESI) [M+H]⁺ = 492.7;
¹H NMR (400 MHz, CDCl3) δ 7.35-7.30 (m, 5H), 5.14-5.08 (m, 2H), 4.33-4.30 (m, 1H), 4.14-4.12 (m, 1H), 2.94-2.80 (m, 6H), 2.13-2.11 (m, 1H), 1.85-1.82 (m, 2H), 1.58-1.55 (m, 4H), 1.40-1.22 (m, 8H), 0.98-0.87 (m, 12H).

### Step 2:

The compound C4A (5.0 g) was dissolved in a methanol solution (100 mL) at room temperature. Pd/C (10%, 1 g) was then added to the reaction mixture and same were stirred and reacted under hydrogen gas for 12 hours. LCMS indicated the reaction was complete. The reaction mixture was filtered, and the filtrate was concentrated under reduced pressure to obtain the target compound C4B (3.68 g) as a white solid.

LCMS (ESI) [M+H]⁺ = 358.3.

### Step 3:

The compound 6-(2-(methanesulfonyl)pyrimidin-5-yl)hex-5-ynoic acid (1.0 g) was dissolved in N,N-dimethylformamide (15 mL). N,N-diisopropylethylamine (1.2 g) and HATU (1.4 g) were then sequentially added, and same were reacted and stirred for 30 minutes. Then, the compound C4B (1.3 g) was added and the reaction mixture was stirred at room temperature for 1 hour. After LCMS detected the reaction was complete, the reaction mixture was directly purified using preparative chromatography (a 0.01% trifluoroacetic acid aqueous solution and acetonitrile) to obtain the target compound N⁶,N⁶-dibutyl-N²-((6-(2-(methanesulfonyl)pyrimidin-5-yl)hex-5-ynoyl)-L-valine)-L-lysine (C4, 500 mg) as a yellow solid.
LCMS (ESI) [M+H]⁺ = 608.7;
¹H NMR (400 MHz, DMSO) δ 9.37 (s, 1H), 9.13 (s, 2H), 8.19 (d, *J* = 7.3 Hz, 1H), 7.92 (d, *J =* 8.8 Hz, 1H), 4.25-4.20 (m, 1H), 4.20-4.13 (m, 1H), 3.42 (s, 3H), 3.06-2.99 (m, 6H), 2.57-2.53 (m, 2H), 2.41-2.30 (m, 2H), 1.99-1.95 (m, 2H), 1.87-1.79 (m, 2H), 1.78-1.71 (m, 1H), 1.62-1.56 (m, 4H), 1.34-1.32 (m, 8H), 0.94-0.85 (m, 12H).

### Example 8. Drug-Linker Compound Synthesis

### Example 8.1: N-((11S,14S)-11-(4-(Di-n-propylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecane-14-yl)-6-(2-(methanesulfonyl)pyrimidin-5-yl)hex-5-ynoylamide (DL-01)

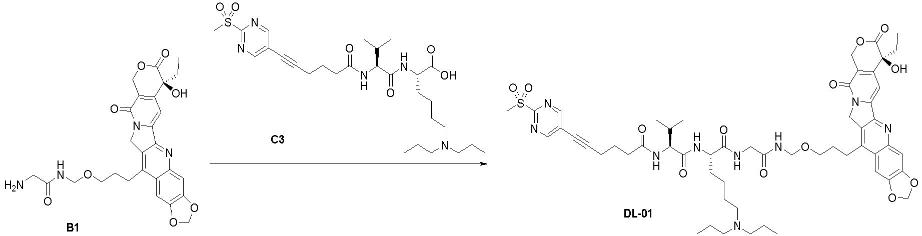

The compound C3 (43 mg, 0.074 mmol) and compound B1 (40 mg, 0.075 mmol) were dissolved in N,N-dimethylformamide (1 mL). HBTU (28 mg, 0.075 mmol) and N,N-diisopropylethylamine (24 mg, 0.187 mmol) were then sequentially added. The reaction mixture was stirred at room temperature for 1 hour. After LCMS detected reaction completion, the reaction mixture was directly purified using preparative chromatography (a 0.01% trifluoroacetic acid aqueous solution and acetonitrile) to obtain the target compound DL-01 (8.5 mg, 10% yield) as a yellow solid.
LCMS (ESI) [M+H]⁺ = 1098.6;
¹H NMR (400 MHz, DMSO-d6) δ 9.10 (s, 2H), 9.03 (s, 1H), 8.64 (t, *J* = 6.4 Hz, 1H), 8.19 (t, *J =* 5.9 Hz, 1H), 8.08 (d, *J =* 7.4 Hz, 1H), 7.92 (d, *J =* 8.5 Hz, 1H), 7.59 (s, 1H), 7.51 (s, 1H), 7.24 (s, 1H), 6.49 (s, 1H), 6.29 (s, 2H), 5.42 (s, 2H), 5.24 (s, 2H), 4.66-4.52 (m, 2H), 4.31-4.21 (m, 1H), 4.19-4.10 (m, 1H), 3.74 (d, *J =* 5.5 Hz, 2H), 3.49-3.48 (m, 2H), 3.40 (s, 3H), 3.15-3.06 (m, 2H), 3.02-2.95 (m, 6H), 2.59-2.52 (m, 3H), 2.41-2.29 (m, 2H), 2.05-1.90 (m, 2H), 1.91-1.77 (m, 6H), 1.63-1.57 (m, 6H), 1.31-1.29 (m, 2H), 0.92-0.80 (m, 15H).

### Example 8.2: N-((11S,14S)-11-(4-(Diethylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecane-14-yl)-6-(2-(methanesulfonyl)pyrimidin-5-yl)hex-5-ynoylamide (DL-02)

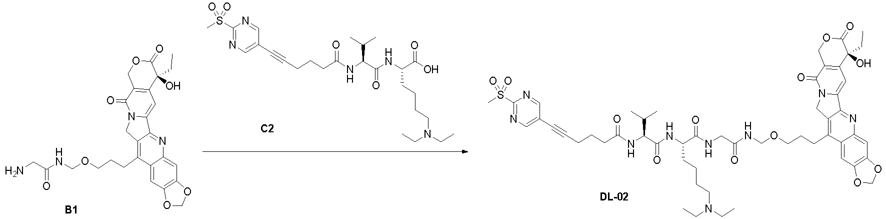

The compound C2 (40 mg, 0.075 mmol) and compound B1 (41 mg, 0.075 mmol) were dissolved in DMF (1 mL), and HOBt (15.2 mg, 0.113 mmol) and 1-(3-dimethylaminopropyl)-3-ethylcarbodiimide hydrochloride (21.5 mg, 0.113 mmol) were then sequentially added, and then triethylamine (23 mg, 0.225 mmol) was added. After this, the reaction mixture was stirred at room temperature for 16 hours. After LCMS detected completion of the reaction, the reaction mixture was concentrated to obtain a crude product, and the crude product was purified using preparative chromatography (0.01% TFA in water, MeCN) to yield the target compound N-((11S,14S)-11-(4-(diethylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecane-14-yl)-6-(2-(methanesulfonyl)pyrimidin-5-yl)hex-5-ynoylamide (16 mg, 20% yield) as a yellow solid.
LCMS (ESI) [M+H]⁺ = 1070.6;
¹H NMR (400 MHz, DMSO-d6) δ 9.13-9.08 (m, 2H), 9.01 (s, 1H), 8.64 (t, *J =* 6.5 Hz, 1H), 8.20 (t, *J* = 5.7 Hz, 1H), 8.09 (d, *J* = 7.4 Hz, 1H), 7.92 (d, *J* = 8.4 Hz, 1H), 7.59 (s, 1H), 7.51 (s, 1H), 7.24 (s, 1H), 6.50 (s, 1H), 6.29 (s, 2H), 5.43 (s, 2H), 5.24 (s, 2H), 4.65-4.53 (m, 2H), 4.26 (d, *J* = 6.5 Hz, 1H), 4.20-4.10 (m, 1H), 3.74 (d, *J* = 5.5 Hz, 2H), 3.50 (t, *J* = 5.8 Hz, 2H), 3.41 (s, 3H), 3.14-3.07 (m, 6H), 2.98 (s, 2H), 2.55 (d, *J =* 7.3 Hz, 2H), 2.41-2.29 (m, 2H), 2.03-1.89 (m, 2H), 1.89-1.77 (m, 7H), 1.61-1.56 (m, 2H), 1.32 (s, 2H), 1.16 (t, *J* = 7.2 Hz, 6H), 0.91-0.78 (m, 9H).

### Example 8.3: N-((11S,14S)-11-(4-(dimethylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecane-14-yl)-6-(2-(methanesulfonyl)pyrimidin-5-yl)hex-5-ynoylamide (DL-03)

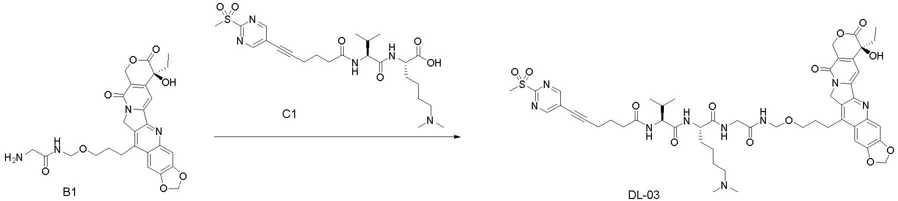

The compound B1 (100 mg, 0.186 mmol) and the compound N⁶,N⁶-dimethyl-N²-((6-(2-(methanesulfonyl)pyrimidin-5-yl)hex-5-ynoyl)-L-valine)-L-lysine (C1) (100 mg, 0.191 mmol) were dissolved in DMF (2 mL). 1-hydroxybenzotriazole (38 mg, 0.280 mmol) and 1-(3-dimethylaminopropyl)-3-ethylcarbodiimide hydrochloride (53 mg, 0.280 mmol) were then added, and then triethylamine (56 mg, 0.559 mmol) was added. After the addition was complete, the reaction mixture was stirred at room temperature for 16 hours. After LCMS detected completion of the reaction, the reaction mixture was purified by means of preparative chromatography (0.01% TFA in water, MeCN) to yield the target compound N-((11S,14S)-11-(4-(dimethylamino)butyl)-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-15-methyl-7,10,13-trioxo-4-oxa-6,9,12-triazahexadecane-14-yl)-6-(2-(methanesulfonyl)pyrimidin-5-yl)hex-5-ynoylamide (DL-03) (20 mg, 10% yield) as a yellow solid.
LCMS (ESI) [M+H]⁺ = 1042.5;
¹H NMR (400 MHz, DMSO-d6) δ 9.28 (s, 1H, TFA), 9.10 (s, 2H), 8.64 (br s, 1H), 8.19 (br s, 1H), 8.09 (d, *J* = 6.4 Hz, 1H), 7.92 (d, *J =* 7.8 Hz, 1H), 7.59 (s, 1H), 7.51 (s, 1H), 7.24 (s, 1H), 6.50 (s, 1H), 6.29 (s, 2H), 5.43 (s, 2H), 5.24 (s, 2H), 4.67-4.52 (m, 2H), 4.30-4.10 (m, 2H), 3.74 (br s, 2H), 3.50 (br s, 2H), 3.41 (s, 3H), 3.17-3.07 (m, 2H), 3.04-2.91 (m, 2H), 2.75 (s, 6H), 2.46-2.24 (m, 3H), 2.04-1.66 (m, 9H), 1.63-1.46 (m, 3H), 1.32-1.29 (m, 2H), 0.87-0.82 (m, 9H).

### Example 8.4: N-((11S,14S)-11-(4-(dibutylamino)butyl)-1-((S)-4-ethyl-8-fluoro-4-hydroxy-9-methyl-3,14-dioxo-3,4,12,14-tetrahydro-1H-pyrano[3',4':6,7]indolizino[1,2-b]quinolin-11-yl)-15-methyl-7,10,13-trioxo-4-oxa-6,9,12-triazahexadecane-14-yl)-6-(2-(methanesulfonyl)pyrimidin-5-yl)hex-5-ynoylamide (DL-04)

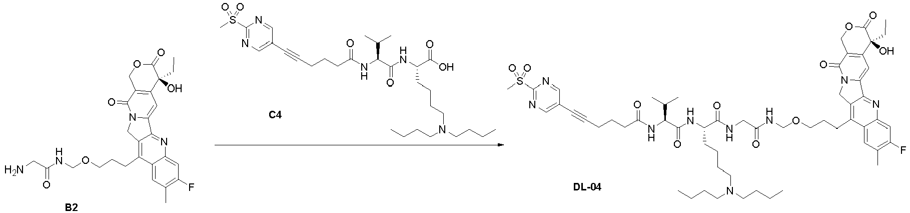

The compound C4 (50 mg, 0.08 mmol) and the compound B2 (43 mg, 0.08 mmol) were dissolved in N,N-dimethylformamide (1.5 mL). Then, DIPEA (26 mg, 0.21 mmol) and HBTU (31 mg, 0.08 mmol) were sequentially added. Same were stirred and reacted at room temperature for 1 hour. After reaction completion was detected by LCMS, the reaction mixture was directly separated and purified using high-performance preparative chromatography (an 0.01% trifluoroacetic acid aqueous solution, MeCN) to yield the target compound DL-04 (13.57 mg) as a yellow solid.
LCMS (ESI) [M+H]⁺ = 1114.6;
¹H NMR (400 MHz, DMSO) δ 9.09 (s, 2H), 9.05 (s, 1H, TFA), 8.64 (t, *J =* 7.2 Hz, 1H), 8.23-8.16 (m, 2H), 8.07 (d, *J =* 7.3 Hz, 1H), 7.93-7.85 (m, 2H), 7.32 (s, 1H), 6.52 (s, 1H), 5.44 (s, 2H), 5.29 (s, 2H), 4.61-4.59 (m, 2H), 4.24-4.22 (m, 1H), 4.19-4.10 (m, 1H), 3.73-3.70 (m, 2H), 3.50 (s, 3H), 3.21-3.18 (m, 6H), 3.00-2.98 (m, 8H), 2.54 (s, 3H), 1.97-1.78 (m, 7H), 1.56-1.52 (m, 8H), 1.36-1.25 (m, 6H), 0.93-0.78 (m, 15H).

### Example 8.5: N-((10S,13S)-10-(4-(N,N-dimethylamino)butyl)-1-(((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-14-methyl-1,6,9,12-tetraoxo-3-oxa-5,8,11-triaza-pentadecane-13-yl)-6-(2-(methanesulfonyl)pyrimidin-5-yl)hex-5-ynoylamide (DL-05)

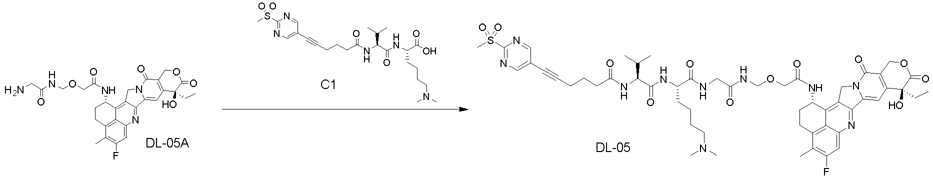

The compound N⁶,N⁶-dimethyl-N²-((6-(2-(methanesulfonyl)pyrimidin-5-yl)hex-5-ynoyl)-L-valine)-L-lysine (C1, 52.3 mg) was dissolved in N,N-dimethylformamide (2 mL), and HBTU (38 mg, 0.10 mmol) and N,N-diisopropylethylamine (26 mg, 0.20 mmol) were then sequentially added. After addition was completed, the reaction mixture was stirred at room temperature for 30 minutes, and the compound 2-amino-N-((2-(((1S, 9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-2-oxoethoxy)methyl)acetamide (DL-05A, 63 mg, which was prepared by following the method described in CN104755494A, pages 147-148) was added. After LCMS detected reaction completion, the reaction mixture was directly purified through preparative chromatography (a 0.01% trifluoroacetic acid aqueous solution and acetonitrile) to obtain the target compound N-((10S,13S)-10-(4-(N,N-dimethylamino)butyl)-1-(((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-14-methyl-1,6,9,12-tetraoxo-3-oxa-5,8,11-triazapentadecane-13-yl)-6-(2-(methanesulfonyl)pyrimidin-5-yl)hex-5-ynoylamide DL-05 (8.1 mg, yield 7%).
LCMS (ESI) [M+H]⁺ = 1085.5;
¹H NMR (400 MHz, DMSO-d6) δ 9.31 (s, 1H), 9.11 (s, 2H), 8.72 (t, *J* = 6.8 Hz, 1H), 8.51 (d, *J* = 8.8 Hz, 1H), 8.19 (t, *J* = 6.8 Hz, 1H), 8.10 (d, *J* = 7.1 Hz, 1H), 7.91 (d, *J* = 8.6 Hz, 1H), 7.79 (d, *J =* 10.9 Hz, 1H), 7.32 (s, 1H), 6.53 (s, 1H), 5.63-5.54 (m, 1H), 5.42 (s, 2H), 5.20 (s, 2H), 4.68-4.58 (m, 2H), 4.26-4.12 (m, 2H), 4.01 (s, 2H), 3.73 (d, *J =* 5.4 Hz, 2H), 3.20-3.10 (m, 2H), 3.05-2.95 (m, 2H), 2.76 (d, *J* = 4.7 Hz, 6H), 2.55-2.53 (m, 2H), 2.42-2.28 (m, 6H), 2.21-2.13 (m, 2H), 2.02-1.77 (m, 6H), 1.76-1.62 (m, 2H), 1.62-1.51 (m, 3H), 1.37-1.21 (m, 2H), 0.90-0.80 (m, 9H).

### Example 9. Preparation of Anti-MSLN Antibody-Drug Conjugates

### Preparation of Samples of Antibody-Drug Conjugates PR300159-B81, PR300159-8-B81, PR300186-B81, or PR300186-10-B81

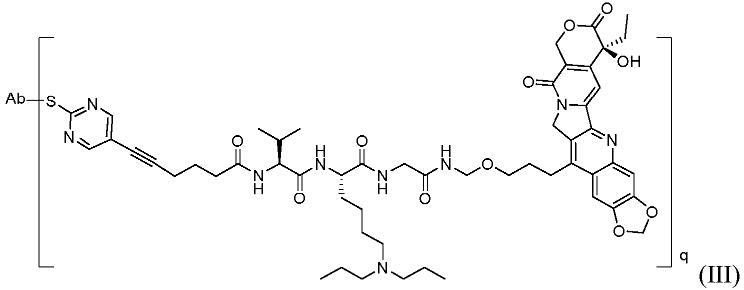

Ab is the anti-MSLN antibodies PR300159, PR300159-8, PR300186, or PR300186-10.

30 mg of anti-MSLN antibody PR300159-8 or PR300186-10 was diluted with a diluent (20 mM PB, pH 7.5), a sodium edetate solution with a final concentration of 5 mM was added, and same were mixed thoroughly; 5.5 molar equivalents of a TCEP solution relative to the antibody were added, same were mixed thoroughly, and same were left to stand at room temperature for 60 minutes; 12 molar equivalents, relative to the antibody, of DL-01 dissolved in dimethyl sulfoxide were added to the solution system, and same were mixed thoroughly and left to stand at room temperature for 2 hours to obtain the conjugated sample. After the reaction, a 30 kDa ultrafiltration tube was used to exchange the sample into a 20 mM histidine buffer solution at pH 6.0 and low-molecular-weight substances were removed. Finally, the sample was concentrated to obtain a solution of the ADC conjugate containing the anti-MSLN antibody PR300159-8 or PR300186-10, named PR300159-B81, PR300159-8-B81, PR300186-B81, and PR300186-10-B81, respectively. The DAR value was determined to be 8.0 using the mass spectrometry method described in Example 10.

Preparation of Sample of Antibody-Drug Conjugate PR300159-B71 or PR300186-B71

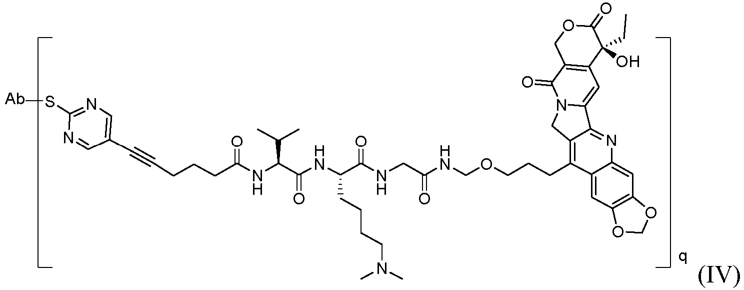

Ab is the anti-MSLN antibodies PR300159 or PR300186.

30 mg of anti-MSLN antibody PR300159 or PR300186 was diluted with a diluent (20 mM PB, pH 7.5), a sodium edetate solution with a final concentration of 5 mM was added, and same were mixed thoroughly; 5.5 molar equivalents, relative to the antibody, of a TCEP solution were added, same were mixed thoroughly, and same were left to stand at room temperature for 60 minutes; 12 molar equivalents, relative to the antibody, of DL-03 dissolved in dimethyl sulfoxide were added to the solution system, same were mixed thoroughly, and left to stand at room temperature for 2 hours to obtain a conjugated sample. After the reaction, a 30 kDa ultrafiltration tube was used to exchange the sample into a 20 mM histidine buffer solution at pH 6.0 and low-molecular-weight substances were removed, then the sample was concentrated to obtain a solution of the ADC conjugate containing the anti-MSLN antibody PR300159 or PR300186, named PR300159-B71 and PR300186-B71, respectively. The DAR value was determined to be 8.0 using the mass spectrometry method described in Example 10.

### Example 10. Determination of DAR Value of Conjugated Samples Using Mass Spectrometry

Detection: The molecular weight and DAR value of PR300159-8-B81 were analyzed using LC-MS under the following conditions:

### Chromatographic Conditions:

Chromatographic column: Xbridge Protein BEH SEC (2.5 µm, 4.6 × 150 mm)
Mobile phase A: 0.1% FA/H₂O
Mobile phase B: 0.1% FA/ACN
Column temperature: 30°C
Sample compartment temperature: 8°C
Flow rate: 0.3 mL/min
Injection volume: 1 µL

**Table 11. Chromatographic Conditions**

| | | | | | |
|---|---|---|---|---|---|
| Time (min) | 0 | 5 | 7 | 7.1 | 10 |
| Mobile Phase A | 90 | 20 | 20 | 90 | 90 |
| Mobile Phase B | 10 | 80 | 80 | 10 | 10 |

Sample Preparation: 50 µg of the sample was taken, respectively, 2 µL of 1M DTT was added, and same were diluted to 50 µL with ultrapure water to achieve an approximate concentration of 1.0 mg/mL. Same were mixed well and reduced at room temperature for 30 minutes.

LC/MS Model: UPLC (AB SCIEX), high-resolution mass spectrometer (AB SCIEX).

Mass Spectrometry Conditions: Gas1:45; Gas2:45; CUR: 30; TEM: 450; ISVF: 5000; DP: 120; CE: 12; mass range: 600-4000
The results are shown in Table 12.

**Table 12. Theoretical and Measured Molecular Weights**

| Peptide Chain | | mAb | DAR1 | DAR2 | DAR3 |
|---|---|---|---|---|---|
| LC | Theoretical Value | 23485.9 | 24504.1 | 25522.3 | 26540.5 |
| | Measured Value | Not Detected | 24503.4 | Not Detected | Not Detected |
| HC | Theoretical Value | 50544.2 | 51562.4 | 52580.6 | 53598.7 |
| | Measured Value | Not Detected | Not Detected | Not Detected | 53598.5 |

In Table 12, mAb represents the unconjugated monoclonal antibody; LC represents the antibody light chain; HC represents the antibody heavy chain; DAR1 represents a conjugate containing one toxin molecule conjugated to the light or heavy chain; DAR2 represents a conjugate containing two toxin molecules conjugated to the light or heavy chain; DAR3 represents a conjugate containing three toxin molecules conjugated to the light or heavy chain. The theoretical molecular weight of the monoclonal antibody is calculated based on the G0F glycoform. In the following text, mAb, LC, HC, DAR1, DAR2, and DAR3 are as explained above.

The test results show that for PR300159-8-B81, the percentage of antibody light chains conjugated with 0-1 toxin molecule(s) (LC, DAR1) is 0% and 100%, respectively. For heavy chains conjugated with 0-3 toxin molecules (mAb, DAR1, DAR2, DAR3), the percentages are 0%, 0%, 0%, and 100.0%, respectively. Based on this, the drug-to-antibody ratio (DAR value) of PR300159-8-B81 and PR300186-10-B81 is calculated to be 8.0.

### Example 11. Construction of the HT29-huMSLN Wild-Type Cell Line and HT29-huMSLN Mutant Cell Line

The HT29-huMSLN wild-type cell line (stably transfected wild-type human MSLN) was prepared in the laboratory, with the specific operations being as follows.

The HT-29 cell suspension was added to a 6-well plate at a density of 5E5/well and cultured at 37°C overnight. A plasmid encoding the human MSLN gene was added to a tube, and Opti-MEM (Gibco, Catalog No. 31985070) was then added for dilution. The transfection reagent Lipofectamine 3K was added to a separate tube and also diluted with Opti-MEM. The ratio of Lipofectamine 3K to DNA was 3:1. The diluted Lipofectamine 3K was added to the diluted plasmid, and same were mixed thoroughly and incubated at room temperature for 15 minutes. This mixture was then added to the cells, and same were gently mixed by shaking and incubated at 37°C overnight. After, the supernatant was discarded, and fresh medium containing 0.3 µg/mL G418 was added as a replacement. The cells were cultured at 37°C, with the G418-containing medium replaced every three days. Once the cells reached confluence, FACS validation was performed. The validated cell pool was diluted to 5 cells/mL, which was inoculated at 100 µL/well into five 96-well plates. The plates were incubated at 37°C for 14 days. Single clones were selected and validated by FACS. The validated clones were subjected to continuous expansion culture and cryopreserved.

The HT29-huMSLN mutant cell line (stably transfected mutant human MSLN) was prepared in the laboratory. The construction method was the same as that for the HT29-huMSLN wild-type cell line, except the mutant human MSLN gene was transfected, wherein, in the mutant MSLN gene, the YLVLDLSV sequence of the wild-type MSLN gene was mutated into GGGGS.

### Example 12. Detection of MSLN Shedding in HT29-huMSLN Wild-Type 2C3 Cells and HT29-huMSLN Mutant 1A11 Cells

HT29-huMSLN wild-type 2C3 cells and HT29-huMSLN mutant 1A11 cells were inoculated into 6-well plates at a density of 5×10⁵ cells/well, respectively. On the following day, the medium was replaced with 1 mL of fresh medium. Every hour, 100 µL of the medium was collected and stored at -80°C for subsequent detection of MSLN shedding in cells.

PR300159 was diluted to a concentration of 5 µg/mL and added to a 96-well plate, 100 µL per well to incubate overnight at 4°C. The 96-well plate was washed three times with a PBST solution, a PBS solution containing 2% BSA was then added, and the plate was left to stand at 37°C for 1 hour. Human MSLN protein (Acro Biosystems, Catalog # MSN-H522) was diluted two-fold starting from 0.2 µg/mL for a total of 11 dilutions to prepare the standard curve. The collected cell supernatant was added to the 96-well plate and incubated at 37°C for 1 hour. The plate was washed three times with a PBST solution, biotin-labeled anti-MSLN positive control antibody PR300175 (which binds to a different epitope than PR300159, with heavy and light chain sequences as shown in SEQ ID NO: 78 and 79, respectively) was added, and same were incubated at 37°C for 1 hour. The plate was washed three times with the PBST solution, the SA-HRP secondary antibody (diluted 5000×) was added and incubated at 37°C for 30-60 minutes. The plate was washed three times with the PBST solution, a TMB color development solution was added for 5-15 minutes, and color development was stopped by adding a stop solution.

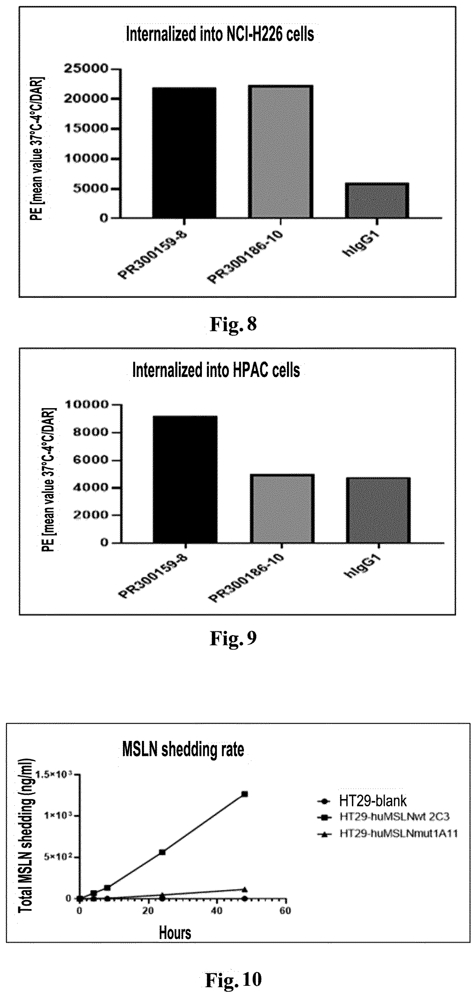

As shown in Figure 10, the HT29-huMSLN mutant 1A11 cell line exhibited only a small amount of MSLN shedding compared to the HT29-huMSLN wild-type 2C3 cell line.

### Example 13. Killing Assay of PR300159-8-B81 Antibody-Drug Conjugate Against MSLN-Expressing Cells

HT29-huMSLN wild-type 2C3 cells were added into a 96-well plate at 2000 cells/well. Then, serially diluted PR300159-8, hIgG-B81 (human IgG control conjugated with DL-01), and PR300159-8-B81 were added. After 6 days, cell viability was measured using the Promega CellTiter Glo assay (G7572).

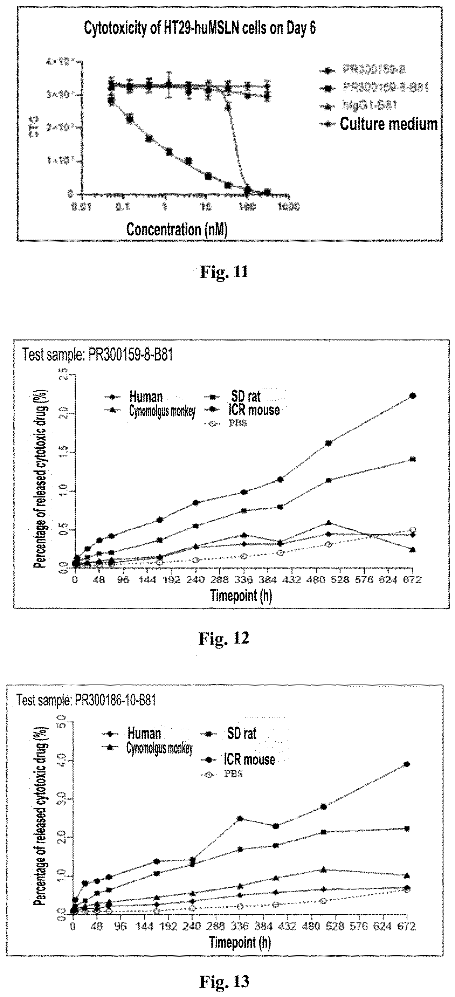

As shown by the experimental results in Figure 11, PR300159-8-B81 exhibits a significant killing effect against HT29-huMSLN cells.

### Example 14. Pharmacokinetic Study of PR300159-8-B81 and PR300186-10-B81 Antibody-Drug Conjugates

Six male SD rats were selected. Antibody-drug conjugates were administered intravenously at a dose of 5 mg/kg. Plasma and serum samples were collected at the following time points: before the dose was given, 10 minutes, 2 hours, 8 hours, 24 hours (Day 1), Day 3, Day 7, Day 10, Day 14, Day 21, and Day 28 after the dose was given.

Plasma sample collection: Whole blood was placed in test tubes containing K2-EDTA and centrifuged at 2-8°C for 6 minutes, and the plasma samples were subjected to cryopreservation at approximately -80°C until analysis.

Serum sample collection: Whole blood was left to clot for 30 minutes and centrifuged at 2200 g for 10 minutes at 4°C, and the separated serum samples were frozen at -80°C until analysis.

Detection of the antibody-drug conjugate (bound antibody): This example used an ELISA method to quantify the concentration of antibody-drug conjugates in rat serum, and specifically involves the following. A mouse Fab monoclonal antibody against the toxin molecule A1 was coated on a 96-well plate to capture the antibody-drug conjugates in the mouse serum. An HRP-labeled goat anti-human Fc secondary antibody was then added for detection.

Detection of total antibodies: This example used an ELISA method to quantify the concentration of total antibodies in rat serum. In the ELISA method, a sheep anti-human Fc monoclonal antibody was coated on a 96-well plate to capture the antibodies in the mouse serum. An HRP-labeled goat anti-human IgG secondary antibody was then added for detection.

Toxin molecule A1 detection: This example used the LC-MS/MS method to determine the concentration of small molecules in serum. 30 µL of a plasma sample was added to 100 µL of acetonitrile containing 0.5 ng/mL internal standard (YL0010098) for protein precipitation. The mixture was vortexed for 1 minute and then centrifuged. The supernatant was taken for LC-MS/MS analysis (Waters XEVO TQ-XS).

Serum drug concentration data was analyzed using Phoenix WinNonlin software version 7.0 and the non-compartmental analysis (NCA) model so as to calculate pharmacokinetic parameters.

Table 13 shows the pharmacokinetic parameters of PR300159-8-B81 and PR300186-10-B81. The results indicate that after administration of PR300159-8-B81 and PR300186-10-B81, the half-life of total antibodies for PR300159-8-B81 and PR300186-10-B81 in rats was 203 and 232 hours, respectively, while the half-life of the antibody-drug conjugate (ADC) for PR300159-8-B81 and PR300186-10-B81 in rats was 193 and 250 hours, respectively.

**Table 13. Pharmacokinetic Parameters of PR300159-8-B81 and PR300186-10-B81**

| | PR300159-8-B81 | | | PR300186-10-B81 | | |
|---|---|---|---|---|---|---|
| | Antibody-Drug Conjugate | Total Antibodies | Toxin Molecule A1 | Antibody-Drug Conjugate | Total Antibodies | Toxin Molecule A1 |
| Cmax ng/mL | 172101 | 133958 | 0.177 | 114266 | 107302 | 0.330 |
| Tmax hr | 0.167 | 0.167 | 2 | 0.167 | 0.167 | 2 |
| T1/2 hr | 193 | 203 | - | 250 | 232 | - |
| AUClast hr*ng/mL | 9123190 | 8737818 | 0.618 | 8906196 | 9561752 | 0.552 |
| AUCINF hr*ng/mL | 9844556 | 9597927 | - | 10376761 | 10758265 | - |
| MRTlast hr | 172 | 179 | 1.80 | 208 | 191 | 1.19 |
| MRTINF hr | 229 | 249 | - | 326 | 282 | - |
| Vss mL/kg | 116 | 130 | - | 158 | 131 | - |
| Vz mL/kg | 142 | 153 | - | 174 | 156 | - |
| CL mL/hr/kg | 0.508 | 0.522 | - | 0.482 | 0.465 | - |
| C0 ng/mL | 176823 | 138257 | 0.117 | 117107 | 109603 | 0.230 |

### Example 15. Stability Study of PR300159-8-B81 and PR300186-10-B81 Antibody-Drug Conjugates in Plasma of Humans, Cynomolgus Monkeys, SD Rats, and ICR Mice

Into plasma from various species (humans, cynomolgus monkeys, SD rats, and ICR mice) and into PBS containing 1% BSA, the antimicrobial agent ProClin was added, wherein the final concentration of ProClin was 0.1%. Sterile operations were conducted at 37°C. The antibody-drug conjugate stock solution was diluted to a working solution with a concentration of 20 mg/mL using PBS or 1 mM EDTA buffer of pH 7. 4 µL of a PR300159-8-B81 or PR300186-10-B81 working solution was respectively mixed with 396 µL of plasma from various species (humans, cynomolgus monkeys, SD rats, or ICR mice) or PBS containing 1% BSA to prepare plasma samples of various species containing PR300159-8-B81 or PR300186-10- at a concentration of 200 µg/mL. The volume of organic solvent did not exceed 1%. The samples were incubated in a 37°C, 5% CO₂ incubator with the sample plates being sealed with film and shaken at 80 cycles/min. The incubation times were set at 0 h, 4 h, 24 h, 2 d, 3 d, 7 d, 10 d, 14 d, 17 d, 21 d, and 28 d. 20 µL plasma samples were transferred, to which 200 µL of precipitant (containing internal standard) was added, and they were thoroughly mixed to precipitate proteins. After all samples had undergone protein precipitation, same were centrifuged, and the supernatant was collected, diluted with water, and analyzed using LC-MS/MS.

Figures 12 and 13 show the stability results of PR300159-8-B81 and PR300186-10-B81 in the plasma of humans, cynomolgus monkeys, SD rats, and ICR mice, and in phosphate-buffered saline (PBS).

The results in Figure 12 show that within 28 days, the percentage of cytotoxic drug released from PR300159-8-B81 was less than 0.5% in human and cynomolgus monkey plasma, less than 1.5% in SD rat plasma, and less than 2.5% in ICR mouse plasma.

The results in Figure 13 show that within 28 days, the percentage of cytotoxic drug released from PR300186-10-B81 was less than 1.0% in human and cynomolgus monkey plasma, less than 2.5% in SD rat plasma, and less than 4.0% in ICR mouse plasma.

### Example 16. Colon Cancer HT29-huMSLN Wild-Type Cell Line Model

HT29-huMSLN wild-type 2C3 cells were cultured in a RPMI-1640 medium (containing 10% fetal bovine serum and 0.3 mg/mL G418) at 37°C in a 5% CO₂ atmosphere. On the day of inoculation, HT29-huMSLN wild-type 2C3 cells in the exponential growth phase were collected. The tumor cells were resuspended in a mixture of PBS and Matrigel (1:1) and were then subcutaneously inoculated. Each BALB/c nude mouse was subcutaneously inoculated with 1 × 10⁶ HT29-huMSLN wild-type 2C3 cells. When the average tumor volume in each group of mice reached 100 mm³, the mice were divided into groups for dosing. Thirty mice were randomly divided into six groups, after which dosing was initiated. The dosing schedule was twice a week for a total of six administrations via tail vein injection at the doses of 5 mg/kg body weight or 1 mg/kg body weight. After dosing began, body weight and tumor volume were measured twice weekly. Tumor volume was calculated using the formula of: Tumor Volume (mm³) = 0.5 × Tumor long diameter × Tumor short diameter². Observations were ended on day 38 post-administration, followed by the euthanasia of all mice. Data analysis was performed using the t-test.

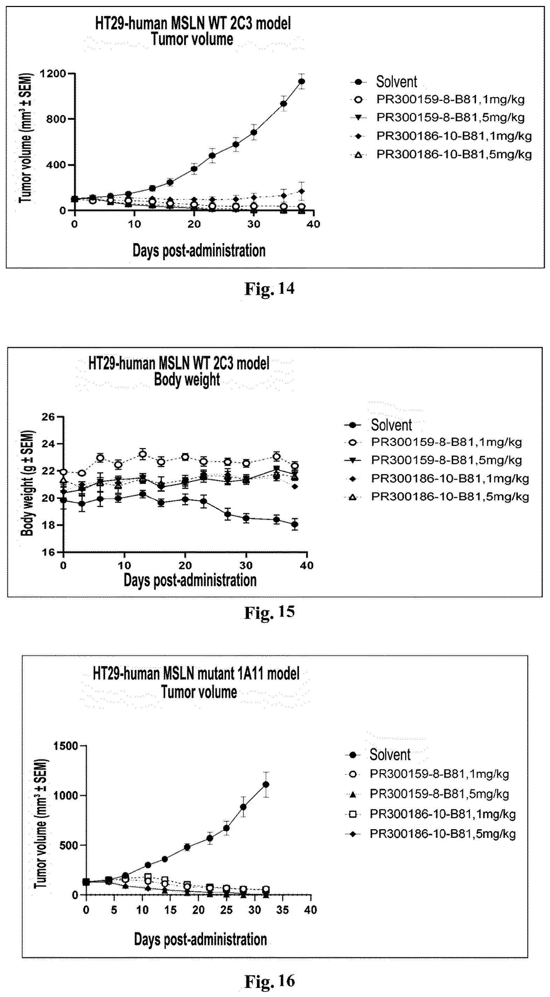

Figure 14 shows the results of the in-vivo pharmacodynamics study of PR300159-8-B81 and PR300186-10-B81. On day 38 post-administration, the average tumor volume in the solvent vehicle control group of mice was 1131 mm³. In the hIgG1-B81 (5 mg/kg) control group of mice, the average tumor volume on day 38 post-administration was 934 mm³. In the test drug PR300159-8-B81 (1 mg/kg) treatment group, the average tumor volume was 34 mm³ on day 38 post-administration, showing a significant difference compared to the solvent control group (p < 0.001) and a tumor growth inhibition rate (TGI) of 97%. In the test drug PR300159-8-B81 (5 mg/kg) treatment group, the tumors of the mice were completely eliminated on day 38 post-administration, showing a significant difference compared to the solvent control group (p < 0.001) and a tumor growth inhibition rate (TGI, %) of 100%. In the test drug PR300186-10-B81 (1 mg/kg) treatment group, the average tumor volume was 168 mm³ on day 38 post-administration, showing a significant difference compared to the solvent control group (p < 0.001) and a tumor growth inhibition rate (TGI, %) of 85%. In the test drug PR300186-10-B81 (5 mg/kg) treatment group, the tumors of the mice were completely eliminated on day 38 post-administration, demonstrating significant tumor inhibition compared to the solvent control group (p < 0.001) and a tumor growth inhibition rate (TGI, %) of 100%.

Figure 15 shows the mouse body weight results. Throughout the treatment and administration period, all animals exhibited good drug tolerance, with no severe weight loss or deaths observed.

### Example 17. Colon Cancer HT29-huMSLN Mutant Cell Line Model

HT29-huMSLN mutant 1A11 cells were cultured in a RPMI-1640 medium (containing 10% fetal bovine serum and 0.3 mg/mL G418) at 37°C in a 5% CO₂ atmosphere. On the day of inoculation, HT29-huMSLN mutant 1A11 cells in the exponential growth phase were collected. Each BALB/c nude mouse was subcutaneously inoculated with 1 × 10⁶ HT29-huMSLN mutant 1A11 cells. Tumor cells were first resuspended in a mixture of PBS and Matrigel (1:1) before subcutaneous inoculation. When the average tumor volume in each group of mice reached 100 mm³, the mice were divided into groups for dosing. 25 mice were divided into 5 groups, after which dosing began. The dosing schedule was twice a week for a total of six administrations via tail vein injection at doses of 1 mg/kg body weight or 5 mg/kg body weight. After dosing began, body weight and tumor volume were measured twice weekly. Tumor volume was calculated using the formula of: Tumor Volume (mm³) = 0.5 × Tumor long diameter × Tumor short diameter². Observations were ended on day 32 post-administration, followed by the euthanasia of all mice. Data analysis was performed using the t-test.

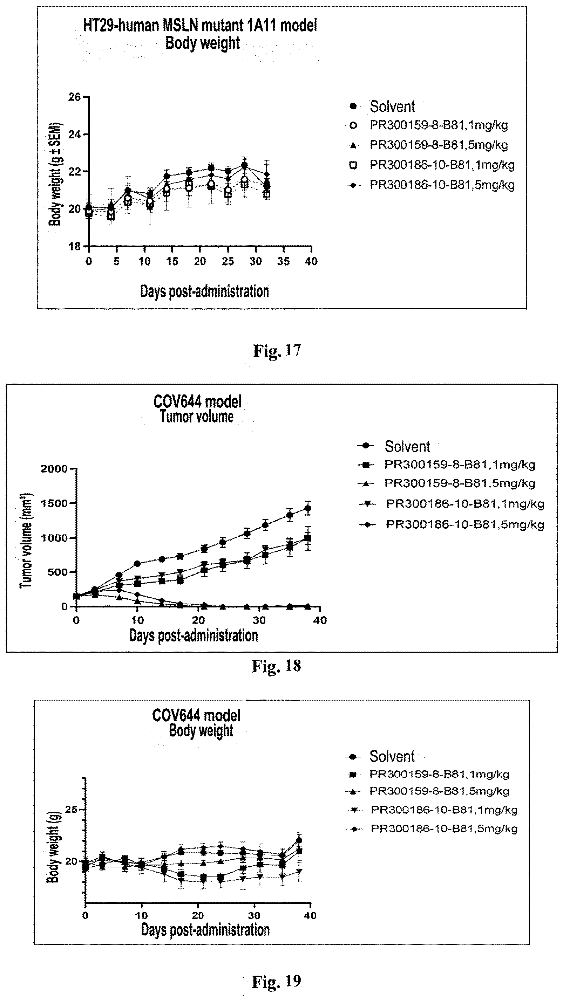

Figure 16 shows the antitumor efficacy results of PR300159-8-B81 and PR300186-10-B81. Figure 17 shows the body weight results of the mice.

As shown in Figure 16, on day 32 post-administration, the average tumor volume in the control group of mice was 1109 mm³. In the test drug PR300159-8-B81 (1 mg/kg) treatment group, the average tumor volume was 60.4 mm³ on day 32 post-administration, showing a significant difference compared to the solvent control group (p < 0.001) and a tumor growth inhibition rate (TGI) of 95%. In the test drug PR300159-8-B81 (5 mg/kg) treatment group, the tumors of the mice were completely eliminated on day 32 post-administration, showing a significant difference compared to the solvent control group (p < 0.001) and a tumor growth inhibition rate (TGI, %) of 100%. In the test drug PR300186-10-B81 (1 mg/kg) treatment group, the average tumor volume was 59.5 mm³ on day 32 post-administration, showing a significant difference compared to the solvent control group (p < 0.001) and a tumor growth inhibition rate (TGI, %) of 95%. In the test drug PR300186-10-B81 (5 mg/kg) treatment group, the tumors of the mice were completely eliminated on day 32 post-administration, demonstrating significant tumor suppression compared to the solvent control group (p < 0.001) and a tumor growth inhibition rate (TGI, %) of 100%. These results indicate that PR300159-8-B81 and PR300186-10-B81 exhibit equivalent antitumor efficacy in the HT29-huMSLN mutant cell in-vivo model.

As shown in Figure 17, throughout the treatment period, all the animals exhibited good drug tolerance, with no severe weight loss or animal deaths observed.

### Example 18. Ovarian Cancer COV644 Model

COV644 cells (ECACC, catalog: 07071908) were cultured in a RPMI-1640 medium containing 10% fetal bovine serum at 37°C in a 5% CO₂ atmosphere. On the day of inoculation, COV644 cells in the exponential growth phase were collected. Tumor cells were resuspended in a mixture of PBS and Matrigel (1:1) and then subcutaneously inoculated. Each BALB/c nude mouse was subcutaneously inoculated with 5 × 10⁶ COV644 tumor cells. When the average tumor volume in each group of mice reached 150 mm³, the mice were divided into groups for dosing. 25 mice were divided into 5 groups, after which dosing began. The dosing schedule was twice a week for a total of five administrations via tail vein injection at doses of 1 mg/kg body weight or 5 mg/kg body weight. After dosing began, body weight and tumor volume were measured twice weekly. Tumor volume was calculated using the formula of: Tumor Volume (mm³) = 0.5 × Tumor long diameter × Tumor short diameter². Observations were ended on day 38 post-administration, followed by the euthanasia of all mice. Data analysis was performed using the t-test.

Figure 18 shows the in-vivo pharmacodynamics results of PR300159-8-B81 and PR300186-10-B81. On day 38 post-administration, the average tumor volume in the solvent control group of mice was 1429 mm³. In the test drug PR300159-8-B81 (1 mg/kg) treatment group, the average tumor volume was 993 mm³ on day 38 post-administration, and a tumor growth inhibition rate (TGI) was 31%. In the test drug PR300159-8-B81 (5 mg/kg) treatment group, tumors were completely eliminated by day 38 post-administration, showing a significant difference compared to the solvent control group (p < 0.001) and a tumor growth inhibition rate (TGI, %) of 100%. In the test drug PR300186-10-B81 (1 mg/kg) treatment group, the average tumor volume was 995 mm³ on day 38 post-administration, and the tumor growth inhibition rate (TGI, %) was 30%. In the test drug PR300186-10-B81 (5 mg/kg) treatment group, on day 38 post-administration, four mice in the group exhibited complete tumor elimination, while one mouse had a tumor volume of 14 mm³. The average tumor volume in this group of mice was 8 mm³, showing significant tumor suppression compared to the solvent control group (p < 0.001) and a tumor growth inhibition rate (TGI, %) of 99%. PR300159-8-B81 and PR300186-10-B81 exhibit equivalent antitumor efficacy in the COV644 in-vivo model.

Figure 19 shows the body weight results of the mice. Throughout the treatment period, all the animals exhibited good drug tolerance, with no severe weight loss or animal deaths observed.

### Example 19. Ovarian Cancer OVCAR3 Model

OVCAR3 cells were cultured in a RPMI-1640 medium (containing 20% fetal bovine serum and 0.01 mg/mL bovine insulin) at 37°C in a 5% CO₂ atmosphere. On the day of inoculation, OVCAR3 cells in the exponential growth phase were collected. Tumor cells were resuspended in a mixture of PBS and Matrigel (1:1) and then subcutaneously inoculated. Each BALB/c nude mouse was subcutaneously inoculated with 1 × 10⁷ OVCAR3 tumor cells. When the average tumor volume in each group of mice reached 200 mm³, the mice were divided into groups for dosing. 35 mice were divided into seven groups, after which dosing began. The dosing schedule was twice a week for a total of five administrations via tail vein injection at doses of 1 mg/kg body weight or 5 mg/kg body weight. After dosing began, body weight and tumor volume were measured twice weekly. Tumor volume was calculated using the formula of: Tumor Volume (mm³) = 0.5 × Tumor long diameter × Tumor short diameter². Observations were ended on day 23 post-administration, followed by the euthanasia of all mice. Data analysis was performed using the t-test.

PR300159-8-DXD was synthesized by ChemPartner (Shanghai) according to a trastuzumab deruxtecan (DS-8201, Enhertu) synthesis method (WO2015115091A1), wherein the trastuzumab in DS-8201 was replaced with PR300159-8.

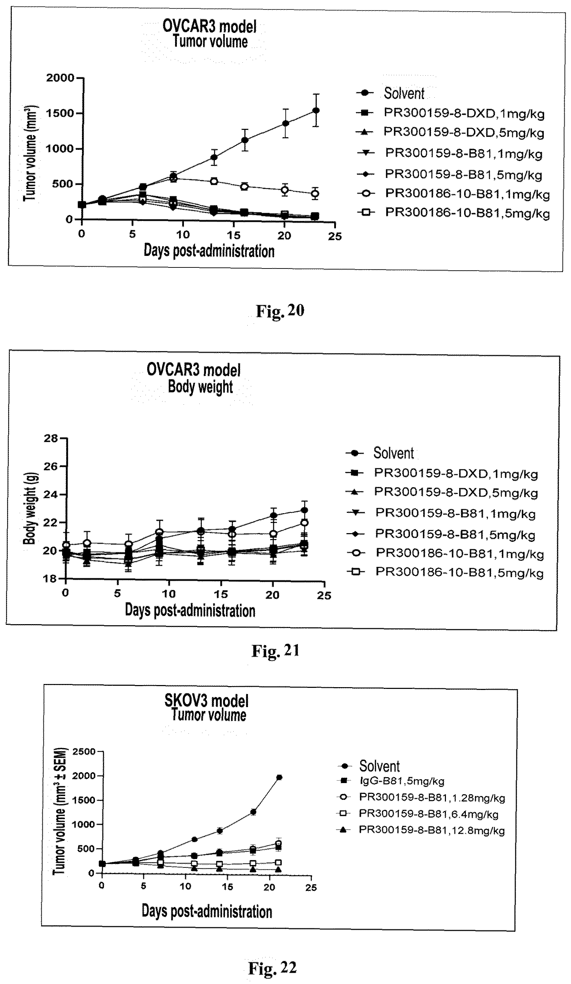

Figure 20 shows the in-vivo pharmacodynamics results of PR300159-8-DXD, PR300159-8-B81, and PR300186-10-B81. On day 23 post-administration, the average tumor volume in the solvent control group of mice was 1811 mm³. In the test drug PR300159-8-DXD (1 mg/kg) treatment group, the average tumor volume was 51 mm³ on day 23 post-administration, showing a significant difference compared to the solvent control group (p = 0.001) and a tumor growth inhibition rate (TGI) of 97%. In the test drug PR300159-8-DXD (5 mg/kg) treatment group, the average tumor volume was 47 mm³ on day 23 post-administration, showing a significant difference compared to the solvent control group (p = 0.001) and a tumor growth inhibition rate (TGI, %) of 97%. In the test drug PR300159-8-B81 (1 mg/kg) treatment group, the average tumor volume was 35 mm³ on day 23 post-administration, showing a significant difference compared to the solvent control group (p = 0.001) and a tumor growth inhibition rate (TGI, %) of 98%. In the test drug PR300159-8-B81 (5 mg/kg) treatment group, the average tumor volume was 54 mm³ on day 23 post-administration, showing a significant difference compared to the solvent control group (p = 0.001) and a tumor growth inhibition rate (TGI, %) of 97%. In the test drug PR300186-10-B81 (1 mg/kg) treatment group, the average tumor volume was 429 mm³ on day 23 post-administration, showing a significant tumor inhibition effect compared to the solvent control group (p = 0.002) and a tumor growth inhibition rate (TGI, %) of 76%. In the test drug PR300186-10-B81 (1 mg/kg) treatment group, the average tumor volume was 67 mm³ on day 23 post-administration, with a significant tumor inhibition effect compared to the solvent control group (p = 0.001), and a tumor growth inhibition rate (TGI, %) of 96%. PR300159-8-DXD, PR300159-8-B81, and PR300186-10-B81 demonstrated significant antitumor effects at both 1 mg/kg and 5 mg/kg, with PR300186-10-B81 showing clear dose dependence.

Figure 21 shows the body weight results of the mice. Throughout the treatment period, all the animals exhibited good drug tolerance, with no severe weight loss or animal deaths observed.

### Example 20. Ovarian Cancer SKOV3 Model

SKOV3 cells were cultured in a DMEM medium containing 10% fetal bovine serum at 37°C in a 5% CO₂ atmosphere. On the day of inoculation, SKOV3 cells in the exponential growth phase were collected. Tumor cells were resuspended in a mixture of PBS and Matrigel (1:1) and then subcutaneously inoculated. Each BALB/c nude mouse was subcutaneously inoculated with 1 × 10⁷ SKOV3 tumor cells. When the average tumor volume in each group of mice reached 200 mm³, the mice were divided into groups for dosing. 42 mice were divided into seven groups, after which dosing began. The dosing schedule was twice a week for a total of four administrations via tail vein injection. After dosing began, body weight and tumor volume were measured twice weekly. Tumor volume was calculated using the formula of: Tumor Volume (mm³) = 0.5 × Tumor long diameter × Tumor short diameter². Observations concluded on day 21 post-administration, followed by the euthanasia of all mice. Data analysis was performed using the t-test.

Figure 22 shows the in-vivo pharmacodynamics results of PR300159-8-B81 and PR300186-10-B81. On day 21 post-administration, the average tumor volume in the solvent control group of mice was 2011 mm³. On day 21 post-administration, the average tumor volume in the test drug PR300159-8-B81 (1.28 mg/kg) treatment group was 657 mm³, showing a significant difference compared to the solvent control group (p < 0.001) and a tumor growth inhibition rate (TGI, %) of 67%. On day 21 post-administration, the average tumor volume in the test drug PR300159-8-B81 (6.4 mg/kg) treatment group was 261 mm³, showing a significant difference compared to the solvent control group (p < 0.001) and a tumor growth inhibition rate (TGI, %) of 87%. On day 21 post-administration, the average tumor volume in the test drug PR300159-8-B81 (12.8 mg/kg) treatment group was 122 mm³, showing a significant difference compared to the solvent control group (p < 0.001) and a tumor growth inhibition rate (TGI, %) of 94%. On day 21 post-administration the average tumor volume in the test drug PR300186-10-B81 (0.78 mg/kg) treatment group was 1064 mm³, showing a significant tumor inhibition effect compared to the solvent control group (p < 0.001) and a tumor growth inhibition rate (TGI, %) of 47%. On day 21 post-administration, the average tumor volume in the test drug PR300186-10-B81 (3.9 mg/kg) treatment group was 478 mm³, showing a significant tumor inhibition effect compared to the solvent control group (p < 0.001) and a tumor growth inhibition rate (TGI, %) of 76%. On day 21 post-administration the average tumor volume in the test drug PR300186-10-B81 (7.8 mg/kg) treatment group was 265 mm³, showing a significant tumor inhibition effect compared to the solvent control group (p < 0.001) and a tumor growth inhibition rate (TGI, 8%) of 87%. PR300159-8-B81 and PR300186-10-B81 demonstrated significant antitumor effects with clear dose dependence.

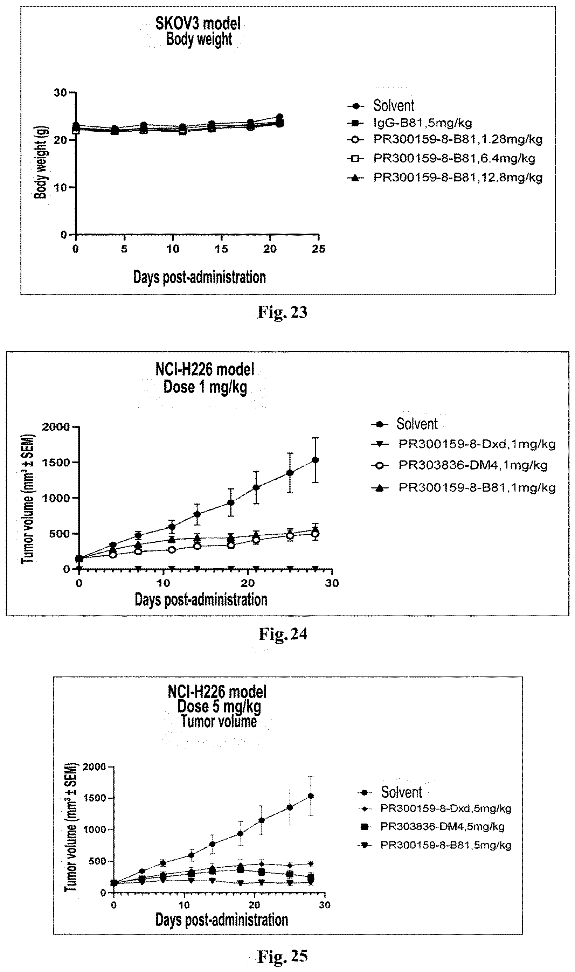

Figure 23 shows the body weight results of the mice. Throughout the treatment period, all the animals exhibited good drug tolerance, with no severe weight loss or animal deaths observed.

### Example 21. Lung Cancer NCI-H226 Model

NCI-H226 cells were cultured in a RPMI-1640 medium containing 10% fetal bovine serum at 37°C in a 5% CO₂ atmosphere. On the day of inoculation, NCI-H226 cells in the exponential growth phase were collected. Tumor cells were first resuspended in a mixture of PBS and Matrigel (1:1) and then subcutaneously inoculated. Each BALB/c Nude mouse was subcutaneously inoculated with 5 × 10⁶ NCI-H226 tumor cells. When the average tumor volume in each group of mice reached 150 mm³, the mice were divided into groups for dosing. 55 mice were divided into 11 groups, after which dosing began. The dosing schedule was twice a week for a total of five administrations via tail vein injection at doses of 1 mg/kg body weight or 5 mg/kg body weight. After dosing began, body weight and tumor volume were measured twice weekly. Tumor volume was calculated using the formula of: Tumor Volume (mm³) = 0.5 × Tumor long diameter × Tumor short diameter². Observations concluded on day 28 post-administration, followed by the euthanasia of all mice. Data analysis was performed using the t-test.

BAY-949343, also known as Anetumab ravtansine, is an ADC developed by Bayer and formed by the fully human anti-MSLN monoclonal antibody MF-T (anetumab) being conjugated to the microtubule inhibitor maytansinoid DM4 by amino means of coupling, wherein the linker is the N-succinimidyl-4-(2-pyridyldithio)butanoate (SPDB), the SPDB linker having a bystander killing effect (WO2010124797A1). BAY-949343 was synthesized by Shanghai Chempartner Co., Ltd.

Figures 24 and 25 show the in-vivo pharmacodynamic results of PR300159-8-DXD, BAY-949343, PR300159-8-B81, and PR300186-10-B81. On day 28 post-administration, the average tumor volume in the solvent control group of mice was 1535 mm³. In the test drug PR300159-8-DXD (1 mg/kg) treatment group, the average tumor volume was 743 mm³ on day 28 post-administration, showing a significant difference compared to the solvent control group (p = 0.05) and a tumor growth inhibition rate (TGI, %) of 52%. In the test drug PR300159-8-DXD (5 mg/kg) treatment group, the average tumor volume was 460 mm³ on day 28 post-administration, showing a significant difference compared to the solvent control group (p = 0.01) and a tumor growth inhibition rate (TGI, %) of 70%. In the test drug BAY-949343 (1 mg/kg) treatment group, the average tumor volume was 497 mm³ on day 28 post-administration, showing a significant difference compared to the solvent control group (p = 0.01) and a tumor growth inhibition rate (TGI, %) of 68%. In the test drug BAY-949343 (5 mg/kg) treatment group, the average tumor volume was 253 mm³ on day 28 post-administration, showing a significant difference compared to the solvent control group (p = 0.004) and a tumor growth inhibition rate (TGI, %) of 84%. In the test drug PR300159-8-B81 (1 mg/kg) treatment group, the average tumor volume was 556 mm³ on day 28 post-administration, showing a significant difference compared to the solvent control group (p = 0.02) and a tumor growth inhibition rate (TGI, %) of 64%. In the test drug PR300159-8-B81 (5 mg/kg) treatment group, the average tumor volume was 164 mm³ on day 28 post-administration, showing a significant difference compared to the solvent control group (p = 0.003) and a tumor growth inhibition rate (TGI, %) of 89%. In the test drug PR300186-10-B81 (1 mg/kg) treatment group, the average tumor volume was 526 mm³ on day 28 post-administration, showing a significant tumor inhibition effect compared to the solvent control group (p = 0.01) and a tumor growth inhibition rate of (TGI, %) of 66%. In the test drug PR300186-10-B81 (5 mg/kg) treatment group, the average tumor volume was 211 mm³ on day 28 post-administration, showing a significant tumor inhibition effect compared to the solvent control group (p = 0.003) and a tumor growth inhibition rate (TGI, %) of 86%. PR300159-8-B81, PR300186-10-B81, and BAY-949343 showed comparable antitumor efficacy, their antitumor efficacies all outperforming PR300159-8-DXD.

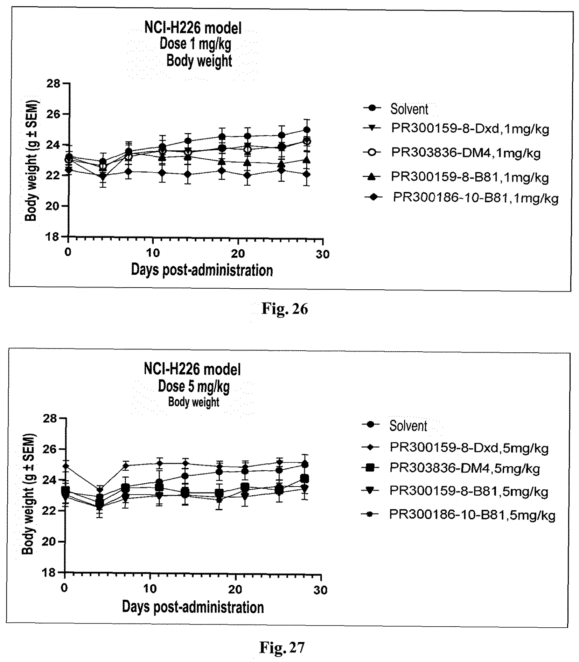

Figures 26 and 27 show the body weight results of the mice. Throughout the treatment period, all the animals exhibited good drug tolerance, with no severe weight loss or animal deaths observed.

### Example 22. Binding Activity Assay of PR300159, PR300159-B71, PR300186, and PR300186-B71 on CHOK1 Cells Overexpressing Human Mesothelin (CHOK1-huMSLN)

Flow cytometry was used to detect the binding activity of PR300159 and PR300186 antibodies, and of the antibody-drug conjugates (ADCs) PR300159-B71 and PR300186-B71, on CHOK1-huMSLN cells.

The antibodies PR300159 and PR300186 and the ADCs PR300159-B71 and PR300186-B71 were serially diluted in a buffer (PBS containing 2% FBS). 50 µL of diluted antibody solution (30 µg/mL) was added to 50 µL of a cell suspension containing 1-2 × 10⁵ cells and incubated at 4°C for 1 hour. The cells were washed twice with a buffer (PBS containing 2% serum), and 100 µL of a fluorescence-labeled anti-human IgG antibody (Alexa Fluor^{®}488 AffiniPure Goat Anti-Human IgG (H+L), Jackson ImmunoResearch, Catalog 109-545-088) was added to each well. After a 1-hour incubation at 4°C, the cells were washed twice with a buffer and analyzed by means of flow cytometry.

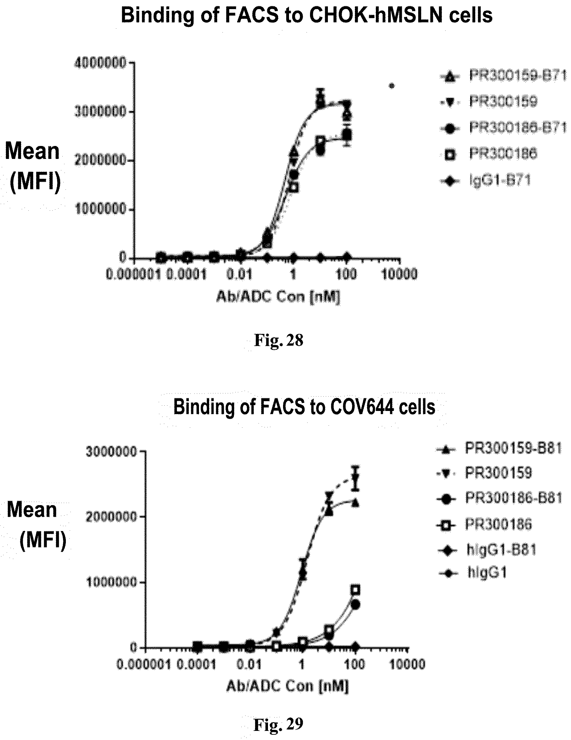

The results are shown in Table 14 and Figure 28. The results indicate that the antibodies PR300159 and PR300186 and their corresponding ADCs PR300159-B71 and PR300186-B71 all exhibited comparable binding activity on the cells.

**Table 14. Binding Activity of Antibodies or ADCs to Cell Surface MSLN as Detected by FACS**

| Sample Name | CHOK1-huMSLN | |
|---|---|---|
| | EC50 (nM) | TOP (MFI) |
| PR300159-B71 | 0.47 | 3181863 |
| PR300159 | 0.67 | 3229451 |
| PR300186-B71 | 0.48 | 2474656 |
| PR300186 | 0.76 | 2557001 |

### Example 23. Experiments of Binding Activity of PR300159, PR300159-B81, PR300186, and PR300186-B81 on Tumor Cells

Flow cytometry was used to detect the binding activity of the antibodies PR300159 and PR300186 antibodies and of the antibody-drug conjugates (ADCs) PR300159-B81 and PR300186-B81 on tumor cells. The tumor cells used in this example were the breast cancer cell line COV644.

The antibodies PR300159 and PR300186 and the ADCs PR300159-B81 and PR300186-B81 were serially diluted in a buffer (PBS containing 2% FBS). 50 µL of diluted antibody solution was added to 50 µL of a cell suspension containing 1-2 × 10⁵ COV644 cells and incubated at 4°C for 1 hour. The cells were washed twice with a buffer (PBS containing 2% serum), and 100 µL of a fluorescence-labeled anti-human IgG antibody (Alexa Fluor^{®}488 AffiniPure Goat Anti-Human IgG (H+L), Jackson ImmunoResearch, Catalog 109-545-088) was added to each well. After a 1-hour incubation at 4°C, the cells were washed twice with a buffer and analyzed by means of flow cytometry.

The results are shown in Table 15 and Figure 29. The results indicate that the antibodies and their corresponding ADCs exhibited comparable binding activity on COV644 cells.

**Table 15. Binding Activity of Antibodies and their ADCs to COV644 Cells**

| Sample Name | COV644 | |
|---|---|---|
| | EC50 (nM) | TOP (MFI) |
| PR300159-B81 | 0.90 | 2263900 |
| PR300159 | 1.43 | 2640283 |
| PR300186-B81 | >100 | 669007 |
| PR300186 | >100 | 890711 |

### Example 24. Experiments of Endocytosis Activity of PR300159, PR300159-B71, PR300186, and PR300186-B71 on Tumor Cells

Flow cytometry was used to assess the endocytosis efficiency of the antibodies PR300159 and PR300186 and of the antibody-drug conjugates (ADCs) PR300159-B71 and PR300186-B71 on COV644 tumor cells. The tumor cells used in this example were breast cancer cells from the COV644 cell line.

In this example, the pHAb Amine Reactive Dye (Promega, Cat # G9841) was used to determine the endocytosis efficiency of anti-MSLN antibodies on COV644 tumor cells. The pHAb dye is a pH sensor dye that exhibits very low fluorescence when the pH value is greater than 7, and the fluorescence significantly increases as the pH value of the solution decreases. Antibodies labeled with pHAb dye exhibit little or no fluorescence when bound to the extracellular membrane under neutral pH conditions. However, after the antibody labeled with the pHAb dye is endocytosed by cells, strong fluorescence is detected in the low-pH environments of the endosomes and lysosomes of the cells.

The antibodies were labeled with a pHAb dye, and the DAR values were calculated according to reagent kit instructions. The antibodies labeled with the pHAb dye were incubated with COV644 at 4°C (a temperature where antibody internalization activity is low, same being used as a background control) or 37°C for 24 hours. The fluorescence was then detected at an excitation value (Ex) of 532 nm and at an emission value (Em) of 560 nm. The way in which the antibody endocytosis results were calculated is: fluorescence intensity at 37°C minus background fluorescence intensity at 4°C, divided by the DAR value of the antibody-conjugated dye. Higher fluorescence values indicate greater endocytosis efficiency of the antibodies on the cells.

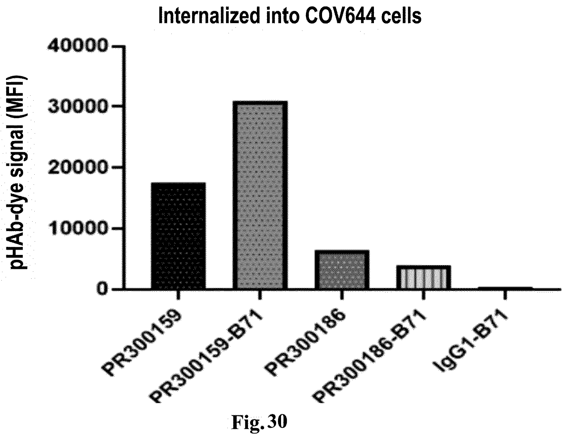

The results are shown in Figure 30. The data indicate that PR300159, PR300159-B71, PR300186, and PR300186-B71 exhibited good endocytosis activity on COV644 tumor cells.

### Example 25. Experiments with PR300159 and PR300186 Antibody-Drug Conjugates on Cells Expressing MSLN

HT29-huMSLN cells were inoculated into 96-well plates, 2000 cells per well, and then serially diluted PR300159, PR300159-B71, PR300186, PR300186-B71, or hIgG-B71 was added. After 6 days, cell viability was measured using the Promega CellTiter Glo assay (G7572).

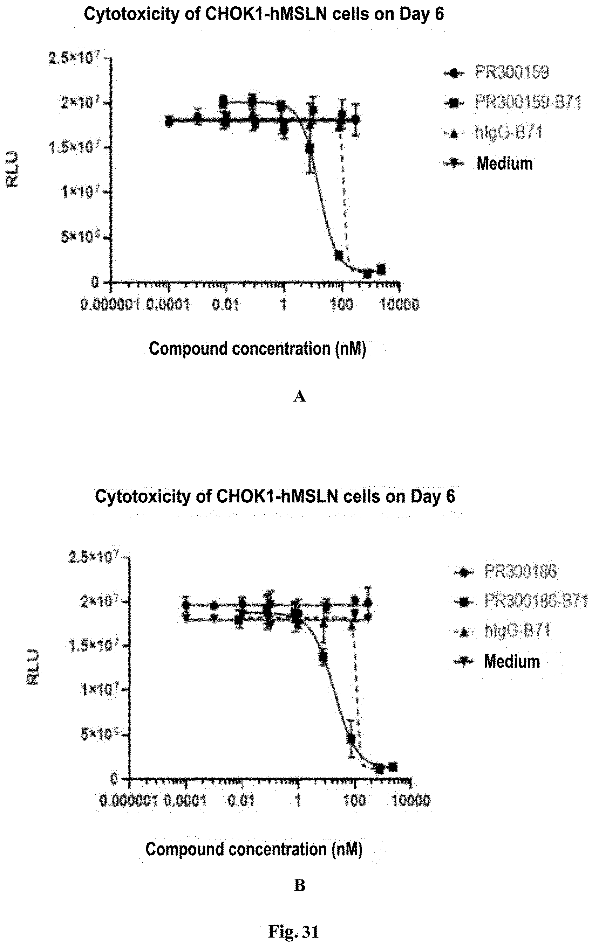

The results are shown in Figure 31. The results indicate that PR300159-B71 and PR300186-B71 exhibit significant killing effects on CHOK1-huMSLN.

### Example 26. Human Ovarian Cancer COV644 Model

COV644 cells (ECACC, catalog: 07071908) were cultured in a RPMI-1640 medium containing 10% fetal bovine serum at 37°C under 5% CO₂ conditions. COV644 cells in the exponential growth phase were collected on the day of inoculation, and 5 × 10⁶ COV644 tumor cells were subcutaneously inoculated into each BALB/c Nude mouse. Tumor cells were firstly resuspended in a mixture of PBS and Matrigel (1:1) before subcutaneous inoculation. When the average tumor volume in each group of mice reached 150 mm³, the mice were divided into groups for dosing. 78 mice were divided into 13 groups, after which dosing began. The dosing schedule was twice a week for a total of five administrations via tail vein injections. After dosing began, tumor volume and body weight were measured twice weekly, and the tumor volume was calculated using the formula of: Tumor Volume (mm³) = 0.5 × tumor long diameter × tumor short diameter². Observations were ended on day 21 post-administration, and then all the mice were euthanized. Data analysis was performed using the t-test.

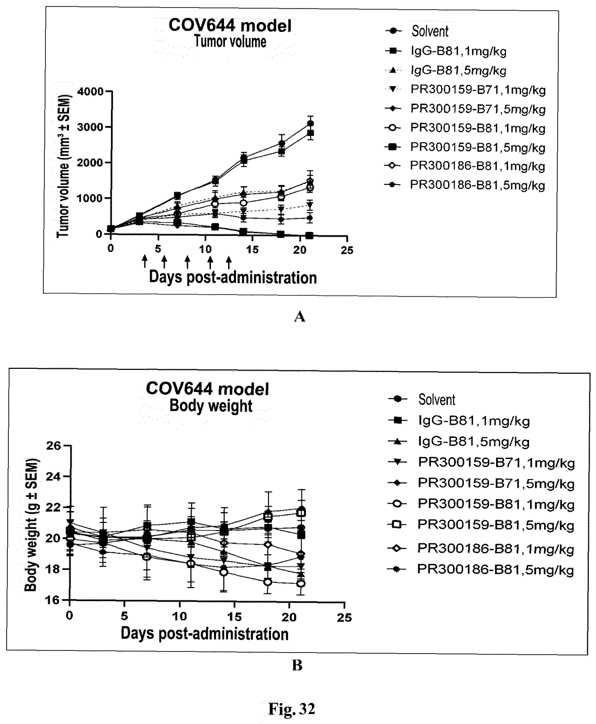

Figures 32A and 32B show the in-vivo pharmacodynamics results for PR300159-B71, PR300159-B81, and PR300186-B81. On day 21 post-administration, the average tumor volume in the control group of mice was 3154.82 mm³. The test drug IgG-B81 (1 mg/kg) treatment group had an average tumor volume of 1508 mm³ on day 21 post-administration, showing no significant difference compared to the solvent control group (p = 0.82) and a tumor growth inhibition rate (TGI, %) of 2.57%. The test drug IgG-B81 (5 mg/kg) treatment group had an average tumor volume of 1514.88 mm³ on day 21 post-administration, showing a significant difference compared to the solvent control group (p < 0.001) and a tumor growth inhibition rate (TGI, %) of 51.98%. The test drug PR300159-B71 (1 mg/kg) treatment group had an average tumor volume of 853.97 mm³ on day 21 post-administration, showing a significant difference compared to the solvent control group (p < 0.001) and a tumor growth inhibition rate (TGI, %) of 72.93%. The test drug PR300159-B71 (5 mg/kg) treatment group achieved complete tumor eradication on day 21 post-administration, showing a significant difference compared to the solvent control group (p < 0.001) and a tumor growth inhibition rate (TGI, %) of 100%. The test drug PR300159-B81 (1 mg/kg) treatment group had an average tumor volume of 1356.25 mm³ on day 21 post-administration, showing a significant difference compared to the solvent control group (p < 0.001) and a tumor growth inhibition rate (TGI, %) of 57.01%. The test drug PR300159-B81 (5 mg/kg) treatment group achieved complete tumor eradication on day 21 post-administration, showing a significant difference compared to the solvent control group (p < 0.001) and a tumor growth inhibition rate (TGI, %) of 100%. The test drug PR300186-B81 (1 mg/kg) treatment group had an average tumor volume of 1544.39 mm³ on day 21 post-administration, showing no significant difference compared to the solvent control group (p < 0.001) and a tumor growth inhibition rate (TGI, %) of 51.05%. The test drug PR300186-B81 (5 mg/kg) treatment group had an average tumor volume of 500.16 mm³ on day 21 post-administration, showing a significant difference compared to the solvent control group (p < 0.001) and a tumor growth inhibition rate (TGI, %) of 84.15%. PR300159-B71, PR300159-B81, and PR300186-B81 demonstrated significant antitumor efficacy in the COV644 in-vivo model.

Throughout the treatment period, all animals exhibited good drug tolerance, with no severe weight loss or deaths observed.

## Claims

1. An antibody-drug conjugate, which is a compound of formula (I) or a tautomer, stereoisomer, or pharmaceutically acceptable salt thereof:
Ab-[S-X-(Y)ₙ-Z-E-D]_{q} (I),
wherein,
Ab is an antibody that specifically binds to mesothelin (MSLN) or an antigen-binding fragment thereof;
S is a sulfur bond connecting Ab and X;
X is selected from C₃-₁₀ cycloalkyl, a 5-10-membered heterocyclic group, C₆-₁₀ aryl, or a 5-10-membered heteroaryl group;
X is optionally substituted with 1, 2, 3, 4, or 5 instance(s) of R*;
R* is selected from H, deuterium, halogen, CN, NO₂, OH, C₁-₆ alkyl, C₁-₆ haloalkyl, C₂-₆ alkenyl, or C₂-₆ alkynyl;
Y is -Y₁-Y₂-Y₃-, Y being linked to X via Y₁ and to Z via Y₃;
Y₁ is selected from -C(O)-, -C(O)O-, -OC(O)-, -C(O)NH-, C₂-₆ alkenyl, C₂-₆ alkynyl, C₃-₁₀ cycloalkyl, a 5-10-membered heterocyclic group, C₆₋₁₀ aryl, or a 5-10-membered heteroaryl;
Y₂ is a bond or C₁-₆ alkylene;
Y₃ is selected from -C(O)-, -OC(O)-, or -NHC(O)-;
Y is optionally substituted with 1, 2, 3, 4, or 5 instance(s) of R#;
R# is selected from H, deuterium, halogen, CN, NO₂, OH, C₁-₆ alkyl, C₁-₆ haloalkyl, C₂-₆ alkenyl, or C₂-₆ alkynyl;
Z is a peptide containing 2 to 8 amino acids, preferably a dipeptide, tripeptide, or tetrapeptide;
E is absent or a spacer unit connecting Z and D;
D is a biologically active molecule drug or a derivative thereof;
n is selected from 0, 1, 2, 3, 4, or 5, preferably 0, 1, 2, or 3;
q is an integer selected from 1 to 10, preferably 8.

2. The antibody-drug conjugate according to Claim 1, wherein,
X is selected from C₆₋₁₀ aryl or a 5-10-membered heteroaryl group;
X is optionally substituted with 1, 2, 3, 4, or 5 instance(s) of R*;
R* is selected from H, deuterium, halogen, CN, NO₂, OH, or C₁-₆ alkyl;
preferably,
X is a 5-6-membered heteroaryl group;
X is optionally substituted with 1, 2, or 3 instance(s) of R*;
R* is selected from H, deuterium, halogen, or C₁-₄ alkyl;
more preferably,
X is pyrimidinyl, e.g.,

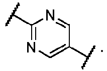

3. The antibody-drug conjugate according to Claim 1 or 2, wherein,
Y is -Y₁-Y₂-Y₃-, Y being linked to X via Y₁ and to Z via Y₃;
wherein,
Y₁ is selected from C₂-₆ alkenyl or C₂-₆ alkynyl;
Y₂ is a bond or C₁-₆ alkylene;
Y₃ is selected from -C(O)- or -NHC(O)-;
Y is optionally substituted with 1, 2, 3, 4, or 5 instance(s) of R#;
R# is selected from H, deuterium, halogen, CN, NO₂, OH, or C₁-₆ alkyl;
preferably,
Y is -Y₁-Y₂-Y₃-, Y being linked to X via Y₁ and to Z via Y₃,
wherein,
Y₁ is selected from C₂-₄ alkenyl or C₂-₄ alkynyl;
Y₂ is C₁-₄ alkylene;
Y₃ is -C(O)-;
Y is optionally substituted with 1, 2, or 3 instance(s) of R#;
R# is selected from H, deuterium, halogen, or C₁-₄ alkyl;
more preferably,
Y is

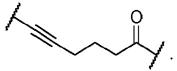

4. The antibody-drug conjugate according to any one of Claims 1 to 3, wherein,
Z is selected from val-cit, val-cit-gly, gly-gly, gly-gly-gly, gly-gly-gly-gly, val-gly-gly, val-gln-gly, val-glu-gly, phe-lys-gly, leu-lys-gly, gly-val-lys-gly, val-lys-gly-gly, val-lys-gly, val-lys-ala, val-lys-leu, leu-leu-gly, gly-gly-phe-gly, gly-gly-phe-gly-gly, val-gly, val-lys-β-ala, or the aforementioned dipeptides, tripeptides, and tetrapeptides, after undergoing modification by means of substitution;
preferably,
Z is 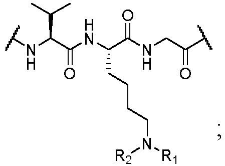
R₁ and R₂ are independently selected from H, deuterium, C₁-₆ alkyl, C₁-₆ haloalkyl, C₂-₆ alkenyl, or C₂-₆ alkynyl;
more preferably,
Z is 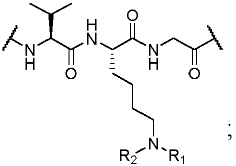
R₁ and R₂ are independently selected from H, deuterium, halogen, or C₁-₆ alkyl, preferably H or C₁-₄ alkyl.

5. The antibody-drug conjugate according to any one of Claims 1-4, wherein,
E is absent or is selected from -NH-C₁-₆ alkylene- or -O-C₁-₆ alkylene-, the -NH-C₁-₆ alkylene- and -O-C₁-₆ alkylene- may optionally be substituted with 1, 2, or 3 substituents selected from H, halogen, C₁-₆ alkyl, and C₁-₆ haloalkyl;
preferably, E is absent or is -NH-C₁-₄ alkylene-, the -NH-C₁-₄ alkylene- may optionally be substituted with 1, 2, or 3 substituents selected from H, halogen, C₁-₄ alkyl, and C₁-₄ haloalkyl;
more preferably, E is -NH-(CH₂)₁-₄-, for example, -NH-CH₂-.

6. The antibody-drug conjugate according to any one of Claims 1 to 5, wherein,
D is selected from metal complexes; glycopeptide antibiotics; DNA topoisomerase inhibitors; drugs that interfere with DNA synthesis; drugs targeting structural proteins; tumor signaling pathway inhibitors; proteasome inhibitors; histone deacetylase inhibitors; tumor angiogenesis inhibitors; cyclin inhibitors; serine/threonine kinase inhibitors, tyrosine kinase inhibitors, aspartate kinase inhibitors, or histidine kinase inhibitors; and other active substances that inhibit tumor cell growth, or promote tumor cell apoptosis or necrosis;
preferably,
D is selected from oxaliplatin; bleomycin or pingyangmycin; camptothecin, camptothecin derivatives (e.g., hydroxycamptothecin, 9-aminocamptothecin, etc.), SN-38, irinotecan, topotecan, belotecan, or rubitecan; actinomycin D, doxorubicin, duocarmycin, daunorubicin, mitoxantrone, podophyllotoxin, or etoposide; methotrexate, 5-fluorouracil, cytarabine, gemcitabine, mercaptopurine, pentostatin, fludarabine, cladribine, or nelarabine; vinca alkaloids, vincristine, vinblastine, paclitaxel, docetaxel, or cabazitaxel; maytansinoid derivatives; calicheamicin derivatives; auristatin derivatives; pyrrolobenzodiazepine dimer derivatives; melphalan; mitomycin C; or chlorambucil;
more preferably,
D is 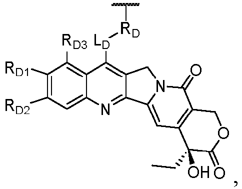
wherein,
R_{D} is absent or selected from -NH- or -O-;
L_{D} is selected from -C₀₋₆alkylene-O-C₀₋₆alkylene- or -C₀₋₆alkylene-O-C₀₋₆alkylene-C(O)NH-CHR_{D4}-;
R_{D1} and R_{D2} are independently selected from H, halogen, C₁₋₆ alkyl, C₁₋₆ haloalkyl or C₁₋₆ alkoxy; or R_{D1} and R_{D2}, together with the carbon atoms to which they are connected, form a ring, e.g. C₆₋₁₀ aryl, a 6-10-membered heteroaryl group, C₃₋₇ cycloalkyl or a 3-7-membered heterocyclic group, preferably a 5-6-membered heterocyclic group;
R_{D3} and R_{D4} are independently selected from H, halogen, C₁₋₆ alkyl, C₁₋₆ haloalkyl or C₁₋₆ alkoxy; or R_{D3} and R_{D4}, together with the carbon atoms to which they are connected, form a ring, e.g., C₆₋₁₀ aryl, a 5-10 heteroaryl, C₅₋₁₀ cycloalkyl or a 5-10-membered heterocyclic group, preferably C₅₋₆ cycloalkyl;
more preferably
D is selected from 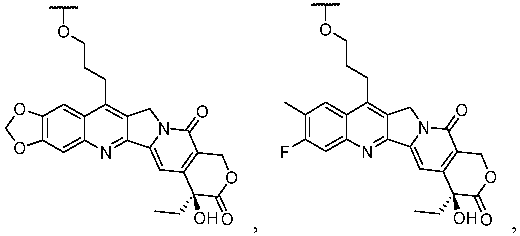 or

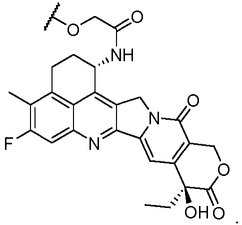

7. The antibody-drug conjugate according to any one of Claims 1 to 6, wherein,
X is selected from C₆₋₁₀ aryl or a 5-10-membered heteroaryl group;
X is optionally substituted with 1, 2, 3, 4, or 5 instance(s) of R*;
R* is selected from H, deuterium, halogen, CN, NO₂, OH, or C₁-₆ alkyl;
Y is -Y₁-Y₂-Y₃-, Y being linked to X via Y₁ and to Z via Y₃;
wherein,
Y₁ is selected from C₂-₆ alkenyl or C₂-₆ alkynyl;
Y₂ is a bond or C₁-₆ alkylene;
Y₃ is selected from -C(O)- or -NHC(O)-;
Y is optionally substituted with 1, 2, 3, 4, or 5 instances of R#;
R# is selected from H, deuterium, halogen, CN, NO₂, OH, or C₁-₆ alkyl;
Z is selected from val-cit, val-cit-gly, gly-gly, gly-gly-gly, gly-gly-gly-gly, val-gly-gly, val-gln-gly, val-glu-gly, phe-lys-gly, leu-lys-gly, gly-val-lys-gly, val-lys-gly-gly, val-lys-gly, val-lys-ala, val-lys-leu, leu-leu-gly, gly-gly-phe-gly, gly-gly-phe-gly-gly, val-gly, val-lys-β-ala, or the aforementioned dipeptides, tripeptides, and tetrapeptides, after undergoing modification by means of substitution;
E is absent or selected from -NH-C₁-₆ alkylene- or -O-C₁-₆ alkylene-, the -NH-C₁-₆ alkylene- and -O-C₁-₆ alkylene- may optionally be substituted with 1, 2, or 3 substituents selected from H, halogen, C₁-₆ alkyl, and C₁-₆ haloalkyl;
D is selected from metal complexes; glycopeptide antibiotics; DNA topoisomerase inhibitors; drugs that interfere with DNA synthesis; drugs targeting structural proteins; tumor signaling pathway inhibitors; proteasome inhibitors; histone deacetylase inhibitors; tumor angiogenesis inhibitors; cyclin inhibitors; serine/threonine kinase inhibitors, tyrosine kinase inhibitors, aspartate kinase inhibitors, or histidine kinase inhibitors; and other active substances that inhibit tumor cell growth or promote tumor cell apoptosis or necrosis.

8. The antibody-drug conjugate according to any one of Claims 1 to 7, wherein,
X is a 5-6-membered heteroaryl group;
X is optionally substituted with 1, 2, or 3 instance(s) of R*;
R* is selected from H, deuterium, halogen, or C₁-₄ alkyl;
Y is -Y₁-Y₂-Y₃-, Y being linked to X via Y₁ and to Z via Y₃,
wherein,
Y₁ is selected from C₂-₄ alkenyl or C₂-₄ alkynyl;
Y₂ is C₁-₄ alkylene;
Y₃ is -C(O)-;
Y is optionally substituted with 1, 2, or 3 instance(s) of R#;
R# is selected from H, deuterium, halogen, or C₁-₄ alkyl;
Z is 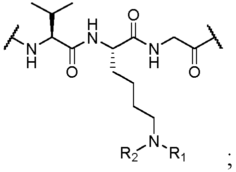
E is absent or is -NH-C₁-₄ alkylene-, the -NH-C₁-₄ alkylene- may optionally be substituted with 1, 2, or 3 substituents selected from H, halogen, C₁-₄ alkyl, or C₁-₄ haloalkyl;
R₁ and R₂ are independently selected from H, deuterium, halogen, C₁-₆ alkyl, C₁-₆ haloalkyl, C₂-₆ alkenyl, or C₂-₆ alkynyl;
D is selected from oxaliplatin; bleomycin or pingyangmycin; camptothecin, camptothecin derivatives (e.g., hydroxycamptothecin, 9-aminocamptothecin, etc.), SN-38, irinotecan, topotecan, belotecan, or rubitecan; actinomycin D, doxorubicin, duocarymcin, daunorubicin, mitoxantrone, podophyllotoxin, or etoposide; methotrexate, 5-fluorouracil, cytarabine, gemcitabine, mercaptopurine, pentostatin, fludarabine, cladribine, or nelarabine; vinca alkaloids, vincristine, vinblastine, paclitaxel, docetaxel, or cabazitaxel; maytansinoid derivatives; calicheamicin derivatives; auristatin derivatives; pyrrolobenzodiazepine dimer derivatives; melphalan; mitomycin C; or chlorambucil;
preferably, D is 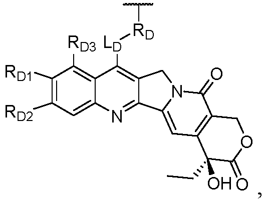
wherein,
R_{D} is absent or is selected from -NH- or -O-;
L_{D} is selected from -C₀₋₆alkylene-O-C₀₋₆alkylene- or -C₀₋₆alkylene-O-C₀₋₆alkylene-C(O)NH-CHR_{D4}-;
R_{D1} and R_{D2} are independently selected from H, halogen, C₁₋₆ alkyl, C₁₋₆ haloalkyl or C₁₋₆ alkoxy; or R_{D1} and R_{D2}, together with the carbon atoms to which they are connected, form a ring, e.g. C₆₋₁₀ aryl, a 5-10-membered heteroaryl group, C₃₋₇ cycloalkyl or a 3-7-membered heterocyclic group, preferably a 5-6-membered heterocyclic group;
R_{D3} and R_{D4} are independently selected from H, halogen, C₁₋₆ alkyl, C₁₋₆ haloalkyl or C₁₋₆ alkoxy; or R_{D3} and R_{D4}, together with the carbon atoms to which they are connected, form a ring, e.g., C₆₋₁₀ aryl, a 5-10-membered heteroaryl group, C₅₋₁₀ cycloalkyl or a 5-10-membered heterocyclic group, preferably C₅₋₆ cycloalkyl.

9. The antibody-drug conjugate according to any one of Claims 1 to 8, wherein,
X is pyrimidinyl, preferably 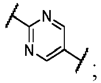
Y is 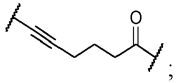
Z is 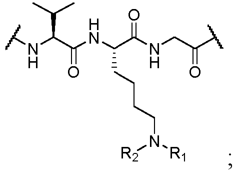
R₁ and R₂ are independently selected from H, deuterium, halogen, C₁-₆ alkyl, preferably H or C₁-₄ alkyl;
E is -NH-CH₂-;
D is selected from 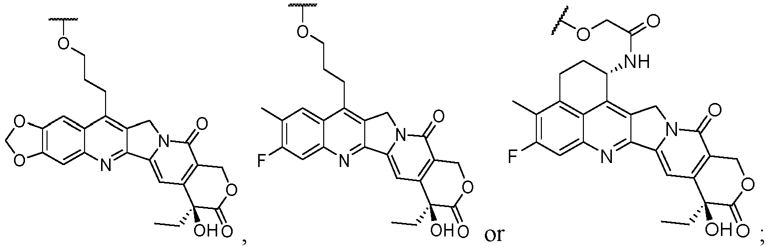
n is selected from 1, 2, or 3, preferably 1;
q is selected from 4, 5, 6, 7, 8, 9, or 10, preferably 8.

10. The antibody-drug conjugate according to any one of Claims 1 to 9, wherein the antibody-drug conjugate is a compound of formula (II) or a tautomer, stereoisomer, or pharmaceutically acceptable salt thereof: 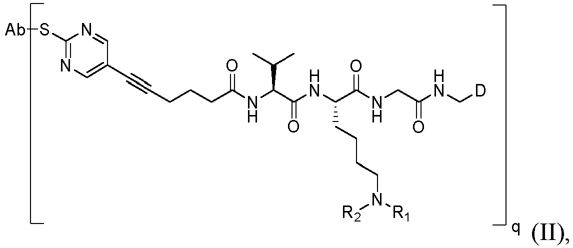 wherein,
Ab is an anti-MSLN antibody;
R₁ and R₂ are independently selected from C₁-₆ alkyl, preferably C₁-₄ alkyl;
D is 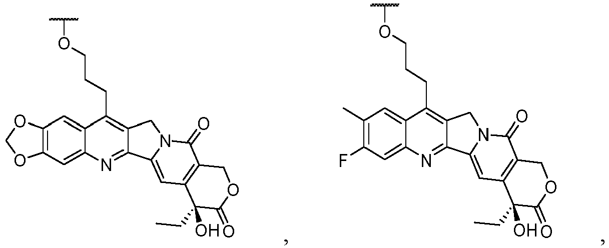 or 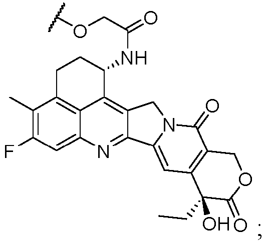
q is an integer selected from 1-10, for example, selected from 4, 5, 6, 7, 8, 9, or 10, preferably 8.

11. The antibody-drug conjugate according to any one of Claims 1 to 10, wherein the antibody-drug conjugate is selected from the following compounds or tautomers, stereoisomers, or pharmaceutically acceptable salts thereof:
1) 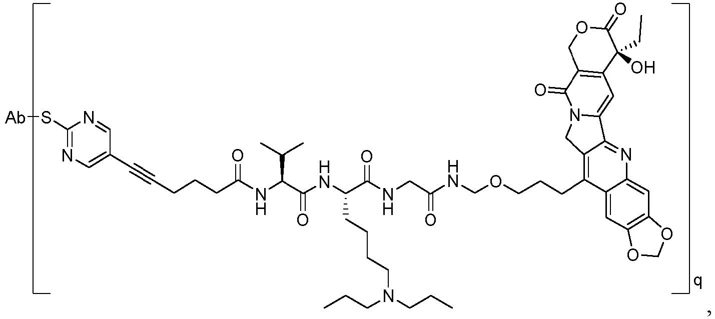
2) 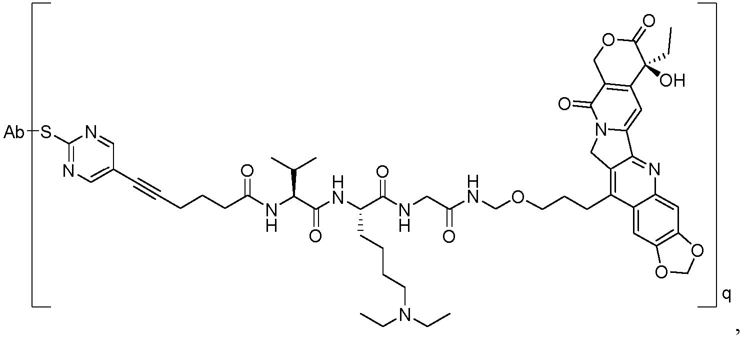
3) 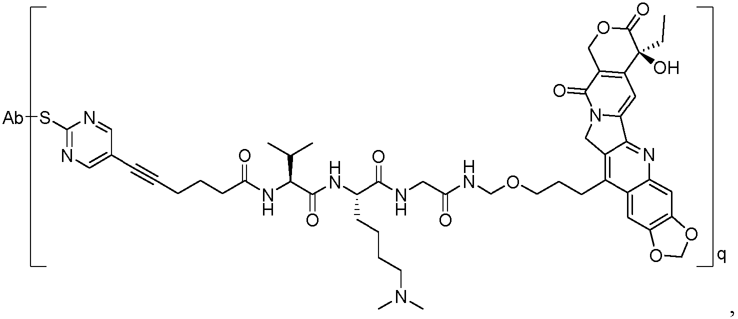
4) 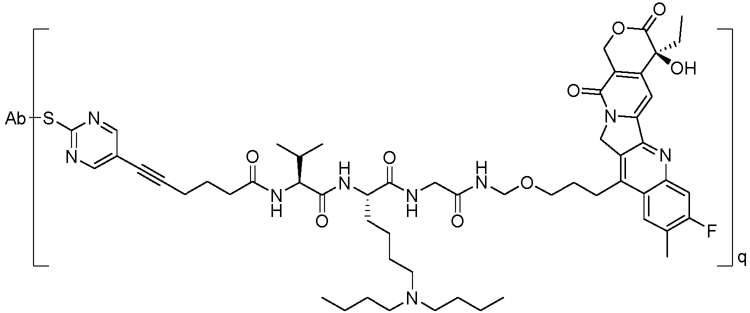 or
5) 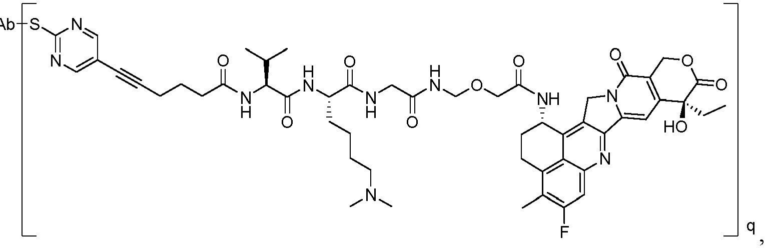
wherein, Ab is an anti-MSLN antibody;
q is an integer selected from 1-10, for example, selected from 4, 5, 6, 7, 8, 9, or 10, preferably 8.

12. The antibody-drug conjugate according to any one of Claims 1 to 11, wherein the antibody comprises a heavy chain variable region (VH) and a light chain variable region (VL), and wherein,
(1) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 27;
(2) the VH comprises HCDR1, HCDR2, and HCDR3 of the VH with the amino acid sequence shown in SEQ ID NO: 25, and the VL comprises LCDR1, LCDR2, and LCDR3 of the VL with the amino acid sequence shown in SEQ ID NO: 28;
(3) the VH comprises HCDR1, HCDR2, and HCDR3 of the VH with the amino acid sequence shown in SEQ ID NO: 26, and the VL comprises LCDR1, LCDR2, and LCDR3 of the VL with the amino acid sequence shown in SEQ ID NO: 27;
(4) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 29;
(5) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 30;
(6) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 31;
(7) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 32;
(8) the VH comprises HCDR1, HCDR2, and HCDR3 of the VH with the amino acid sequence shown in SEQ ID NO: 24, and the VL comprises LCDR1, LCDR2, and LCDR3 of the VL with the amino acid sequence shown in SEQ ID NO: 33;
(9) the VH comprises HCDR1, HCDR2, and HCDR3 of the VH with the amino acid sequence shown in SEQ ID NO: 25, and the VL comprises LCDR1, LCDR2, and LCDR3 of the VL with the amino acid sequence shown in SEQ ID NO: 29;
(10) the VH comprises HCDR1, HCDR2, and HCDR3 of the VH with the amino acid sequence shown in SEQ ID NO: 63, and the VL comprises LCDR1, LCDR2, and LCDR3 of the VL with the amino acid sequence shown in SEQ ID NO: 67;
(11) the VH comprises HCDR1, HCDR2, and HCDR3 of the VH with the amino acid sequence shown in SEQ ID NO: 64, and the VL comprises LCDR1, LCDR2, and LCDR3 of the VL with the amino acid sequence shown in SEQ ID NO: 67;
(12) the VH comprises HCDR1, HCDR2, and HCDR3 of the VH with the amino acid sequence shown in SEQ ID NO: 65, and the VL comprises LCDR1, LCDR2, and LCDR3 of the VL with the amino acid sequence shown in SEQ ID NO: 67;
(13) the VH comprises HCDR1, HCDR2, and HCDR3 of the VH with the amino acid sequence shown in SEQ ID NO: 65, and the VL comprises LCDR1, LCDR2, and LCDR3 of the VL with the amino acid sequence shown in SEQ ID NO: 68;
(14) the VH comprises HCDR1, HCDR2, and HCDR3 of the VH with the amino acid sequence shown in SEQ ID NO: 63, and the VL comprises LCDR1, LCDR2, and LCDR3 of the VL with the amino acid sequence shown in SEQ ID NO: 68;
(15) the VH comprises HCDR1, HCDR2, and HCDR3 of the VH with the amino acid sequence shown in SEQ ID NO: 66, and the VL comprises LCDR1, LCDR2, and LCDR3 of the VL with the amino acid sequence shown in SEQ ID NO: 67;
(16) the VH comprises HCDR1, HCDR2, and HCDR3 of the VH with the amino acid sequence shown in SEQ ID NO: 64, and the VL comprises LCDR1, LCDR2, and LCDR3 of the VL with the amino acid sequence shown in SEQ ID NO: 69;
(17) the VH comprises HCDR1, HCDR2, and HCDR3 of the VH with the amino acid sequence shown in SEQ ID NO: 66, and the VL comprises LCDR1, LCDR2, and LCDR3 of the VL with the amino acid sequence shown in SEQ ID NO: 69;
(18) the VH comprises HCDR1, HCDR2, and HCDR3 of the VH with the amino acid sequence shown in SEQ ID NO: 63, and the VL comprises LCDR1, LCDR2, and LCDR3 of the VL with the amino acid sequence shown in SEQ ID NO: 69; or
(19) the VH comprises HCDR1, HCDR2, and HCDR3 of the VH with the amino acid sequence shown in SEQ ID NO: 65, and the VL comprises LCDR1, LCDR2, and LCDR3 of the VL with the amino acid sequence shown in SEQ ID NO: 69.

13. The antibody-drug conjugate according to Claim 12, wherein,
(1) the VH comprises HCDR1, HCDR2, and HCDR3, the HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 13;
(2) the VH comprises HCDR1, HCDR2, and HCDR3, the HCDR1 comprising the amino acid sequence shown in SEQ ID NO: 15, HCDR2 comprising the amino acid sequence shown in SEQ ID NO: 16, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 18;
(3) the VH comprises HCDR1, HCDR2, and HCDR3, the HCDR1 comprising the amino acid sequence shown in SEQ ID NO: 15, HCDR2 comprising the amino acid sequence shown in SEQ ID NO: 17, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 13;
(4) the VH comprises HCDR1, HCDR2, and HCDR3, the HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 19;
(5) the VH comprises HCDR1, HCDR2, and HCDR3, the HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 20;
(6) the VH comprises HCDR1, HCDR2, and HCDR3, the HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 21;
(7) the VH comprises HCDR1, HCDR2, and HCDR3, the HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 22;
(8) the VH comprises HCDR1, HCDR2, and HCDR3, the HCDR1 comprising the amino acid sequence shown in SEQ ID NO: 2, HCDR2 comprising the amino acid sequence shown in SEQ ID NO: 4, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 23;
(9) the VH comprises HCDR1, HCDR2, and HCDR3, the HCDR1 comprising the amino acid sequence shown in SEQ ID NO: 15, HCDR2 comprising the amino acid sequence shown in SEQ ID NO: 16, and HCDR3 comprising the amino acid sequence shown in SEQ ID NO: 6, and the VL comprises LCDR1, LCDR2, and LCDR3, the LCDR1 comprising the amino acid sequence shown in SEQ ID NO: 9, LCDR2 comprising the amino acid sequence shown in SEQ ID NO: 11, and LCDR3 comprising the amino acid sequence shown in SEQ ID NO: 19;
(10) the VH comprises HCDR1, HCDR2, and HCDR3, the HCDR1 comprising the amino acid sequence shown in SEQ ID NO: 45, HCDR2 comprising the amino acid sequence shown in SEQ ID NO: 47, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 52, LCDR2 comprising the amino acid sequence shown in SEQ ID NO: 54, and LCDR3 comprising the amino acid sequence shown in SEQ ID NO: 56;
(11) the VH comprises HCDR1, HCDR2, and HCDR3, the HCDR1 comprising the amino acid sequence shown in SEQ ID NO: 58, HCDR2 comprising the amino acid sequence shown in SEQ ID NO: 47, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 52, LCDR2 comprising the amino acid sequence shown in SEQ ID NO: 54, and LCDR3 comprising the amino acid sequence shown in SEQ ID NO: 56;
(12) the VH comprises HCDR1, HCDR2, and HCDR3, the HCDR1 comprising the amino acid sequence shown in SEQ ID NO: 58, HCDR2 comprising the amino acid sequence shown in SEQ ID NO: 59, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 52, LCDR2 comprising the amino acid sequence shown in SEQ ID NO: 54, and LCDR3 comprising the amino acid sequence shown in SEQ ID NO: 56;
(13) the VH comprises HCDR1, HCDR2, and HCDR3, the HCDR1 comprising the amino acid sequence shown in SEQ ID NO: 58, HCDR2 comprising the amino acid sequence shown in SEQ ID NO: 59, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 61, LCDR2 comprising the amino acid sequence shown in SEQ ID NO: 54, and LCDR3 comprising the amino acid sequence shown in SEQ ID NO: 56;
(14) the VH comprises HCDR1, HCDR2, and HCDR3, the HCDR1 comprising the amino acid sequence shown in SEQ ID NO: 45, HCDR2 comprising the amino acid sequence shown in SEQ ID NO: 47, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 61, LCDR2 comprising the amino acid sequence shown in SEQ ID NO: 54, and LCDR3 comprising the amino acid sequence shown in SEQ ID NO: 56;
(15) the VH comprises HCDR1, HCDR2, and HCDR3, the HCDR1 comprising the amino acid sequence shown in SEQ ID NO: 58, HCDR2 comprising the amino acid sequence shown in SEQ ID NO: 60, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 52, LCDR2 comprising the amino acid sequence shown in SEQ ID NO: 54, and LCDR3 comprising the amino acid sequence shown in SEQ ID NO: 56;
(16) the VH comprises HCDR1, HCDR2, and HCDR3, the HCDR1 comprising the amino acid sequence shown in SEQ ID NO: 58, HCDR2 comprising the amino acid sequence shown in SEQ ID NO: 47, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 62, LCDR2 comprising the amino acid sequence shown in SEQ ID NO: 54, and LCDR3 comprising the amino acid sequence shown in SEQ ID NO: 56;
(17) the VH comprises HCDR1, HCDR2, and HCDR3, the HCDR1 comprising the amino acid sequence shown in SEQ ID NO: 58, HCDR2 comprising the amino acid sequence shown in SEQ ID NO: 60, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 62, LCDR2 comprising the amino acid sequence shown in SEQ ID NO: 54, and LCDR3 comprising the amino acid sequence shown in SEQ ID NO: 56;
(18) the VH comprises HCDR1, HCDR2, and HCDR3, the HCDR1 comprising the amino acid sequence shown in SEQ ID NO: 45, HCDR2 comprising the amino acid sequence shown in SEQ ID NO: 47, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 62, LCDR2 comprising the amino acid sequence shown in SEQ ID NO: 54, and LCDR3 comprising the amino acid sequence shown in SEQ ID NO: 56;
(19) the VH comprises HCDR1, HCDR2, and HCDR3, the HCDR1 comprising the amino acid sequence shown in SEQ ID NO: 58, HCDR2 comprising the amino acid sequence shown in SEQ ID NO: 59, and HCDR3 comprising the amino acid sequence shown in SEQ ID NO: 49, and the VL comprises LCDR1, LCDR2, and LCDR3, the LCDR1 comprising the amino acid sequence shown in SEQ ID NO: 62, LCDR2 comprising the amino acid sequence shown in SEQ ID NO: 54, and LCDR3 comprising the amino acid sequence shown in SEQ ID NO: 56.

14. The antibody-drug conjugate according to Claim 12 or 13, wherein,
(1) the VH comprises an amino acid sequence with at least 80%, at least 85%, at least 90%, at least 95%, at least 98%, at least 99%, or 100% sequence identity to in SEQ ID NO: 24, and the VL comprises an amino acid sequence with at least 80%, at least 85%, at least 90%, at least 95%, at least 98%, at least 99%, or 100% sequence identity to SEQ ID NO: 27;
(2) the VH comprises an amino acid sequence with at least 80%, at least 85%, at least 90%, at least 95%, at least 98%, at least 99%, or 100% sequence identity to SEQ ID NO: 25, and the VL comprises an amino acid sequence with at least 80%, at least 85%, at least 90%, at least 95%, at least 98%, at least 99%, or 100% sequence identity to SEQ ID NO: 28;
(3) the VH comprises an amino acid sequence with at least 80%, at least 85%, at least 90%, at least 95%, at least 98%, at least 99%, or 100% sequence identity to SEQ ID NO: 26, and the VL comprises an amino acid sequence with at least 80%, at least 85%, at least 90%, at least 95%, at least 98%, at least 99%, or 100% sequence identity to SEQ ID NO: 27;
(4) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 29;
(5) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 30;
(6) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 31;
(7) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 32;
(8) the VH comprises an amino acid sequence with at least 80%, at least 85%, at least 90%, at least 95%, at least 98%, at least 99%, or 100% sequence identity to SEQ ID NO: 24, and the VL comprises an amino acid sequence with at least 80%, at least 85%, at least 90%, at least 95%, at least 98%, at least 99%, or 100% sequence identity to SEQ ID NO: 33;
(9) the VH comprises an amino acid sequence with at least 80%, at least 85%, at least 90%, at least 95%, at least 98%, at least 99%, or 100% sequence identity to SEQ ID NO: 25, and the VL comprises an amino acid sequence with at least 80%, at least 85%, at least 90%, at least 95%, at least 98%, at least 99%, or 100% sequence identity to SEQ ID NO: 29;
(10) the VH comprises an amino acid sequence with at least 80%, at least 85%, at least 90%, at least 95%, at least 98%, at least 99%, or 100% sequence identity to SEQ ID NO: 63, and the VL comprises an amino acid sequence with at least 80%, at least 85%, at least 90%, at least 95%, at least 98%, at least 99%, or 100% sequence identity to SEQ ID NO: 67;
(11) the VH comprises an amino acid sequence with at least 80%, at least 85%, at least 90%, at least 95%, at least 98%, at least 99%, or 100% sequence identity to SEQ ID NO: 64, and the VL comprises an amino acid sequence with at least 80%, at least 85%, at least 90%, at least 95%, at least 98%, at least 99%, or 100% sequence identity to SEQ ID NO: 67;
(12) the VH comprises an amino acid sequence with at least 80%, at least 85%, at least 90%, at least 95%, at least 98%, at least 99%, or 100% sequence identity to SEQ ID NO: 65, and the VL comprises an amino acid sequence with at least 80%, at least 85%, at least 90%, at least 95%, at least 98%, at least 99%, or 100% sequence identity to SEQ ID NO: 67;
(13) the VH comprises an amino acid sequence with at least 80%, at least 85%, at least 90%, at least 95%, at least 98%, at least 99%, or 100% sequence identity to SEQ ID NO: 65, and the VL comprises an amino acid sequence with at least 80%, at least 85%, at least 90%, at least 95%, at least 98%, at least 99%, or 100% sequence identity to SEQ ID NO: 68;
(14) the VH comprises an amino acid sequence with at least 80%, at least 85%, at least 90%, at least 95%, at least 98%, at least 99%, or 100% sequence identity to SEQ ID NO: 63, and the VL comprises an amino acid sequence with at least 80%, at least 85%, at least 90%, at least 95%, at least 98%, at least 99%, or 100% sequence identity to SEQ ID NO: 68;
(15) the VH comprises an amino acid sequence with at least 80%, at least 85%, at least 90%, at least 95%, at least 98%, at least 99%, or 100% sequence identity to SEQ ID NO: 66, and the VL comprises an amino acid sequence with at least 80%, at least 85%, at least 90%, at least 95%, at least 98%, at least 99%, or 100% sequence identity to SEQ ID NO: 67;
(16) the VH comprises an amino acid sequence with at least 80%, at least 85%, at least 90%, at least 95%, at least 98%, at least 99%, or 100% sequence identity to SEQ ID NO: 64, and the VL comprises an amino acid sequence with at least 80%, at least 85%, at least 90%, at least 95%, at least 98%, at least 99%, or 100% sequence identity to SEQ ID NO: 69;
(17) the VH comprises an amino acid sequence with at least 80%, at least 85%, at least 90%, at least 95%, at least 98%, at least 99%, or 100% sequence identity to SEQ ID NO: 66, and the VL comprises an amino acid sequence with at least 80%, at least 85%, at least 90%, at least 95%, at least 98%, at least 99%, or 100% sequence identity to SEQ ID NO: 69;
(18) the VH comprises an amino acid sequence with at least 80%, at least 85%, at least 90%, at least 95%, at least 98%, at least 99%, or 100% sequence identity to SEQ ID NO: 63, and the VL comprises an amino acid sequence with at least 80%, at least 85%, at least 90%, at least 95%, at least 98%, at least 99%, or 100% sequence identity to SEQ ID NO: 69; or
(19) the VH comprises an amino acid sequence with at least 80%, at least 85%, at least 90%, at least 95%, at least 98%, at least 99%, or 100% sequence identity to SEQ ID NO: 65, and the VL comprises an amino acid sequence with at least 80%, at least 85%, at least 90%, at least 95%, at least 98%, at least 99%, or 100% sequence identity to the SEQ ID NO: 69.

15. The antibody-drug conjugate according to any one of Claims 12-14, wherein the antibody comprises a heavy chain (HC) and a light chain (LC), and wherein:
(1) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 37;
(2) the HC comprises an amino acid sequence with at least 80%, at least 85%, at least 90%, at least 95%, at least 98%, at least 99%, or 100% sequence identity to SEQ ID NO: 35, and the LC comprises an amino acid sequence with at least 80%, at least 85%, at least 90%, at least 95%, at least 98%, at least 99%, or 100% sequence identity to SEQ ID NO: 38;
(3) the HC comprises an amino acid sequence with at least 80%, at least 85%, at least 90%, at least 95%, at least 98%, at least 99%, or 100% sequence identity to SEQ ID NO: 36, and the LC comprises an amino acid sequence with at least 80%, at least 85%, at least 90%, at least 95%, at least 98%, at least 99%, or 100% sequence identity to SEQ ID NO: 37;
(4) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 39;
(5) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 40;
(6) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 41;
(7) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to SEQ ID NO: 42;
(8) the HC comprises an amino acid sequence with at least 80%, at least 85%, at least 90%, at least 95%, at least 98%, at least 99%, or 100% sequence identity to SEQ ID NO: 34, and the LC comprises an amino acid sequence with at least 80%, at least 85%, at least 90%, at least 95%, at least 98%, at least 99%, or 100% sequence identity to in SEQ ID NO: 43;
(9) the HC comprises an amino acid sequence with at least 80%, at least 85%, at least 90%, at least 95%, at least 98%, at least 99%, or 100% sequence identity to SEQ ID NO: 35, and the LC comprises an amino acid sequence with at least 80%, at least 85%, at least 90%, at least 95%, at least 98%, at least 99%, or 100% sequence identity to SEQ ID NO: 39;
(10) the HC comprises an amino acid sequence with at least 80%, at least 85%, at least 90%, at least 95%, at least 98%, at least 99%, or 100% sequence identity to SEQ ID NO: 70, and the LC comprises an amino acid sequence with at least 80%, at least 85%, at least 90%, at least 95%, at least 98%, at least 99%, or 100% sequence identity to SEQ ID NO: 74;
(11) the HC comprises an amino acid sequence with at least 80%, at least 85%, at least 90%, at least 95%, at least 98%, at least 99%, or 100% sequence identity to SEQ ID NO: 71, and the LC comprises an amino acid sequence with at least 80%, at least 85%, at least 90%, at least 95%, at least 98%, at least 99%, or 100% sequence identity to SEQ ID NO: 74;
(12) the HC comprises an amino acid sequence with at least 80%, at least 85%, at least 90%, at least 95%, at least 98%, at least 99%, or 100% sequence identity to SEQ ID NO: 72, and the LC comprises an amino acid sequence with at least 80%, at least 85%, at least 90%, at least 95%, at least 98%, at least 99%, or 100% sequence identity to SEQ ID NO: 74;
(13) the HC comprises an amino acid sequence with at least 80%, at least 85%, at least 90%, at least 95%, at least 98%, at least 99%, or 100% sequence identity to SEQ ID NO: 72, and the LC comprises an amino acid sequence with at least 80%, at least 85%, at least 90%, at least 95%, at least 98%, at least 99%, or 100% sequence identity to SEQ ID NO: 75;
(14) the HC comprises an amino acid sequence with at least 80%, at least 85%, at least 90%, at least 95%, at least 98%, at least 99%, or 100% sequence identity to SEQ ID NO: 70, and the LC comprises an amino acid sequence with at least 80%, at least 85%, at least 90%, at least 95%, at least 98%, at least 99%, or 100% sequence identity to SEQ ID NO: 75;
(15) the HC comprises an amino acid sequence with at least 80%, at least 85%, at least 90%, at least 95%, at least 98%, at least 99%, or 100% sequence identity to SEQ ID NO: 73, and the LC comprises an amino acid sequence with at least 80%, at least 85%, at least 90%, at least 95%, at least 98%, at least 99%, or 100% sequence identity to SEQ ID NO: 74;
(16) the HC comprises an amino acid sequence with at least 80%, at least 85%, at least 90%, at least 95%, at least 98%, at least 99%, or 100% sequence identity to SEQ ID NO: 71, and the LC comprises an amino acid sequence with at least 80%, at least 85%, at least 90%, at least 95%, at least 98%, at least 99%, or 100% sequence identity to SEQ ID NO: 76;
(17) the HC comprises an amino acid sequence with at least 80%, at least 85%, at least 90%, at least 95%, at least 98%, at least 99%, or 100% sequence identity to SEQ ID NO: 73, and the LC comprises an amino acid sequence with at least 80%, at least 85%, at least 90%, at least 95%, at least 98%, at least 99%, or 100% sequence identity to SEQ ID NO: 76;
(18) the HC comprises an amino acid sequence with at least 80%, at least 85%, at least 90%, at least 95%, at least 98%, at least 99%, or 100% sequence identity to SEQ ID NO: 70, and the LC comprises an amino acid sequence with at least 80%, at least 85%, at least 90%, at least 95%, at least 98%, at least 99%, or 100% sequence identity to SEQ ID NO: 76; or
(19) the HC comprises an amino acid sequence with at least 80%, at least 85%, at least 90%, at least 95%, at least 98%, at least 99%, or 100% sequence identity to SEQ ID NO: 72, and the LC comprises an amino acid sequence with at least 80%, at least 85%, at least 90%, at least 95%, at least 98%, at least 99%, or 100% sequence identity to SEQ ID NO: 76.

16. The antibody-drug conjugate according to any one of Claims 12-15, wherein the antibody is a chimeric antibody, a humanized antibody or a human antibody

17. The antibody-drug conjugate according to any one of Claims 12-16, wherein the antigen-binding fragment is selected from Fab, Fab', F(ab')₂ Fd, Fd', Fv, scFv and ds-scFv.

18. The antibody-drug conjugate according to any one of Claims 12-17, wherein the antibody is a monoclonal antibody, a bispecific antibody, or a multispecific antibody.

19. The antibody-drug conjugate according to any one of Claims 1-18, wherein the antibody-drug conjugate is selected from the following compounds or tautomers, stereoisomers, or pharmaceutically acceptable salts thereof:
1) 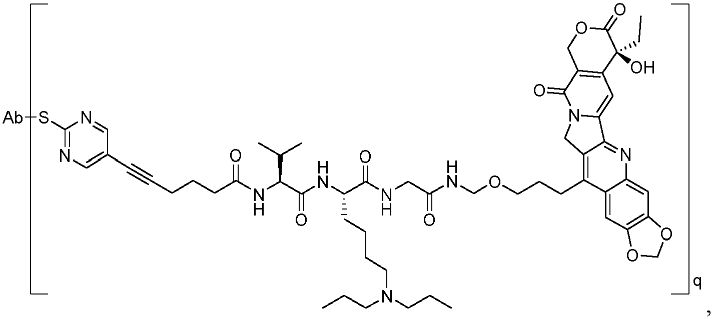
2) 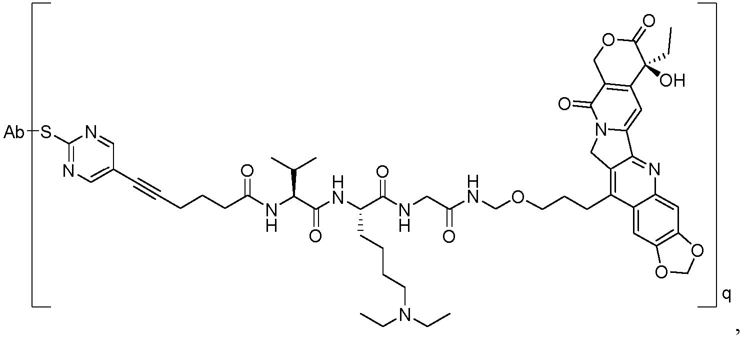
3) 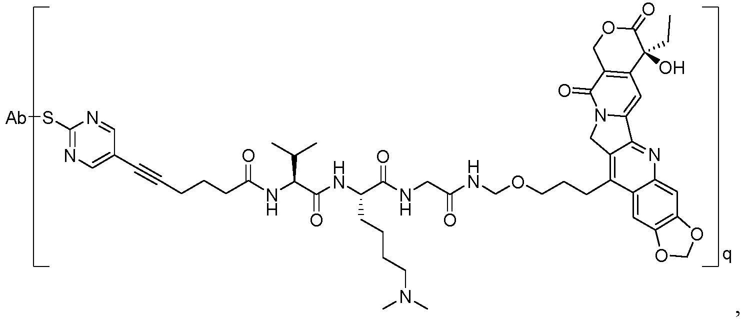
4) 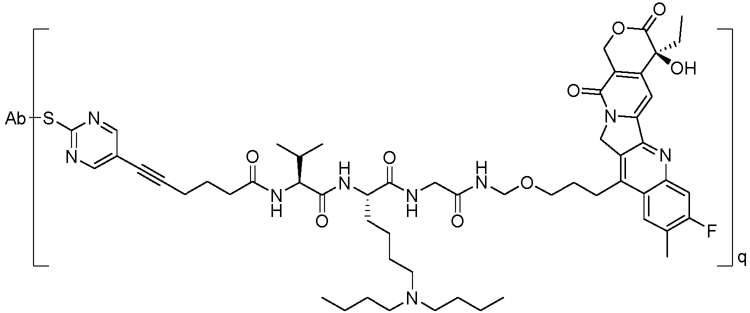 or
5) 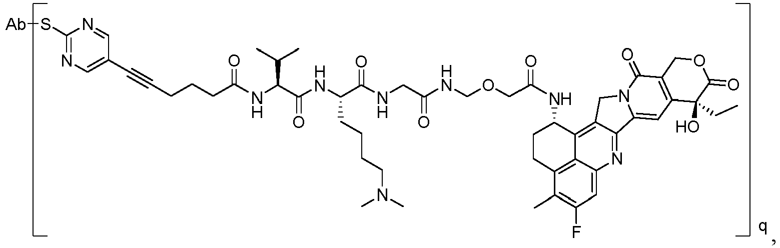
wherein, Ab is an anti-MSLN antibody;
q is an integer selected from 1-10, for example, selected from 4, 5, 6, 7, 8, 9, or 10, preferably 8;
the anti-MSLN antibody comprises a heavy chain (HC) and a light chain (LC), wherein:
(1) the HC comprises the amino acid sequence shown in SEQ ID NO: 34, and the LC comprises the amino acid sequence shown in SEQ ID NO: 37;
(2) the HC comprises the amino acid sequence shown in SEQ ID NO: 34, and the LC comprises the amino acid sequence shown in SEQ ID NO: 43;
(3) the HC comprises the amino acid sequence shown in SEQ ID NO: 70, and the LC comprises the amino acid sequence shown in SEQ ID NO: 74; or
(4) the HC comprises the amino acid sequence shown in SEQ ID NO: 72, and the LC comprises the amino acid sequence shown in SEQ ID NO: 76.

20. A pharmaceutical composition comprising the antibody-drug conjugate according to any one of Claims 1-19, and optionally a pharmaceutically acceptable carrier or excipient.

21. The pharmaceutical composition of Claim 20, wherein the composition further comprises a second therapeutic agent, and the second therapeutic agent is selected from chemotherapeutic agents, monoclonal antibody drugs, bispecific/multispecific antibody drugs, recombinant protein drugs, nucleotide drugs (including siRNA and antisense oligonucleotides), small molecule drugs, immunomodulatory drugs, and cell therapy drugs.

22. A method of treating cancer in a subject, comprising administering to the subject an effective amount of the antibody-drug conjugate of any one of Claims 1-19 or the pharmaceutical composition of Claim 20 or 21.

23. The method according to Claim 22, wherein the cancer is selected from colon cancer, mesothelioma, ovarian cancer, lung cancer, breast cancer, pancreatic cancer, gastric cancer, prostate cancer, squamous cell carcinoma, and cholangiocarcinoma.

24. The method according to Claim 22 or 23, further comprising administering a second therapeutic agent to the subject.

25. The method according to Claim 24, wherein the second therapeutic agent is selected from chemotherapeutic agents, monoclonal antibody drugs, bispecific/multispecific antibody drugs, recombinant protein drugs, nucleotide drugs (including siRNA and antisense oligonucleotides), small molecule drugs, immunomodulatory drugs, and cell therapy drugs.
